(19) Europäisches Patentamt / European Patent Office / Office européen des brevets

(11) EP 4 667 460 A1

(12) # EUROPEAN PATENT APPLICATION
published in accordance with Art. 153(4) EPC

(43) Date of publication:
**24.12.2025 Bulletin 2025/52**

(21) Application number: **24756221.8**

(22) Date of filing: **07.02.2024**

(51) International Patent Classification (IPC):
***C07D 241/44*** (2006.01) ***C07D 241/42*** (2006.01)
***C07D 403/02*** (2006.01) ***C07D 403/04*** (2006.01)
***A61K 31/498*** (2006.01) ***A61K 31/496*** (2006.01)
***A61P 35/00*** (2006.01) ***A61P 29/00*** (2006.01)
***A61P 25/28*** (2006.01) ***A61P 25/16*** (2006.01)

(52) Cooperative Patent Classification (CPC):
**A61K 31/496; A61K 31/498; A61P 25/16; A61P 25/28; A61P 29/00; A61P 35/00; C07D 241/42; C07D 241/44; C07D 403/02; C07D 403/04**

(86) International application number:
**PCT/CN2024/076609**

(87) International publication number:
**WO 2024/169848 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.02.2023 CN 202310130954**
**18.04.2023 CN 202310415260**

(71) Applicant: **Nanjing Synnocare Pharmaceutical Technology Co., Ltd.**
**Nanjing, Jiangsu 211122 (CN)**

(72) Inventors:
- **FAN, Houxing**
  **Nanjing, Jiangsu 211122 (CN)**
- **JIANG, Qiangqiang**
  **Nanjing, Jiangsu 211122 (CN)**
- **WANG, Tiancai**
  **Nanjing, Jiangsu 211122 (CN)**

(74) Representative: **Lavoix**
**Bayerstraße 83**
**80335 München (DE)**

(54) **MIR-124 INDUCER, AND PREPARATION METHOD THEREFOR AND USE THEREOF**

(57) The present invention relates to a compound represented by formula (1) and a preparation method therefor, a composition containing the compound represented by formula (1), and/or a pharmaceutically acceptable salt thereof, a preparation method therefor, and a use of the compound as a miR-124 inducer in the preparation of a medication for treating miR-124 mediated diseases.

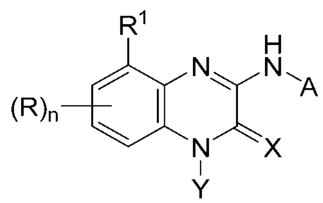

**(1)**

EP 4 667 460 A1

## Description

### FIELD OF THE INVENTION

The invention belongs to the field of medicinal chemistry, and more specifically, relates to a kind of miR-124 inducers as shown in Formula (1), and preparation methods therefor, as well as the use of these compounds for preparing pharmaceutial compositions for treating, regulating, and/or preventing diseases mediated by miR-124.

### BACKGROUND OF THE INVENTION

MicroRNAs (miRNAs) are a class of endogenous small RNAs, approximately 20-24 nucleotides in length, that play diverse and critical regulatory roles within cells. Each miRNA can target multiple genes, and several miRNAs may collectively regulate a single gene, forming a complex regulatory network. This complex regulatory network can either regulate the expression of multiple genes through a single miRNA, or finely regulate the expression of a certain gene through the combination of several miRNAs. It is estimated that miRNAs regulate approximately one-third of all human genes. Although miRNAs account for only about 2% of the human genome, they control the expression of over 30% of human genes, making them as central players in gene regulatory networks [Stem Cells, 2013, 31: 2205-2217]. miRNAs regulate cellular processes such as proliferation, differentiation, and apoptosis, playing crucial roles in individual growth and development, as well as in the initiation and progression of various diseases, including inflammation and cancer. A growing body of research indicates that miRNAs participate in nearly all biological life processes [Trends Mol Med, 2014, 331: 1-10].

MiR-124 is a highly conserved miRNA expressed across species, from lower organisms (e.g., Caenorhabditis elegans) to higher organisms (e.g., humans). Current research indicates that the three encoding genes of human miR-124 are located at chromosomal loci 8p23.1, 8q12.3, and 20q13.33, respectively. The promoters of these genes contain CpG islands, whose methylation silences miR-124 transcriptionally [Oncogene,2012, 31: 1609-1622]. MiR-124 is the most abundantly expressed miRNA in the central nervous system (CNS). Evidence suggests that miR-124 is closely associated with damage repair in the CNS. Niina Vuokila et al. found that after brain injury, the hippocampal gene expression network is subject to long-term regulation by miR-124, indicating that miR-124 acts as a chronic modulator of gene expression following brain injury [Cell Mol Life Sci, 2018, 75: 4557-4581]. MiR-124 demonstrates significant therapeutic potential in Alzheimer's disease (AD). It is highly and specifically expressed in the brain, yet its levels are markedly downregulated in the brains of AD patients. MiR-124 regulates the expression of multiple genes (e.g., BACE1, Caveolin-1, GSK-3β, PTPN1, DACT1), thereby influencing synaptic plasticity, neuroinflammation, Aβ production, and Tau protein phosphorylation. Moreover, miR-124 is found to be under-expressed in various cancers, including brain tumors, gastric cancer, breast cancer, cervical cancer, and prostate cancer, suggesting its potential as a therapeutic target for multiple malignancies.

In recent years, miR-124 has attracted significant attention due to its anti-inflammatory activity. ABX464, a small-molecule drug, exerts potent anti-inflammatory and antiviral effects by modulating RNA splicing, along with the ability to inhibit HIV virus replication. The anti-inflammatory mechanism of ABX464 is triggered by its binding to the cap-binding complex, which is located at the 5' end of non-coding RNA molecules within cells. This binding induces splicing of a long non-coding RNA, leading to the overexpression of a specific microRNA product-miR-124. Subsequently, miR-124 initiates a cascade of events that ultimately generates a robust anti-inflammatory response [Retrovirology, 2015, 12: 1-15]. ABX464 is currently in development for treating inflammatory and viral diseases, such as Crohn's disease, ulcerative colitis, rheumatoid arthritis, HIV/AIDS, and COVID-19.

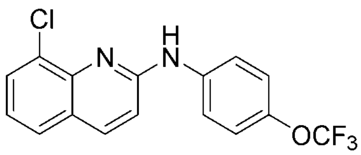

**ABX464**

However, ABX464 still suffers from poor physicochemical properties and unsatisfactory oral absorption, leaving significant room for improvement. Therefore, there is an urgent need in the field to develop novel miR-124 inducers with higher potency, improved metabolic stability, and/or enhanced safety profiles.

### SUMMARY OF THE INVENTION

The objetive of the present invention is to provide a novel miR-124 inducer with profiles of potent activity, good

metabolic stability, and/or good safety, as well as preparation therefor and uses therof.

In the first aspect of the present invention, it provides a kind of compounds as shown in Formula (1), or an isomer, crystal form, pharmaceutically acceptable salt, hydrate, or solvate thereof:

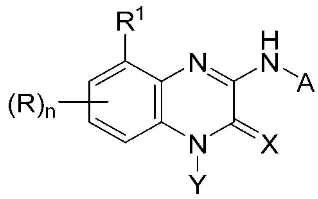

**(1)**

wherein:

n is 0, 1, 2 or 3;
each R is independently selected from the group consisting of: halogen, CN, nitro, $C_{1-3}$ alkyl, $C_{1-3}$ haloalkyl, $C_{1-3}$ alkoxy or $C_{1-3}$ haloalkoxy;
$R^1$ is selected from the group consisting of: H, CN, $C_{1-3}$ alkyl, $C_{1-3}$ alkoxy or halogen;
A is selected from the group consisting of: 6-10 membered aryl, 5-7 membered heteroaryl, benzo-5-7-membered cycloalkyl or benzo 5-7-membered heterocyclyl, wherein the aryl, heteroaryl, benzo cycloalkyl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$ haloalkoxy, $C_{1-3}$ haloalkylthio or $SF_5$;
X is O, and Y is $C_{1-3}$ alkyl, $C_{1-3}$ haloalkyl or $C_{3-6}$ cycloalkyl; or
X and Y are taken together with the atoms to which they are bound to form a 5-membered heteroaryl or a 5-membered heterocyclyl, wherein the heteroaryl and heterocyclyl can be further substituted by 0-2 substituents of the following groups: $C_{1-3}$ alkyl or $C_{1-3}$ haloalkyl.

In another preferred embodiment, wherein at least one of R and $R^1$ is halogen.

In another preferred embodiment, wherein each R is independently selected from the group consisting of: F, Cl, Br, Me, Et, OMe, OEt, $CF_3$, CN, $OCF_3$ or $NO_2$.

In another preferred embodiment, wherein $R^1$ is selected from the group consisting of: H, CN, Me, Et, OMe, OEt, F, Cl, or Br.

In another preferred embodiment, wherein A is selected from the group consisting of:

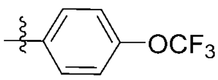 , 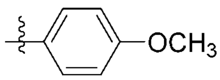 , 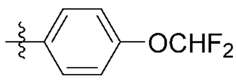 , 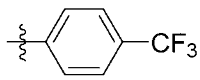 ,

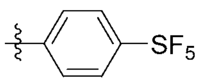 , 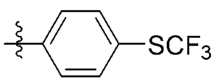 , 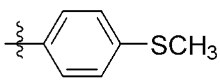 , 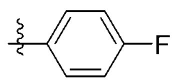 , 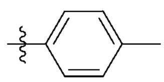 ,

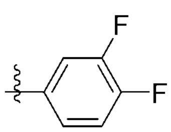 , 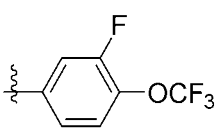 , 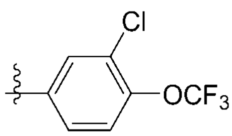 , 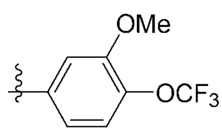 , 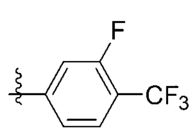 ,

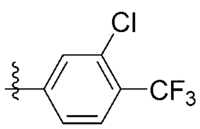 , 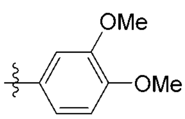 , 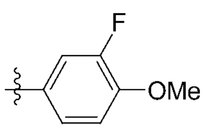 , 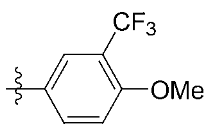 , 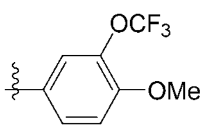 ,

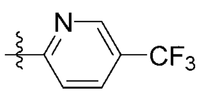, 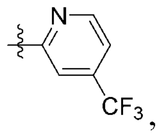, 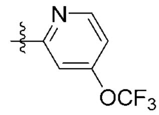, 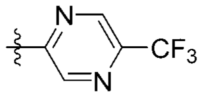, 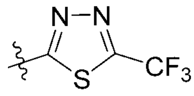, 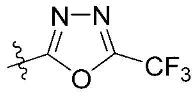,

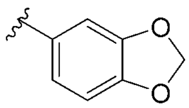, 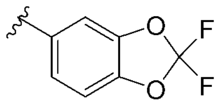, 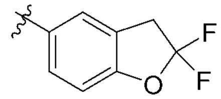, 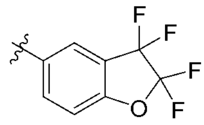, 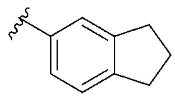,

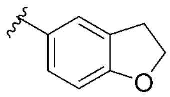, 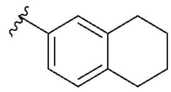, 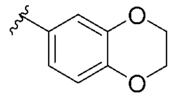 or 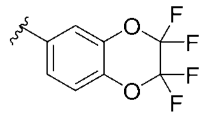.

In another preferred embodiment, the compound has the structure as shown in Formula (1A):

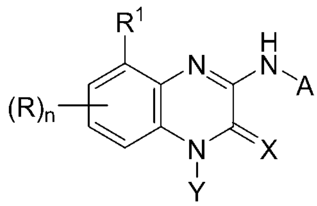

**(1A)**

wherein:

n is 0, 1 or 2;
R is selected from the group consisting of: halogen, CN, nitro, $C_{1-3}$ alkyl, $C_{1-3}$ haloalkyl or $C_{1-3}$ alkoxy;
$R^1$ is a halogen;
A is selected from the group consisting of: 6-10 membered aryl, 5-7 membered heteroaryl or benzo-5-7-membered heterocyclyl, wherein the aryl, heteroaryl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$ haloalkoxy, $C_{1-3}$ haloalkylthio or $SF_5$;
X is O, and Y is $C_{1-3}$ alkyl, $C_{1-3}$ haloalkyl or $C_{3-6}$ cycloalkyl; or
X and Y are taken together with the atoms to which they are bound to form a 5-membered heteroaryl or a 5-membered heterocyclyl, wherein the heteroaryl and heterocyclyl can be further substituted by 0-2 substituents of the following groups: $C_{1-3}$ alkyl or $C_{1-3}$ haloalkyl.

In another preferred embodiment, wherein

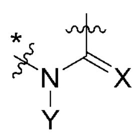

has a structure selected from the group consisting of:

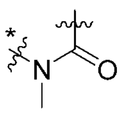, 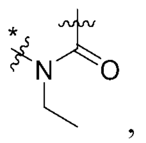, 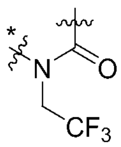, 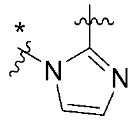, 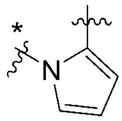,

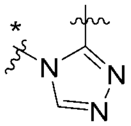, 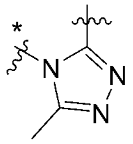, 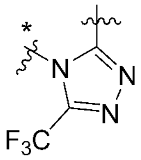, 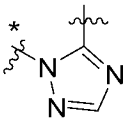, 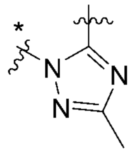 or 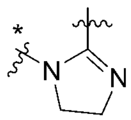;

wherein "*" represents the position connected to the benzene ring.

In another preferred embodiment, the compound has a structure selected from the group consisting of:

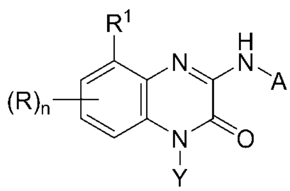

**(1A-1)**

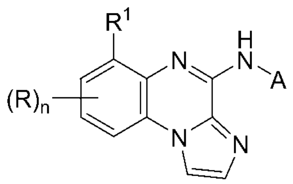

**(1A-2)**

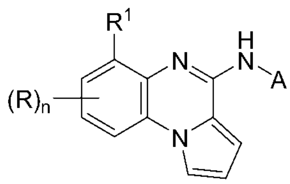

**(1A-3)**

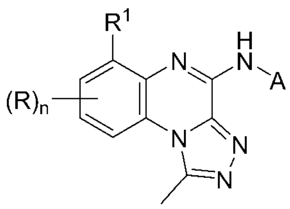

**(1A-4)**

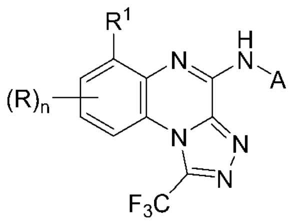

**(1A-5)**

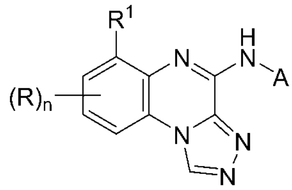

**(1A-6)**

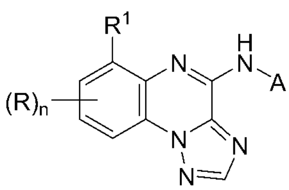

**(1A-7)**

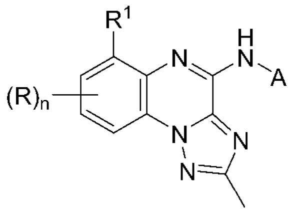

**(1A-8)**

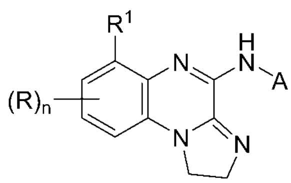

**(1A-9)**

Wherein n, Y, R, $R^1$ and A are as described in the first aspect of the present invention.

In another preferred embodiment, the compound has the structure as shown in Formula (1B):

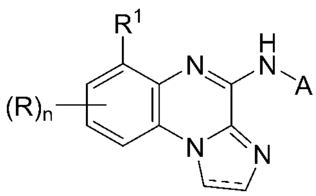

**(1B)**

wherein:

"- - -" indicates a single bond or a double bond;

n is 1, 2 or 3;

each R is independently selected from the group consisting of: halogen, CN, $C_{1-3}$ alkyl, $C_{1-3}$ haloalkyl, $C_{1-3}$ alkoxy or $C_{1-3}$ haloalkoxy;

$R^1$ is selected from the group consisting of: H, CN, $C_{1-3}$ alkyl or $C_{1-3}$ alkoxy;

A is selected from the group consisting of: 6-10 membered aryl, 5-7 membered heteroaryl, benzo 5-7 membered

cycloalkyl or benzo 5-7-membered heterocyclyl, wherein the aryl heteroaryl, benzo-cycloalkyl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$ haloalkoxy, $C_{1-3}$ haloalkylthio or $SF_5$.

In another preferred embodiment, the compound has the structure as shown in Formula (2):

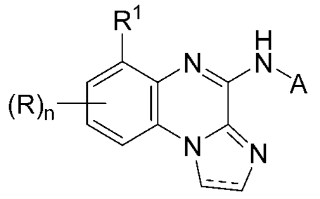

(2)

wherein:

"- - -" indicates a single bond or a double bond;
n is 0, 1 or 2;
$R^1$ is selected from the group consisting of: H, F, Cl or Br;
each R is independently selected from the group consisting of: F, Cl or Br.

In another preferred embodiment, wherein A is selected from the group consisting of:

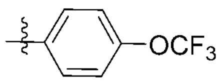,
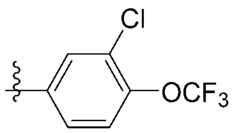 or
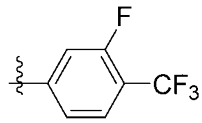.

In another preferred embodiment, wherein n is 1 or 2 when $R^1$ is H.

In another preferred embodiment, wherein the compound has a structure selected from the group consisting of:

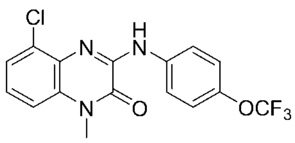

**1**

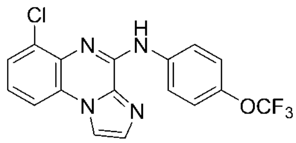

**2**

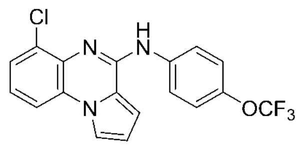

**3**

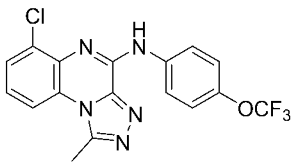

**4**

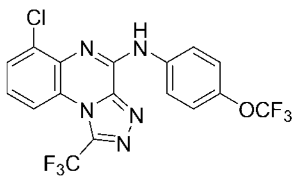

**5**

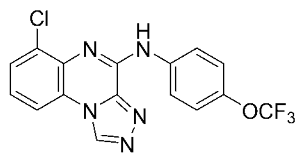

**6**

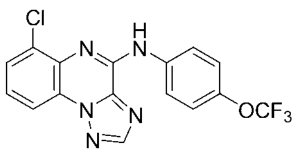

**7**

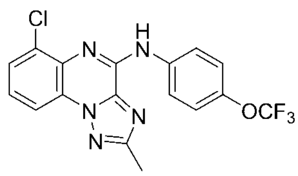

**8**

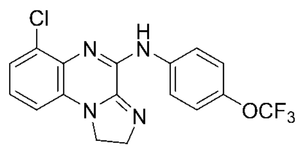

**9**

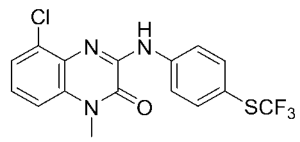
**10**

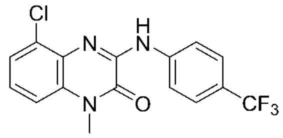
**11**

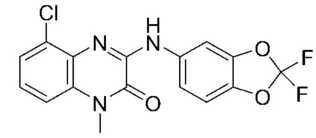
**12**

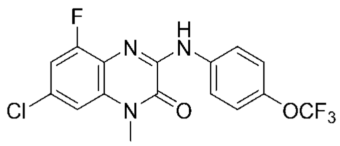
**13**

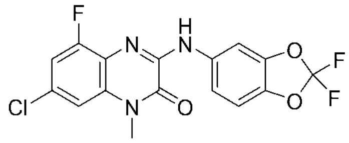
**14**

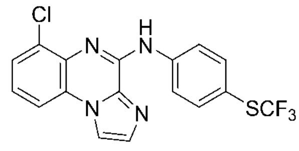
**15**

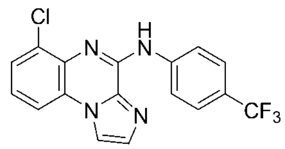
**16**

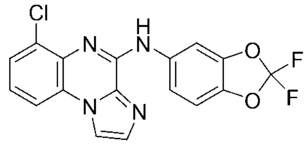
**17**

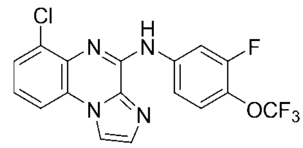
**18**

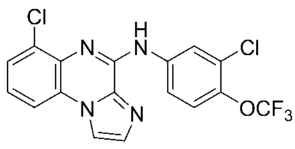
**19**

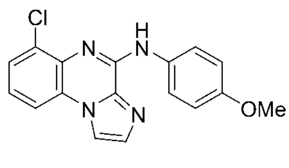
**20**

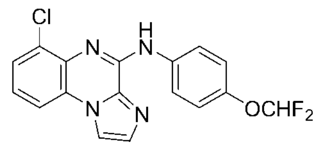
**21**

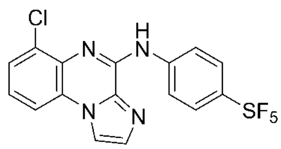
**22**

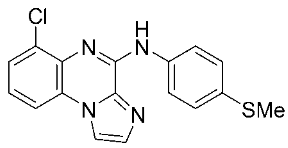
**23**

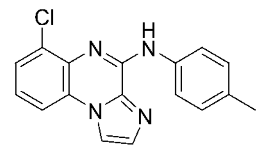
**24**

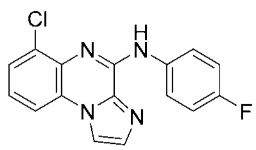
**25**

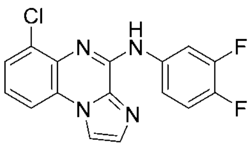
**26**

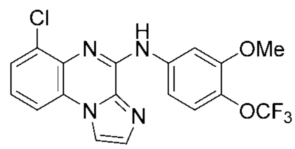
**27**

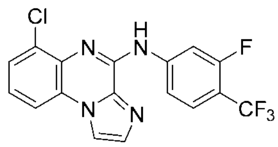
**28**

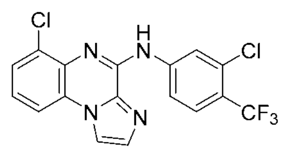
**29**

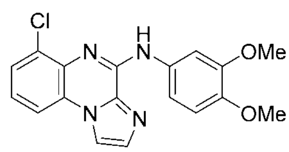
**30**

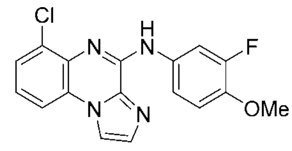

**31**

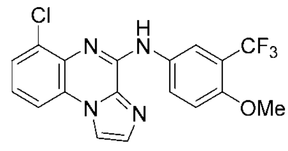

**32**

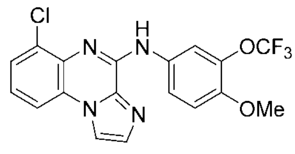

**33**

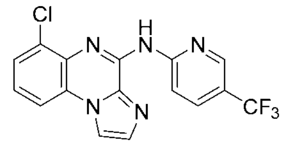

**34**

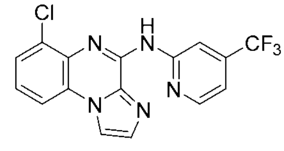

**35**

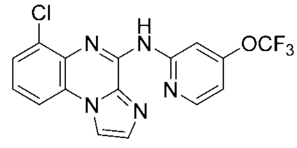

**36**

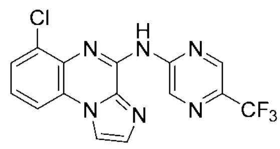

**37**

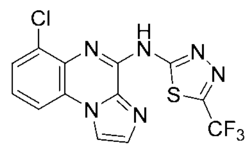

**38**

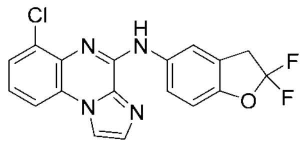

**39**

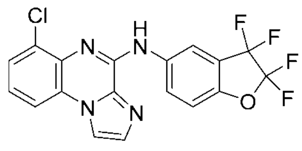

**40**

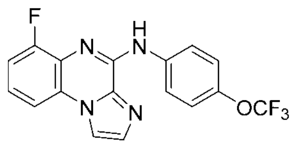

**41**

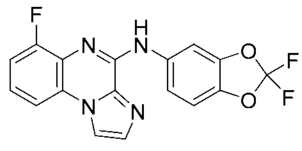

**42**

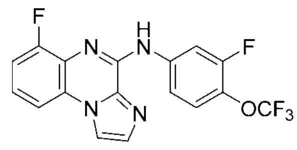

**43**

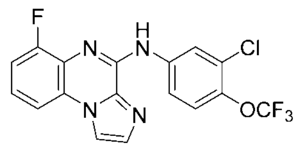

**44**

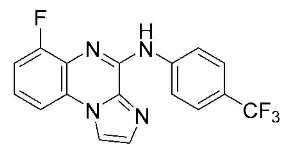

**45**

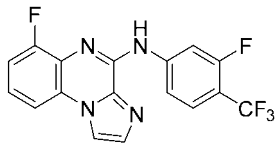

**46**

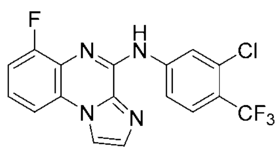

**47**

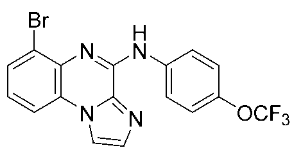

**48**

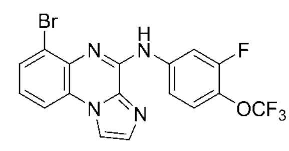

**49**

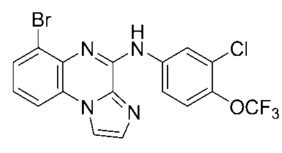

**50**

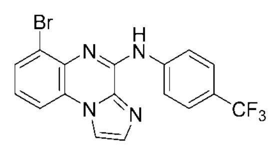

**51**

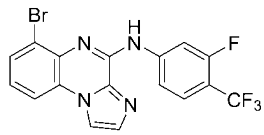

52

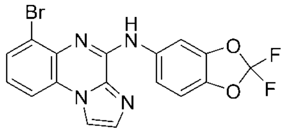

53

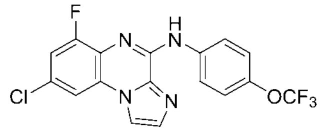

54

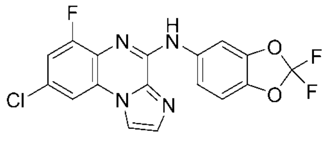

55

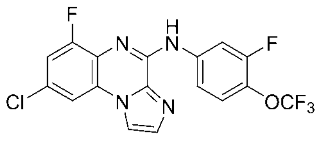

56

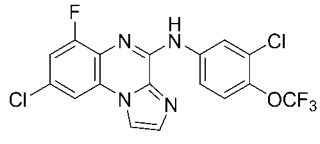

57

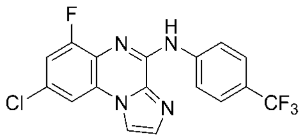

58

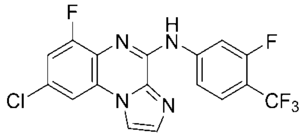

59

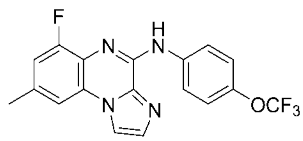

60

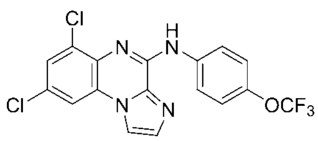

61

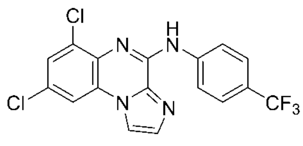

62

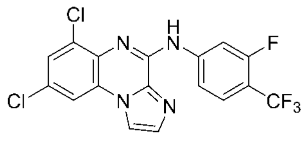

63

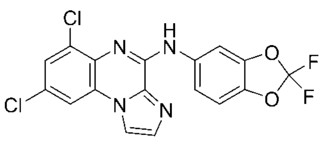

64

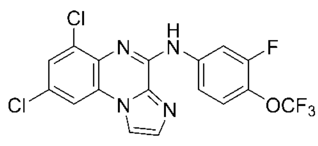

65

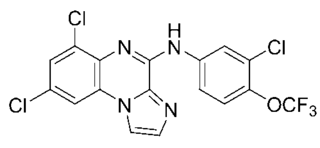

66

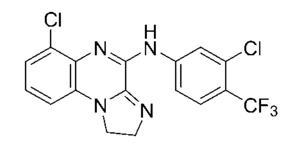

67

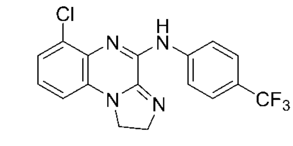

68

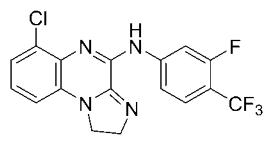

69

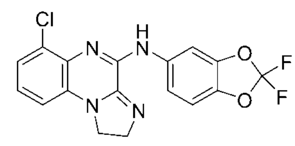

70

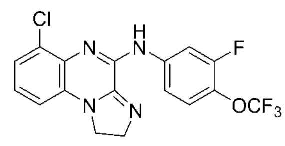

71

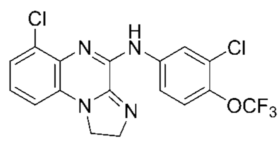

72

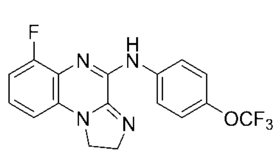

73

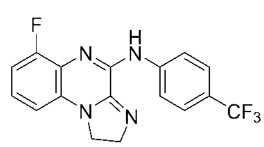

74

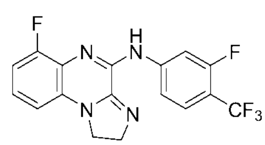

75

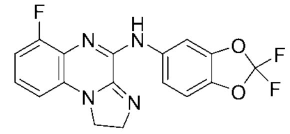

**76**

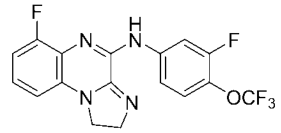

**77**

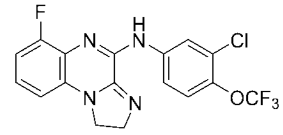

**78**

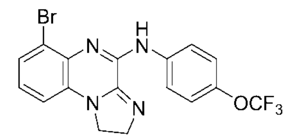

**79**

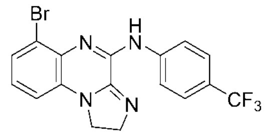

**80**

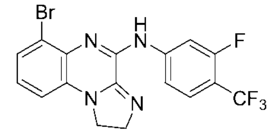

**81**

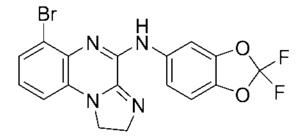

**82**

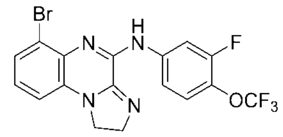

**83**

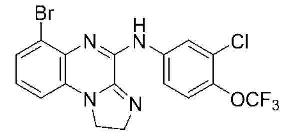

**84**

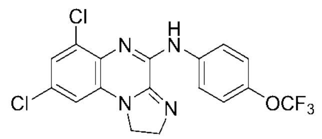

**85**

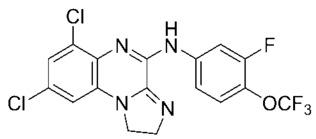

**86**

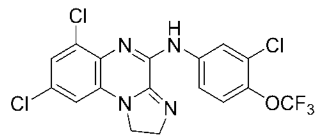

**87**

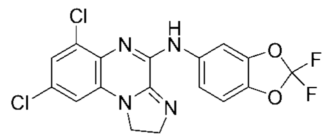

**88**

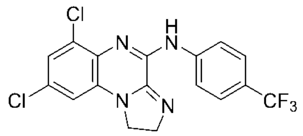

**89**

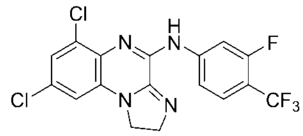

**90**

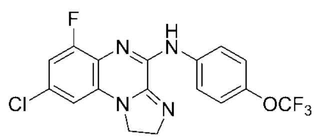

**91**

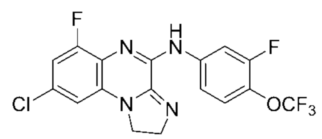

**92**

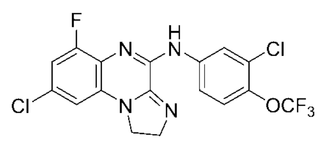

**93**

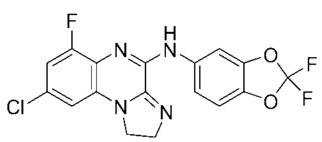

**94**

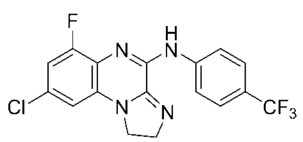

**95**

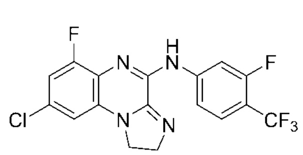

**96**

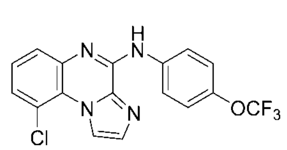

**B1**

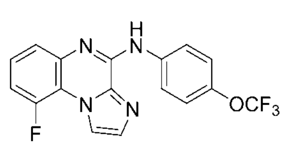

**B2**

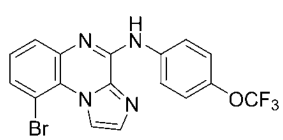

**B3**

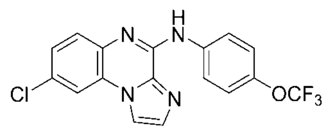

**B4**

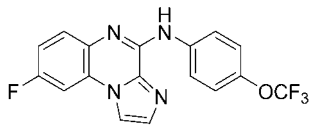

**B5**

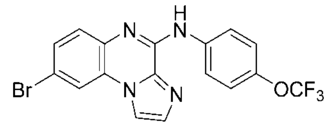

**B6**

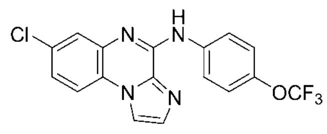

**B7**

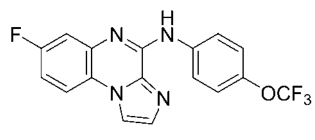

**B8**

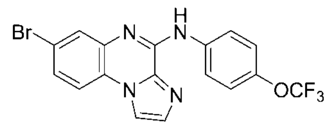

**B9**

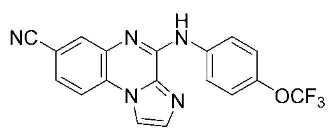

**B10**

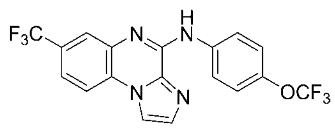

**B11**

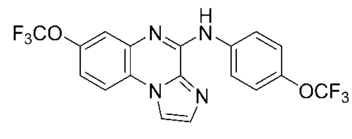

**B12**

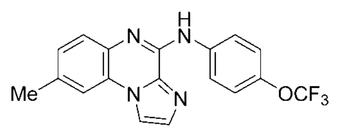

**B13**

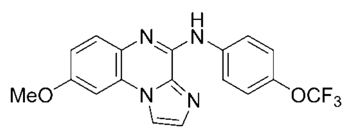

**B14**

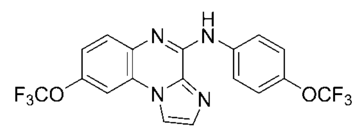

**B15**

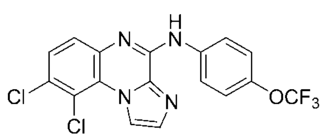

**B16**

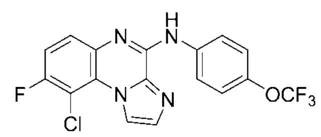

**B17**

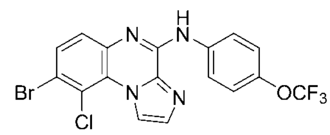

**B18**

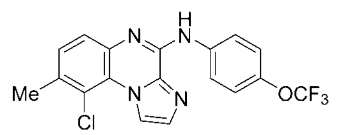

**B19**

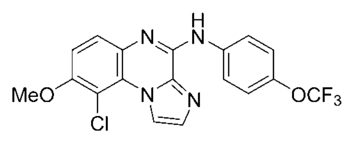

**B20**

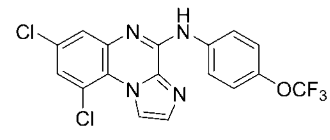

**B21**

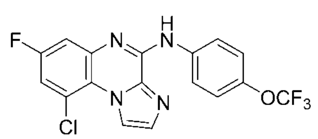

**B22**

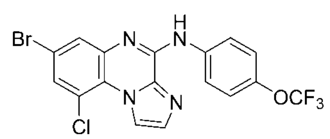

**B23**

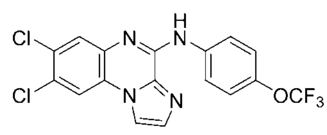

**B24**

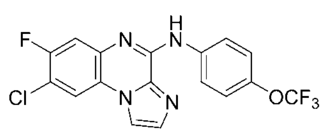

**B25**

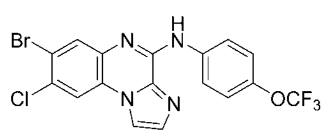

**B26**

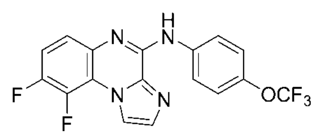

**B27**

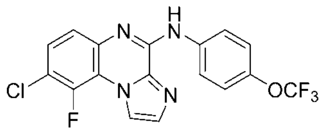

**B28**

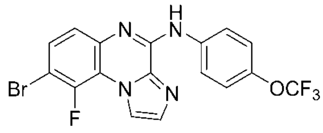

**B29**

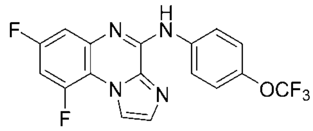

**B30**

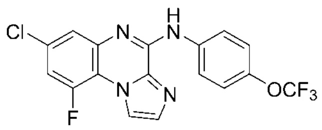

**B31**

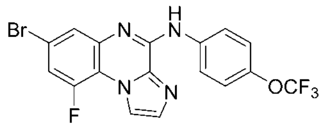

**B32**

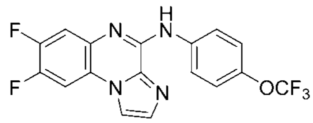

**B33**

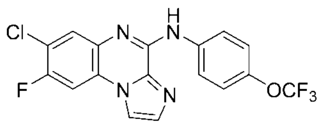

**B34**

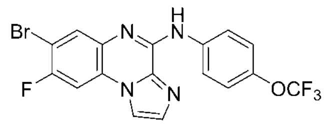

**B35**

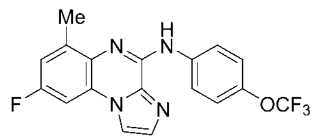

**B36**

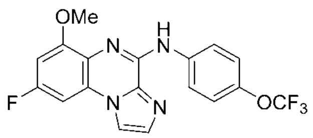

**B37**

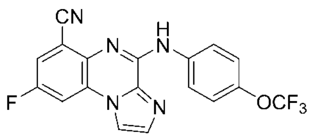

**B38**

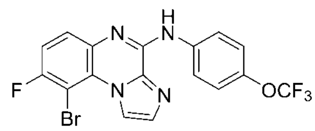

**B39**

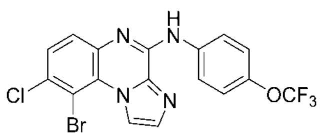

**B40**

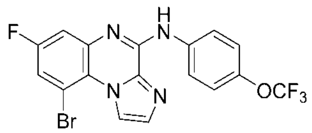

**B41**

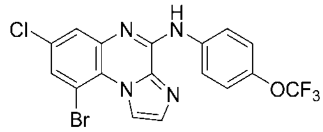

**B42**

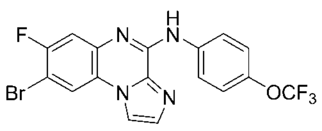

**B43**

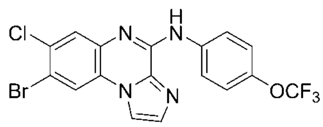

**B44**

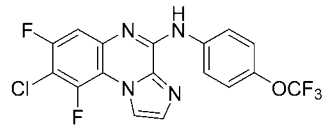

**B45**

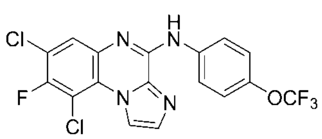

**B46**

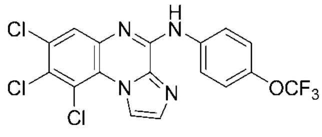

**B47**

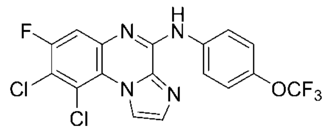

**B48**

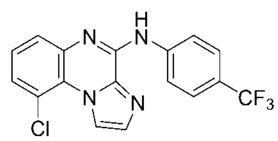

**B49**

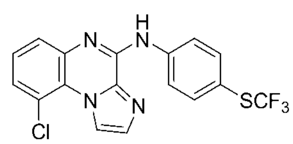

**B50**

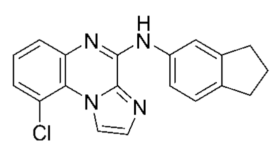

**B51**

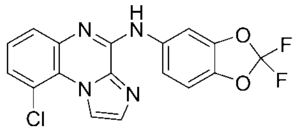

B52

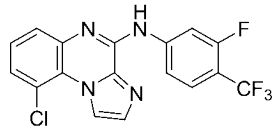

B53

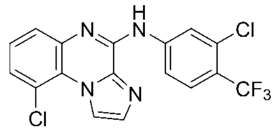

B54

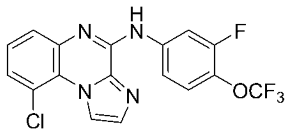

B55

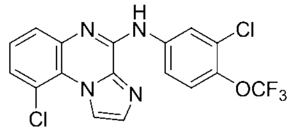

B56

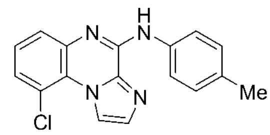

B57

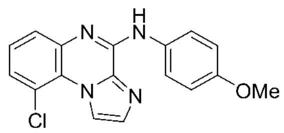

B58

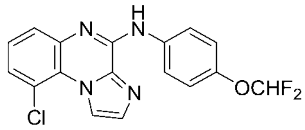

B59

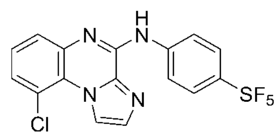

B60

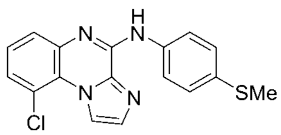

B61

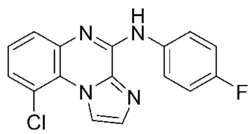

B62

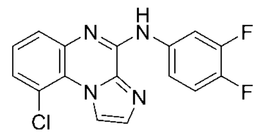

B63

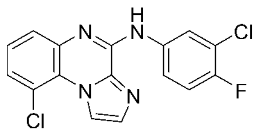

B64

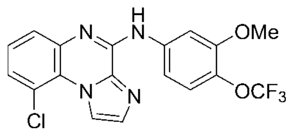

B65

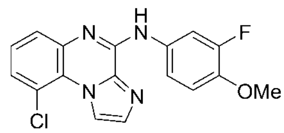

B66

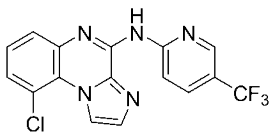

B67

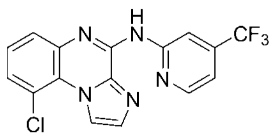

B68

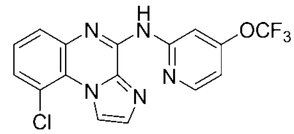

B69

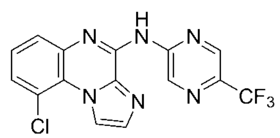

B70

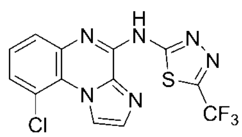

B71

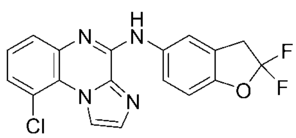

B72

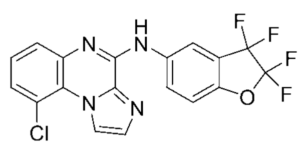

B73

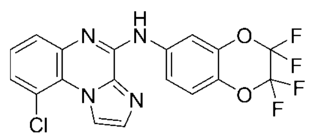

B74

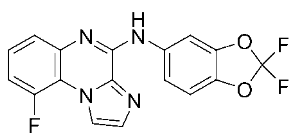

B75

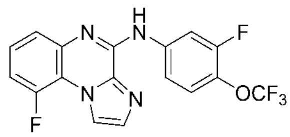

**B76**

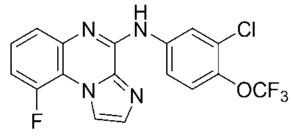

**B77**

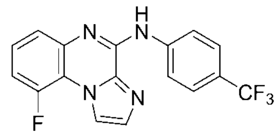

**B78**

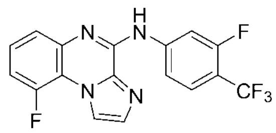

**B79**

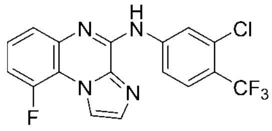

**B80**

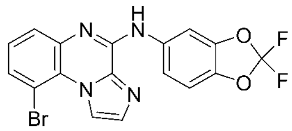

**B81**

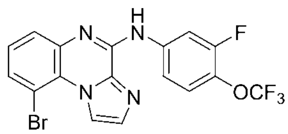

**B82**

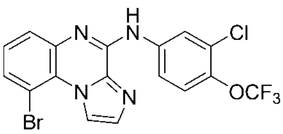

**B83**

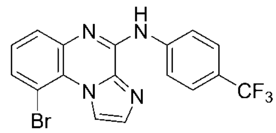

**B84**

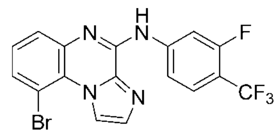

**B85**

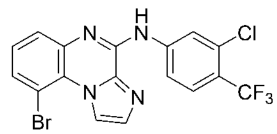

**B86**

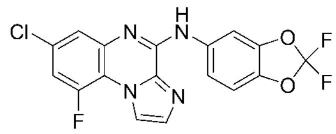

**B87**

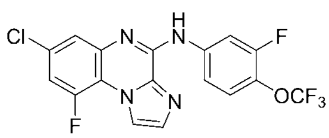

**B88**

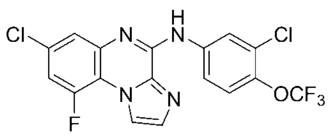

**B89**

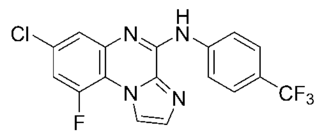

**B90**

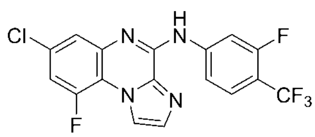

**B91**

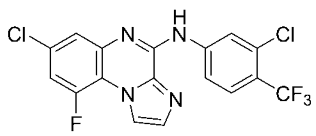

**B92**

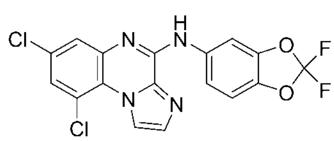

**B93**

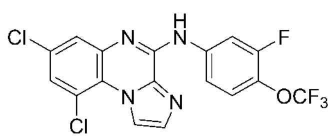

**B94**

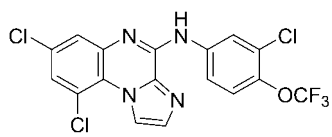

**B95**

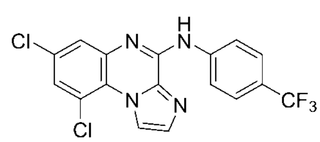

**B96**

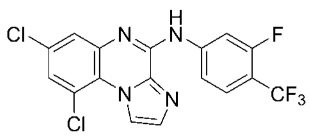

**B97**

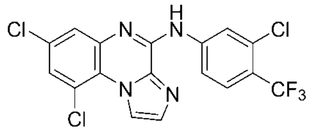

**B98**

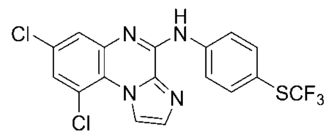

**B99**

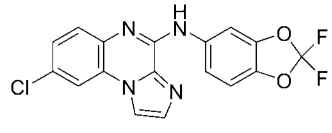
**B100**

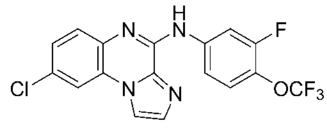
**B101**

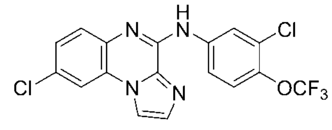
**B102**

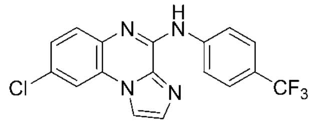
**B103**

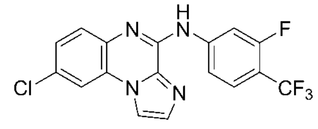
**B104**

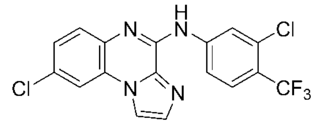
**B105**

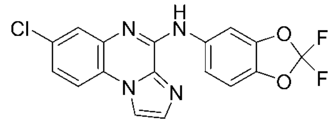
**B106**

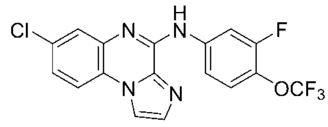
**B107**

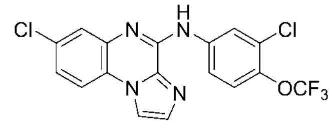
**B108**

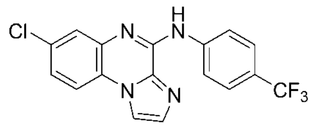
**B109**

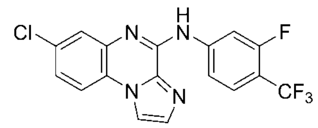
**B110**

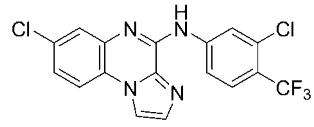
**B111**

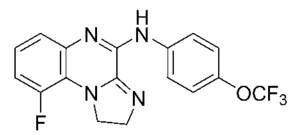
**B112**

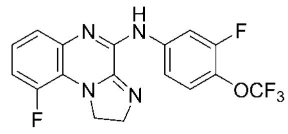
**B113**

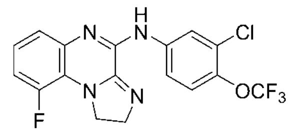
**B114**

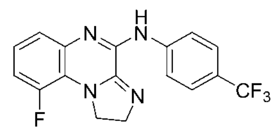
**B115**

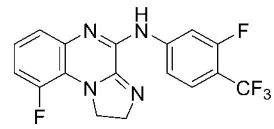
**B116**

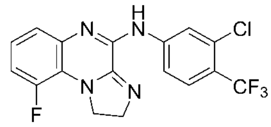
**B117**

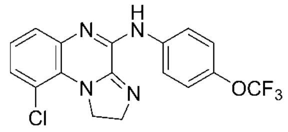
**B118**

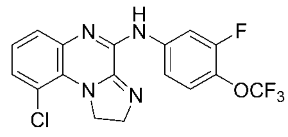
**B119**

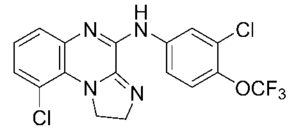
**B120**

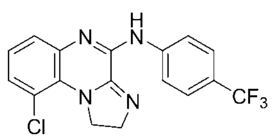
**B121**

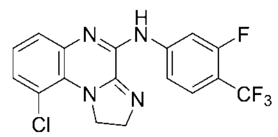
**B122**

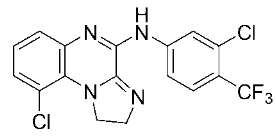
**B123**

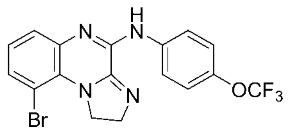

**B124**

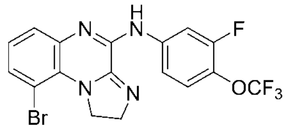

**B125**

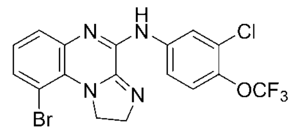

**B126**

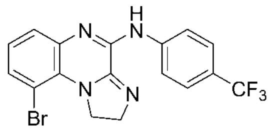

**B127**

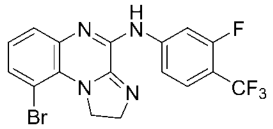

**B128**

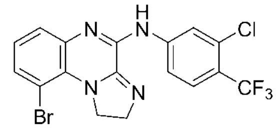

**B129**

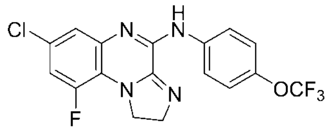

**B130**

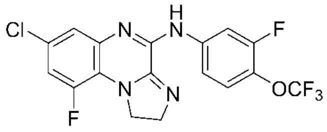

**B131**

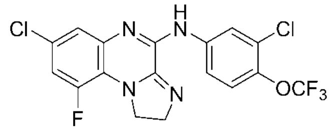

**B132**

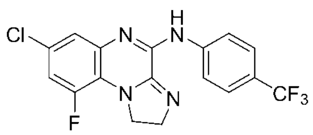

**B133**

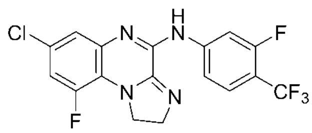

**B134**

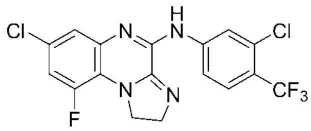

**B135**

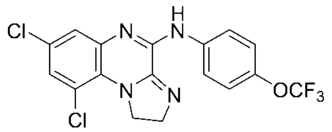

**B136**

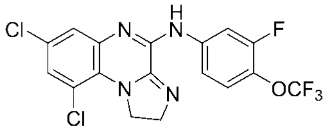

**B137**

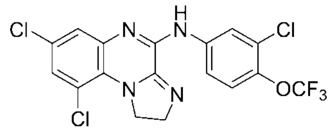

**B138**

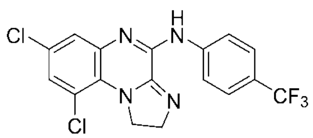

**B139**

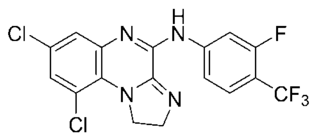

**B140**

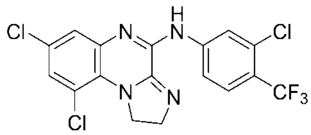

**B141**

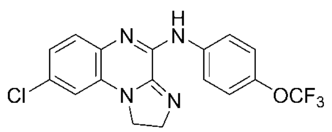

**B142**

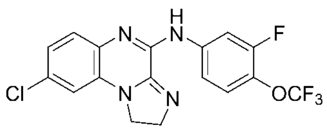

**B143**

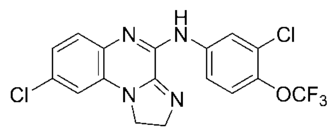

**B144**

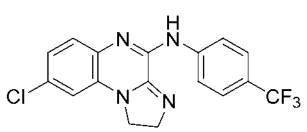

**B145**

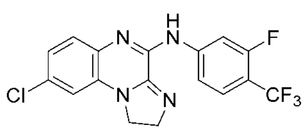

**B146**

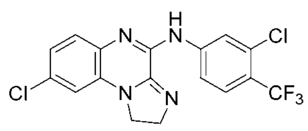

**B147**

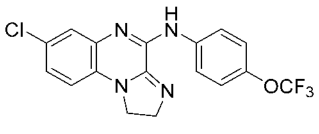

B148

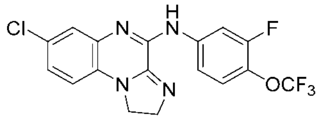

B149

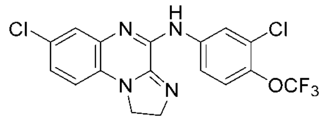

B150

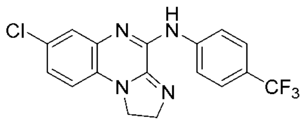

B151

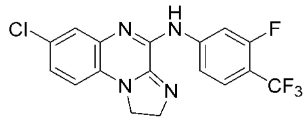

B152

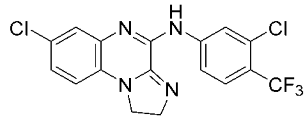

B153

.

In the second aspect of the present invention, it provides a pharmaceutical composition for treating, regulating, and/or preventing a disease associated with miR-124, characterized in that the pharmaceutical composition comprises:

(1) the compound as an active ingredient according to any one of claims 1-13, or an isomer, crystal form, pharmaceutically acceptable salt, hydrate, or solvate thereof; and

optionally (2) a pharmaceutically acceptable excipient, or carrier.

In the third aspect of the present invention, it provides use of the compound as described in the first aspect of the present invention, or a isomer, crystal form, pharmaceutically acceptable salt, hydrate, or solvate thereof, or the pharmaceutical composition as described in the second aspect of the present invention for preparing a pharmaceutical composition for treating, regulating, and/or preventing a disease mediated by miR-124.

In another preferred embodiment, wherein the disease mediated by miR-124 is selected from the group consisting of: Alzheimer's disease, Parkinson's disease, brain tumor, gastric cancer, liver cancer, lung cancer, colorectal cancer, pancreatic cancer, breast cancer, cervical cancer, endometrial cancer, prostate cancer, Crohn's disease, ulcerative colitis, rheumatoid arthritis, allergic rhinitis, osteoarthritis, fibrosis disease, AIDS and/or COVID-19.

In the forth aspect of the present invention, it provides a method for preparing a compound of Formula (1), characterized in that the method includes the steps of:

(1) in an inert solvent, the compound of formula (A) and its tautomer react to obtain the compound of formula (B):

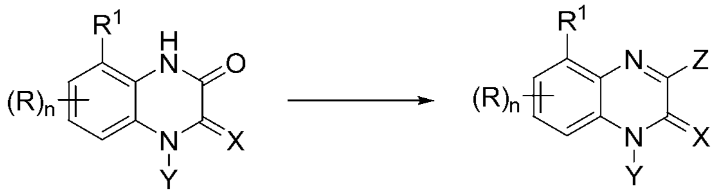

A B ;

(2) in an inert solvent, the compound of formula (B) reacts with the compound of formula (d) to obtain the compound of formula (1):

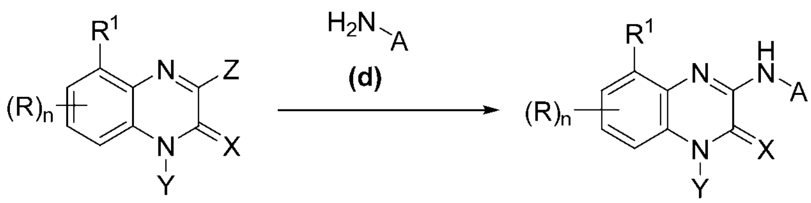


B (1) ;

wherein Z is a halogen or OTf; X, Y, R, $R^1$, A and n are as defined in the first aspect of the present invention.

In another preferred embodiment, wherein the compound of formula (1) is a compound of formula (1a) or (1b),

and the prepartion method includes the following steps:

(s1) in an inert solvent, the compound of formula (A1) and its tautomer react to obtain the compound of formula (B1):

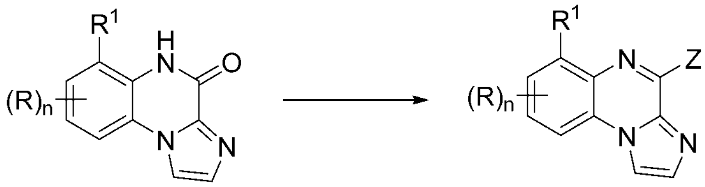

A1 B1 ;

(s2) in an inert solvent, the compound of formula (B1) reacts with the compound of formula (d) to obtain the compound of formula (1a):
wherein Z is a halogen or OTf; R, $R^1$, A and n are as defined in the first aspect of the present invention.

In another preferred embodiment, the prepartion method includes the following steps:

(t1) in an inert solvent, the compound of formula (C) undergoes a cyclization reaction to yield the compound of formula (D):

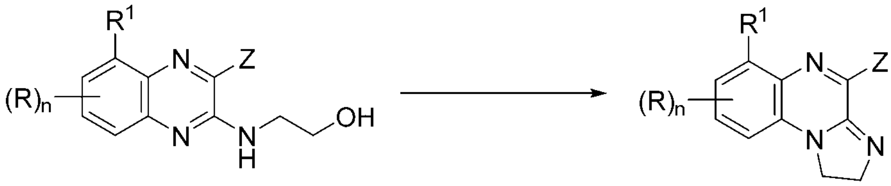

C D ;

(t2) in an inert solvent, the compound of formula (D) reacts with the compound of formula (d) to obtain the compound of formula (1b):

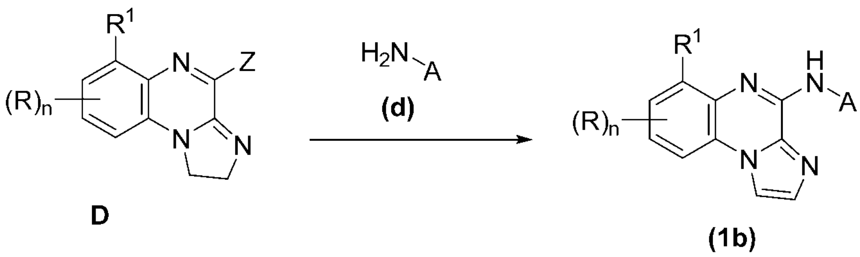


D (1b)

wherein Z is a halogen or OTf; R, $R^1$, A and n are as defined in the first aspect of the present invention.

In the fifth aspect of the present invention, it provides a method for treating, regulating, and/or preventing a disease mediated by miR-124, which includes the steps of: administering to an individual in need thereof an amount of the compound as described in the first aspect of the present invention, or an isomer, crystal form, pharmaceutically acceptable salt, hydrate, or solvate thereof, or the pharmaceutical composition described in the second aspect of the present invention.

In another preferred embodiment, the individual includes humans and non-human mammals.

It should be understood that within the scope of the present invention, all the technical features of the present invention and those specifically described in the following text (e.g., examples) can be combined with each other to form new or preferred technical solutions. Due to the limited space, we will not enumerate them one by one here.

## FIRURE DESCRIPTION

Figure 1 shows the body weight changes of mice over time after the administration of the compound of each experimental group.

## DETAILED DESCRIPTION OF THE ILLUSTRATED EMBODIMENTS

The inventor, after extensive and intensive research, and through a large number of screenings and tests, first discovered a kind of compounds of Formula (1), which have a significant therapeutic effect on diseases mediated by miR-124. The compounds of the present invention have a potent ability to induce the expression of miR-124 in PBMC cells stimulated by PMA/IO, and the compounds have excellent pharmacokinetic properties. At the same time, the compounds have significant *in vivo* activity and good pharmaceutical properties. On this basis, the present invention was completed.

## THE COMPOUND OF THE PRESENT INVENTION AND ITS SYNTHESIS

The present invention provides an inducer of miR-124, i.e., a compound of Formula (1), or an isomer, crystal form, pharmaceutically acceptable salt (inorganic salt or organic salt), hydrate, or solvate thereof. Preferably, the compound of the present invention is as described in the first aspect of the present invention.

The present invention also provides a method for preparing the compound of Formula (1) of the present invention. The following specifically describes the method for preparing the compound of Formula (1) of the present invention, but these specific methods do not constitute any limitation on the present invention.

In one aspect, the compounds described herein are prepared according to the well-known methods. However, the conditions of the process, such as reactants, solvents, bases, amounts of compounds used, reaction temperature, time required for the reactions, etc., are not limited to the following explanations. The compounds of the present invention may also be conveniently prepared, optionally in combination with various synthesis methods described in this specification or well-known in the art, and such combinations are readily carried out by those skilled technicians in the art. On the other aspect, the present invention also provides the preparation methods of the compounds represented by the general formula (1A) or the general formula (1B), which are prepared by the following Schemes 1-9:

**Scheme 1:**

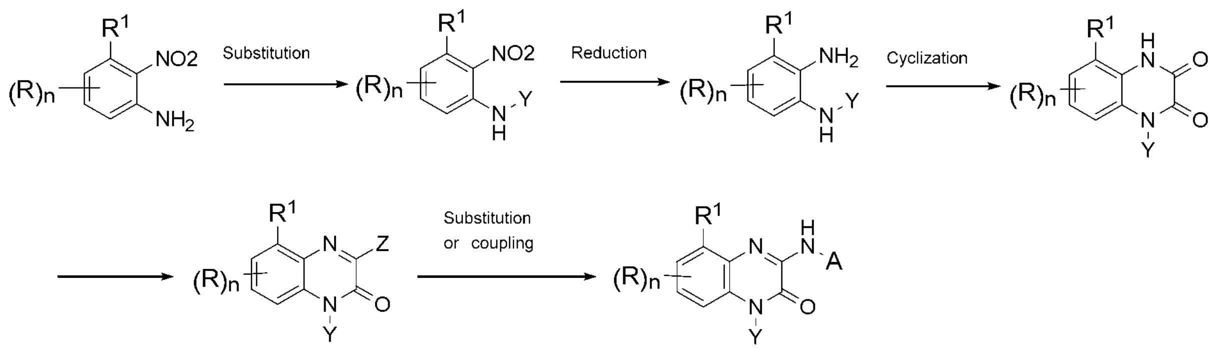


The embodiments of the compound of Formula (1) can be prepared according to the scheme 1, wherein Z is a halogen or OTf, and R, $R^1$, Y, A and n are as defined above.

## Scheme 2:

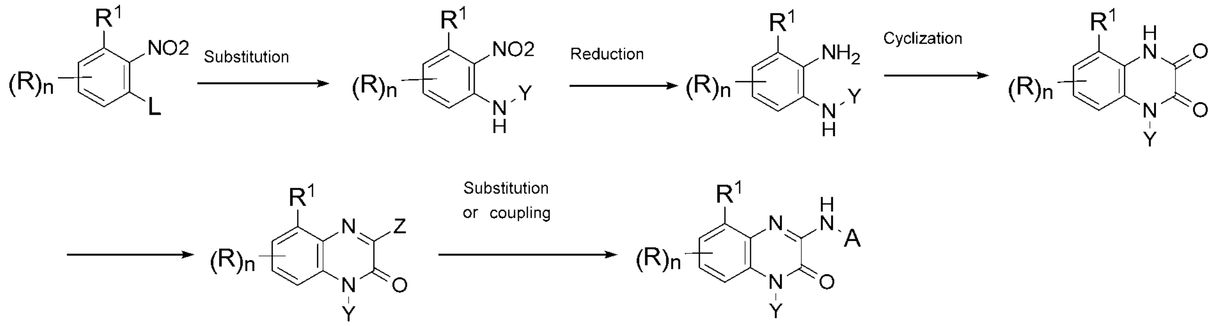


The embodiments of the compound of Formula (1) can be prepared according to the scheme 2, wherein L is a halogen, Z is a halogen or OTf, and R, $R^1$, Y, A and n are as defined above.

## Scheme 3:

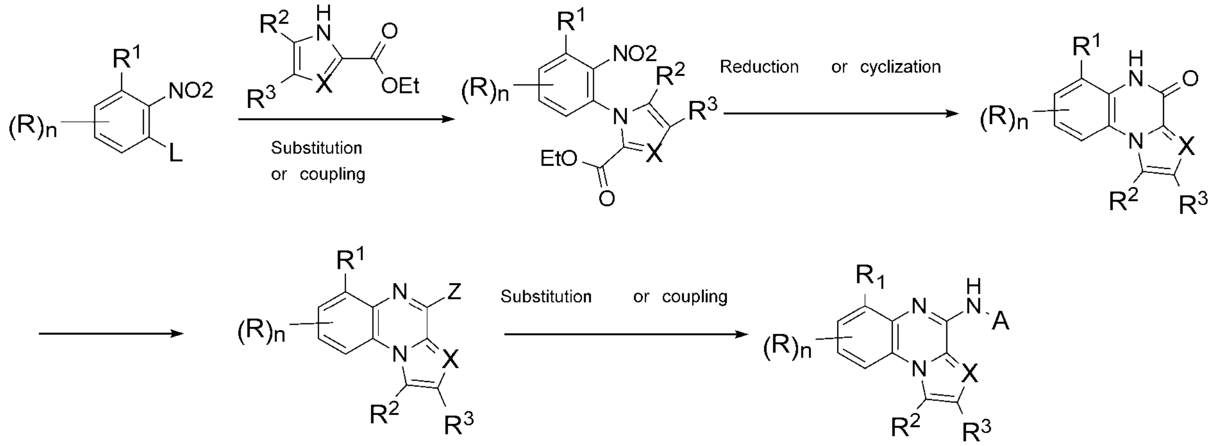


The embodiments of compounds of Formula (1A) or Formula (1B) can be prepared according to the scheme 3, wherein L is a halogen, X is N or CH, Z is a halogen or OTf, $R^2$ and $R^3$ are independently selected from H, $C_{1-3}$ alkyl or $C_{1-3}$ haloalkyl, and R, $R^1$, A and n are as defined above.

## Scheme 4:

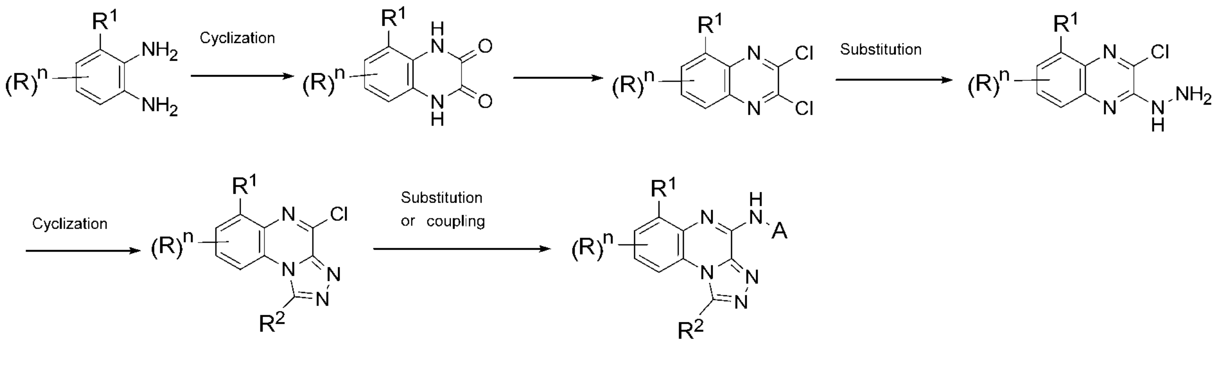


The embodiments of the compound of Formula (1) can be prepared according to the scheme 4, wherein $R^2$ is H, $C_{1-3}$ alkyl or $C_{1-3}$ haloalkyl, and R, $R^1$, Y, A and n are as defined above.

## Scheme 5:

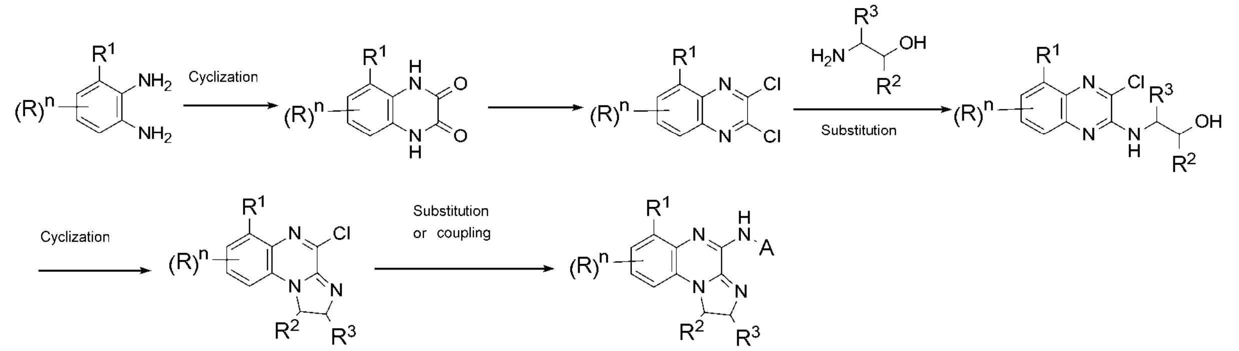


The embodiments of the compound of Formula (1) can be prepared according to the scheme 5, wherein $R^2$ and $R^3$ are independently selected from H, $C_{1-3}$ alkyl or $C_{1-3}$ haloalkyl, and R, $R^1$, A and n are as defined above.

## Scheme 6:

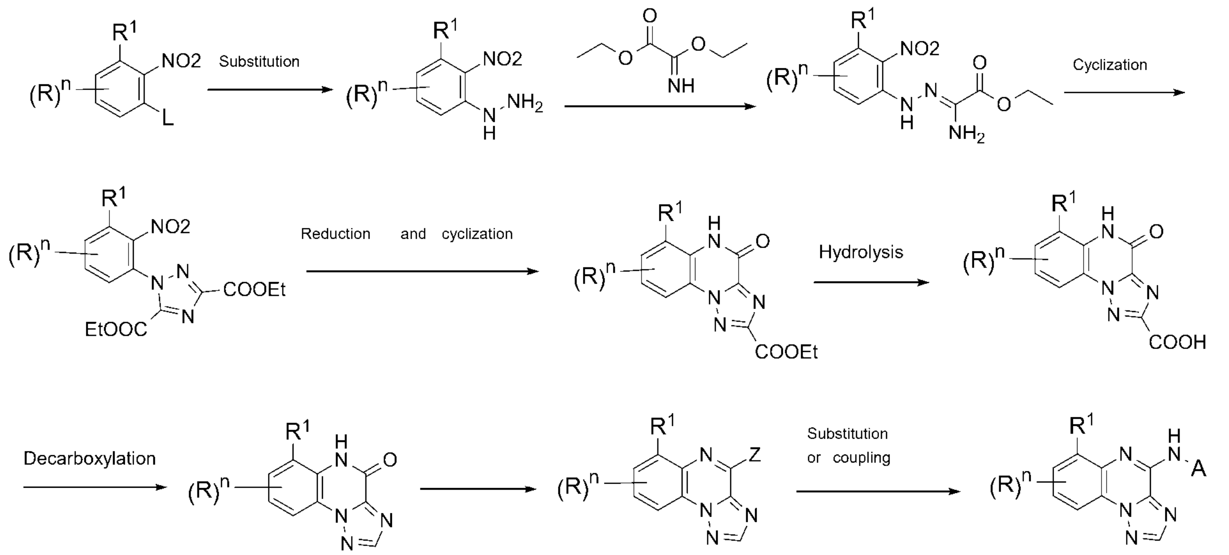


The embodiments of the compound of Formula (1) can be prepared according to the scheme 6, wherein L is a halogen, Z is a halogen or OTf, and R, $R^1$, Y, A and n are as defined above.

**Scheme 7:**

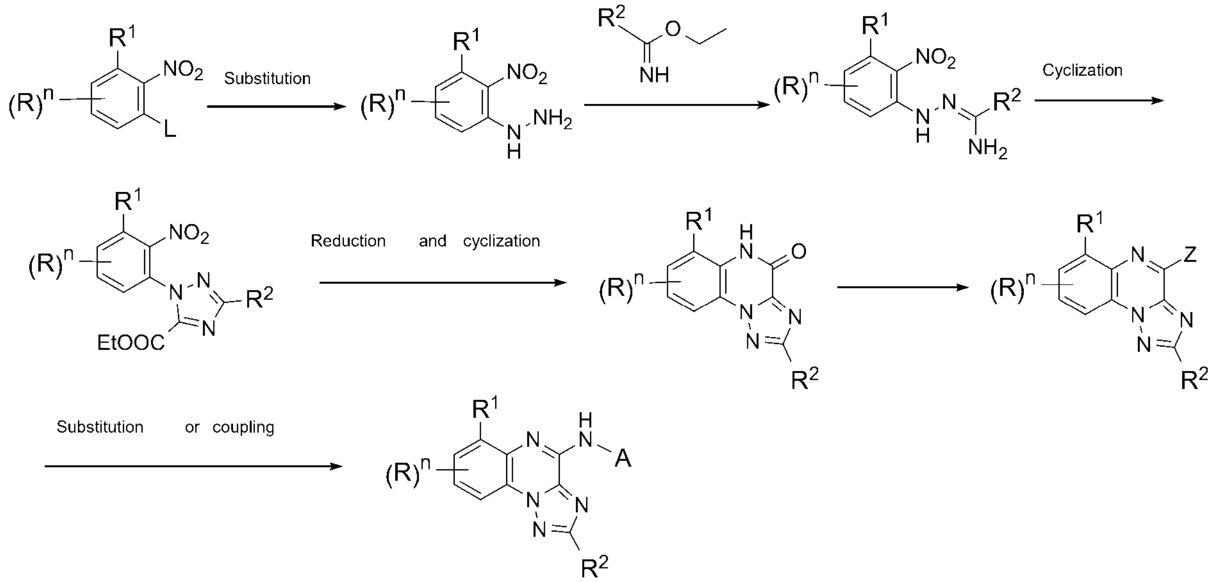


The embodiments of the compound of Formula (1) can be prepared according to the scheme 7, wherein L is a halogen, Z is a halogen or OTf, $R^2$ is $C_{1-3}$ alkyl or $C_{1-3}$ haloalkyl, and R, $R^1$, Y, A and n are as defined above.

**Scheme 8:**

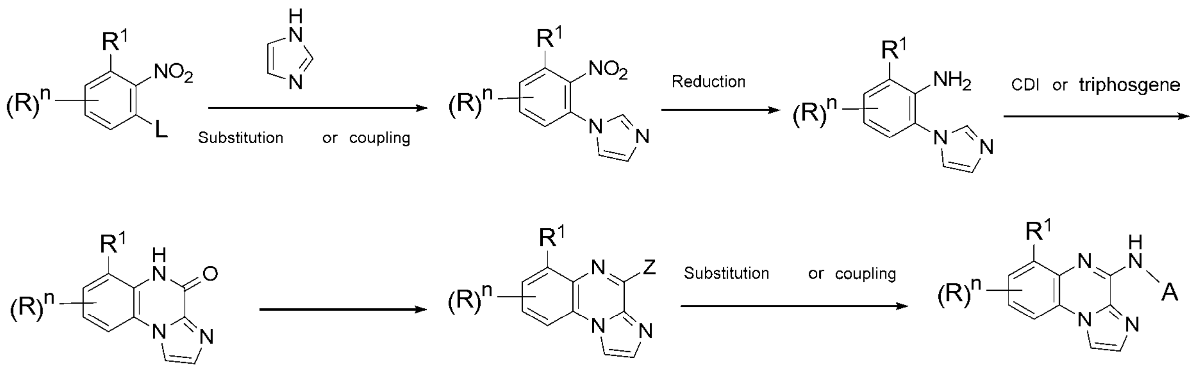


The embodiments of the compound of Formula (1B) can be prepared according to the scheme 8, wherein L is a halogen, Z is a halogen or OTf, and R, $R^1$, Y, A and n are as defined above.

**Scheme 9:**

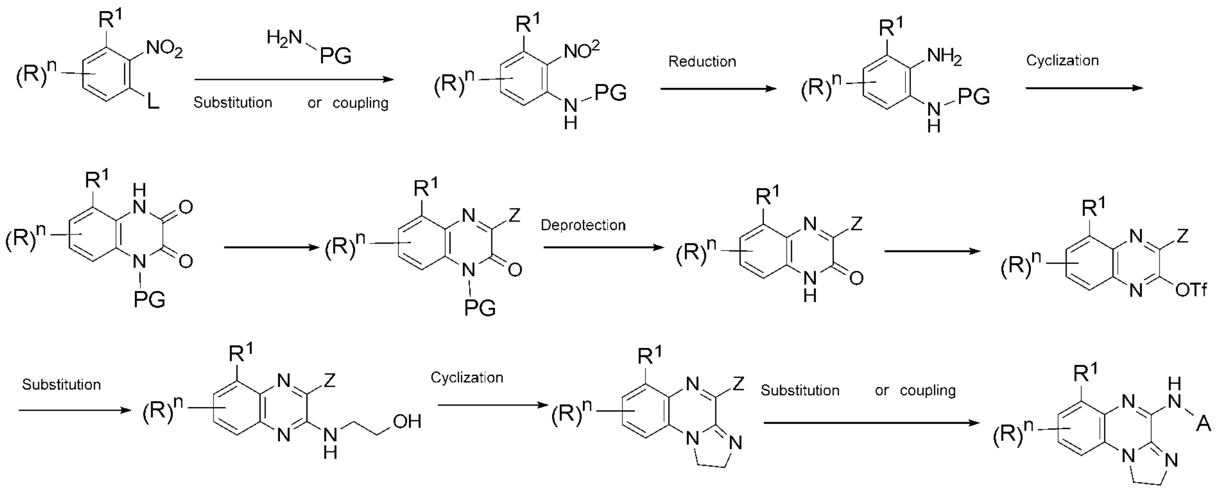


The embodiments of the compound of Formula (1B) can be prepared according to the scheme 9, wherein L is a halogen, Z is a Cl or Br, PG is an amino protecting group such as Boc, Cbz, Bn, PMB, etc., and R, $R^1$, Y, A and n are as defined above.

## Related definitions

Unless otherwise specified, the following terms and phrases used herein are intended to have the following meanings. A specific term or phrase should not be considered indefinite in the absence of a particular definition, but should be understood according to its ordinary meaning. When a trade name appears herein, it is intended to refer to its corresponding commerical product or active ingredient thereof.

The term "pharmaceutically acceptable" used herein in terms of those compounds, materials, compositions, and/or dosage forms, which are suitable for use in contact with human and animal tissues within the scope of reliable medical judgment, without excessive toxicity, irritation, allergic reactions or other problems or complications, commensurate with a reasonable benefit/risk ratio.

The term "pharmaceutically acceptable salt" refers to a salt of the compound of the present disclosure that is prepared by reacting the compound having a specific substituent of the present invention with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salts includes a salt of sodium, potassium, calcium, ammonium, organic amine or magnesium, or similar salts. When the compound of the present disclosure contains a relatively basic functional group, an acid addition salt can be obtained by contacting the compound with a sufficient amount of acid in a pure solution or a suitable inert solvent. Examples of pharmaceutically acceptable acid addition salts include an inorganic acid salt, wherein the inorganic acid include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphinic acid and the like; and an organic acid salt, wherein the organic acid includes, for example, acetic acid, propionic acid, isobutyric acid, maleic acid, malonic acid, benzoic acid, succinic acid, octanedioic acid, trans-butenedioic acid, lactic acid, mandelic acid, phthalic acid, benzenesulfonic acid, p-toluenesulfonic acid, citric acid, tartaric acid, and methanesulfonic acid, and the like; and salts of amino acids (such as arginine and the like), and a salt of organic acids such as glucuronic acid and the like. Certain specific compounds of the present invention contain both basic and acidic functional groups, thus can be converted to any base or acid addition salt.

The pharmaceutically acceptable salt of the present disclosure can be prepared from the parent compound that contains an acid or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplatess all such compounds, including cis and trans isomers, (-)- and (+)-enantiomers, (*R*)- and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror

images of each other.

Unless otherwise specified, the term "cis-trans isomer" or "geometric isomer" is caused by the inability to rotate freely double bonds or single bonds of ring -forming carbon atoms.

Unless otherwise specified, the term "diastereomer" refers to a stereoisomer in which a molecule has two or more chiral centers and the relationship between the molecules is not mirror images.

Unless otherwise specified, "(*D*)" or " (+)" refers to dextrorotation, "(*L*)" or " (-)" refers to levorotation, and "(*DL*)" or " (±)" refers to racemic.

Unless otherwise specified, the absolute configuration of a stereogenic center is represented by a wedged solid bond ( ) and a wedged dashed bond ( ), and the reative configuration of a stereogenic center is represented by a straight solid bond ( ) and a straight dashed bond ( ), a wave line ( ) is used to represent a wedged solid bond ( ) or a wedged dashed bond ( ), or the wave line ( ) is used to represent a straight solid bond ( ) or a straight dashed bond ( ).

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, then the isomer or enantiomer excess (ee value) is 80%.

Optically active (*R*)- and (*S*)-isomers, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as an amino) or an acidic functional group (such as a carboxyl), the compound reacts with an appropriate optically active acid or base to form a salt of the diastereomeric isomer which is then subjected to diastereomeric resolution through the conventional method to diastereomeric rsolution through the conventional method in the art to give the pure enantiomer. In additional, the enantiomers and diastereoisomer are generally isolated through chromatography which uses a chiral stationary phase and optionally combines with a chemical derivative method (such as carbamate generated from amine).

Compounds disclosed herein may contain unnatural proportions of atomic isotopes at one or more of the atoms that make up the compounds. For example, a compound may be labeled with a radioisotope such as tritium ($^3H$), iodine-125 ($^{125}I$) or C-14 ($^{14}C$). For another example, hydrogen can be replaced by heavy hrdrogen to form a deuterated drug. The bond between deuterium and carbon is stronger than that between ordinary hydrogen and carbon. Compared with undeuterated drugs, deuterated drugs usually have the advantages of reduced toxic side effects, increased drug stability, enhanced efficacy, and prolonged biological half-life of the drug. All changes in the isotopic compositions of compounds disclosed herein, regardless of radioactivity are included within the scope of the present disclosure.

The term "optional" or "optionally" means that the subsequent event or condition may occur but not requisite, that the term includes the instance in which the event or condition occurs and the instance in which the event or condition does not occur.

The term "substituted" refers to that one or more than one hydrogen atoms on a specific atom are substituted by a substituent, including deuterium and hydrogen variants, as long as the valence of the specific atom is normal and the substituted compound is stable. When the substituent is oxo (i.e., =O), it means that two hydrogen atoms are substituted. Positions on an aromatic ring cannot be substituted by oxo. The term "optionally substituted" means an atom can be substituted with a substitutent or not, unless otherwise specified, the type and the number of the substitutent may be arbitrary so long as being cheically achievable.

When any variable (e.g., R) occurs in the constitution or structure of the compound more than once, the definition of the variable at each occurrence is independent. Thus, for example, if a group is substituted by 0-2 R, the group can be optionally substituted by up to two R, wherein the option of R at each occurrence is independent. Moreover, the combination of the substituent and/or the variant thereof is allowed only when the combination results in a stable compound.

When the number of a linking group is 0, such as $-(CH_2)_0-$, it means that the linking group is a single bond.

When one of the variables is a single bond, it means that the two groups linked by the single bond are connected directly. For example, when L in X-L-Y represents a single bond, the structure of X-L-Y is actually X-Y.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$ and $C_{12}$, also includes any range from n to n+m, for example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$ and $C_{9-12}$, etc.; Similarly, n membered to n+m membered indicates the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11

membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, etc.

Unless otherwise specified, "$C_{1-3}$ alkyl" refers to a linear or branched saturated hydrocarbon group composed of 1 to 3 carbon atoms. The $C_{1-3}$ alkyl includes $C_{1-2}$ and $C_{2-3}$ alkyl and the like, it can be monovalent (such as methyl), divalent (such as methylene), or multivalent (such as methine). Examples of $C_{1-3}$ alkyl include but not limited to methyl, ethyl, propyl, n-propyl, isopropyl and the like. In the present disclosure, "alkyl" also intends to include substituted alkyl, that is, one or more positions in alkyl are substituted, especially by 1-4 substituents, which can be substituted at any position. "Haloalkyl" refers to alkyl defined herein in which one or more hydrogens are replaced by the same or different halogens. Examples of haloalkyl include $-CH_2Cl$, $-CH_2CF_3$, $-CH_2CCl_3$, perfluoroalkyl (for example, $-CF_3$) and the like.

Unless otherwise specified, "$C_{3-6}$ cycloalkyl" refers to a saturated cyclic hydrocarbon group composed of 3 to 6 carbon atoms, which includes monocyclic and bicyclic systems, and the $C_{3-6}$ cycloalkyl includes $C_{3-5}$, $C_{4-5}$, and $C_{5-6}$ cycloalkyl and the like, it can be monovalent, divalent, or multivalent. Examples of $C_{3-6}$ cycloalkyl include but are not limited to cyclopropyl, cyclobutyl, cyclopentyl, cyclohexyl and the like. The cycloalkyl group can be optionally substituted by one or more substituents, wherein each substituent is independently selected from hydroxyl, alkyl, alkoxy, halogen, haloalkyl, amino, monoalkylamino, or dialkylamino.

Unless otherwise specified, "$C_{1-3}$ alkoxy" refers to an alkyl containing 1 to 3 carbon atoms that are connected to the rest of the molecule through an oxygen atom. The $C_{1-3}$ alkoxy includes $C_{1-2}$, $C_2$, and $C_3$ alkoxy and the like. Examples of $C_{1-3}$ alkoxy include but are not limited to methoxy, ethoxy, propoxy, n-propyloxy, isopropyl and the like.

Unless otherwise specified, "$C_{1-3}$ alkylthio" refers to an alkyl containing 1 to 3 carbon atoms connected to the rest of the molecule through a sulfur atom. The $C_{1-3}$ alkylthio includes $C_{1-2}$, $C_2$, and $C_3$ alkylthio and the like. Examples of $C_{1-3}$ alkylthio include but are not limited to methylthio, ethylthio, propylthio, n-propylthio, isopropylthio and the like.

Unless otherwise specified, "aryl" refers to a 6 to 14-membered, perferably 6-to 10-membered, all-carbon monocyclic or fused polycyclic (i.e., rings that shares a pair of adjacent carbon atoms) group having a conjugated $\pi$-electron system, for example, phenyl and naphthyl.

Unless otherwise specified, "benzene 5-7-membered heterocyclyl" refers to a benzene heterocyclyl, which has a benzene structure fused to a heterocyclyl ring. The "heterocyclyl" in "benzene heterocyclyl" represents a saturated cyclic group composed of 5-7 ring atoms, one or two of which are heteroatoms independently selected from O, S and N, and the rest are carbon atoms. Non-limiting Examples of benzene heterocyclyl include but are not limited to

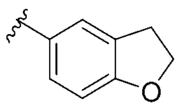,
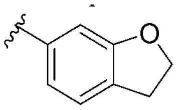,

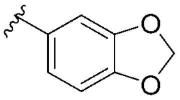,
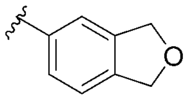,
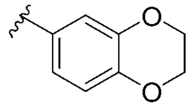,
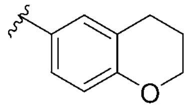,
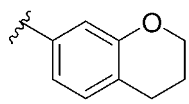

and the like.

Unless otherwise specified, "benzene 5-7-membered cycloalkyl" refers to benzene cycloalkyl, which has a benzene structure fused to a cycloalkyl ring. "cycloalkyl" in "benzene cycloalkyl" represents a saturated cyclic group composed of 5-7 carbon atoms. Examples of benzene-cycloalkyl include but are not limited to

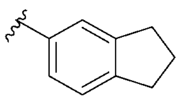,
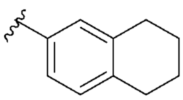,
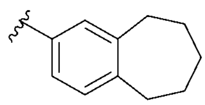

and the like.

"Heteroaryl" refers to a heteroaromatic system containing 1 to 4 heteroatoms and 5 to 14 ring atoms, wherein the heteroatoms are selected from the group consisting of oxygen, sulfur and nitrogen. Heteroaryl is preferably 5 to 10-membered, and more preferably 5-membered or 6-membered, such as furanyl, thiophenyl, pyridinyl, pyrrolyl, N-alkylpyrrolyl, isoxazolyl, thiazolyl, oxazolyl, pyrimidinyl, pyrazinyl, dazinyl, imidazolyl, pyrazolyl, triazolyl, tetrazolyl,

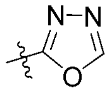

,

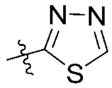

and the like.

Unless otherwise specified, "heterocyclyl" itself or in combination with other terms separately represents a saturated or partially saturated cyclic group composed of 4-14 ring atoms, in which 1, 2, 3 or 4 ring atoms are heteroatoms independently selected from O, S and N, and the rest are carbon atoms, wherein the nitrogen atom is optionally quaternized, and the carbon, nitrogen and sulfur atoms can be optionally oxidized (such as C(=O), SO and $SO_2$). The ring comprises monocyclic, bicyclic and tricyclic systems, wherein the bicyclic and tricyclic systems include spirocyclic, fused cyclic and bridged cyclic rings. In addition, heteroatoms can occupy the connection position between the heterocyclyl and the rest of the molecule, and the heterocyclyl includes saturated as well as partially unsaturated heterocyclyl. Examples of heterocyclyl include but are not limited to azetidinyl, oxazetidinyl, thiazetidinyl, pyrrolidinyl, pyrazolyl, imidazolyl, tetrahydrothiophenyl (including tetrahydrothiophen-2-yl and tetrahydrothiophen-3-yl, etc.), tetrahydrofuranyl (including tetrahydrofuranyl-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl,

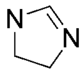

and the like.

The term "halo" or "halogen" refers to fluorine, chlorine, bromine and iodine atom.

The term "cyano" refers to -CN.

## SPECIFIC PHARMACEUTICAL AND MEDICAL TERMS

The term "acceptable," as used herein, means that a prescription component or active ingredient does not have excessively harmful effects on the health of subject.

As used herein, the term "treatment," "course of treatment" or "therapy" includes alleviating, inhibiting or improving symptoms or conditions of diseases; inhibiting the occurrence of complications; improving or preventing potential metabolic syndrome; suppressing the occurrence of diseases or symptoms, such as controlling the development of diseases or conditions; alleviating diseases or symptoms; reducing disease or symptoms; alleviating complications caused by diseases or symptoms, or preventing or treating symptoms caused by diseases or symptoms. As used herein, a certain compound or pharmaceutical composition, which can improve a certain disease, symptom or condition after administration, especially reduce its severity, delay the onset, slow down the progress of the disease, or shorten the duration of the disease. Whether fixed administration or temporary administration, continuous administration or intermittent administration, which can be attributed to related administration.

The term "active ingredient" refers to the compounds represented by the general formula (1) and the pharmaceutically acceptable inorganic or organic salts of the compounds of the general formula (1). Compounds of the present invention may contain one or more asymmetric centers, and thus appear in the form of racemates, racemic mixtures, single enantiomers, diastereomeric compounds or single diastereomers. The asymmetric centers that can exist depend on the properties of various substituents on the molecule. Each such asymmetric center will independently produce two optical isomers, and all possible optical isomers, diastereomer mixtures and pure or partially pure compounds are included in the scope of the present disclosure. The present disclosure is meant to include all such isomeric forms of these compounds.

The terms such as "compound," "composition," "agent" or "medicine ormedicament" can be used interchangeably here, and all refer to a compound or composition which can induce the desired pharmaceutical and/or physiological response through local and/or systemic action when administered to a person (human or animal).

The term "administered, administering or administration" here refers to the direct administration of the compounds or compositions, or the administration of prodrugs, derivatives or analogs of the active compounds.

Although the numerical ranges and parameters used to define the wide range of the present invention are approximate values, the relevant values in the specific embodiments have been presented here as accurately as possible.

However, any numerical value inevitably contains standard deviation caused by individual test methods. Here, "about" usually means that the actual value is within plus or minus 10%, 5%, 1% or 0.5% of a specific value or range. Alternatively, the word "about" means that the actual value falls within the acceptable standard error of the average value, depending on the consideration of those skilled in the art. Except for experimental examples, or unless otherwise specified, it is understood that all ranges, quantities, values and percentages used herein (for example, to describe the amount of materials, the length of time, the temperatures, the operating conditions, the proportions of quantities and other similar ones) are modified by "about". Therefore, unless otherwise stated, the numerical parameters disclosed in this specification and the appended claims are approximate values, and can be changed as required. At least these numerical parameters should be understood as the indicated effective digits and the numerical values obtained by applying the general carry method.

Unless otherwise defined in this specification, the meanings of scientific and technical terms used herein are the same as those understood and used by those skilled in the art. In addition, the singular noun used in this specification covers the plural form of the noun without conflict with the context; the plural nouns used also cover the singular form of the noun.

## ROUTE OF ADMINISTRATION

The compound of the present invention and its pharmaceutically acceptable salts can be made into various preparations, which contain the compound of the present invention or its pharmaceutically acceptable salts and pharmaceutically acceptable excipients or carriers in a safe and effective amount range. Among them, "safe and effective amount" means that the amount of the compound is capable of obviously improving the condition without causing serious side effects. The safe and effective dose of the compound is determined according to the age, illness, course of treatment and other specific conditions of the subject.

"Pharmaceutically acceptable excipient or carrier" refers to one or more compatible solid or liquid fillers or gel substances, which are suitable for human use and must have sufficient purity and low toxicity. "Compatibility" here means that each component in the composition can be mixed with the compounds of the present invention and between them, without significantly reducing the efficacy of the compound. Examples of pharmaceutically acceptable excipients or carriers include cellulose and its derivatives (such as sodium carboxymethyl cellulose, sodium ethyl cellulose, cellulose acetate, etc.), gelatin, talc, solid lubricants (such as stearic acid and magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agent, flavoring agent, stabilizer, antioxidant, preservative, pyrogen-free water, etc.

The compounds of the present invention can be administered orally, rectally, parenterally (intravenously, intramuscularly or subcutaneously) or topically.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with: (a) a filler or compatibilizer, such as starch, lactose, sucrose, glucose, mannitol, and silicic acid; (b) binders such as hydroxymethyl cellulose, alginates, gelatin, polyvinyl pyrrolidone, sucrose and acacia; (c) humectant, such as glycerol; (d) disintegrant such as agar, calcium carbonate, potato or tapioca starch, alginic acid, certain complex silicates, and sodium carbonate; (e) slow solvents, such as paraffin; (f) an absorption accelerator, such as a quaternary amine compound; (g) wetting agents, such as cetyl alcohol and glyceryl monostearate; (h) adsorbent, such as kaolin; and (i) a lubricant such as talc, calcium stearate, magnesium stearate, solid polyethylene glycol, sodium lauryl sulfate, or mixtures thereof. In capsule, tablets and pill, the dosage form may also contain a buffer.

Solid dosage forms such as tablets, sugar pills, capsules, pills and granules may be prepared using coatings and shell materials such as casings and other materials well-known in the art. They may comprise an opacifying agent and the release of the active compound or compound in such a composition may be released in a delayed manner in a portion of the digestive tract. Examples of embedding components that may be used are polymeric substances and waxes. If desired, the active compound may also form microcapsules with one or more of the above excipients.

Liquid dosage forms for oral administration include pharmaceutically acceptable emulsions, solutions, suspensions, syrups or tinctures. In addition to the active compound, the liquid dosage form may comprise inert diluents conventionally used in the art, such as water or other solvents, solubilizers and emulsifiers, for example ethanol, isopropanol, ethyl carbonate, ethyl acetate, propylene glycol, 1,3-butanediol, dimethylformamide and oils, particularly cottonseed oil, peanut oil, corn germ oil, olive oil, castor oil, sesame oil or mixtures of these and the like.

In addition to these inert diluents, the composition may also contain adjuvants such as wetting agents, emulsifiers and suspending agents, sweeteners, flavoring agents and flavorants.

In addition to the active compounds, the suspension may comprise suspending agents such as ethoxylated isostearyl alcohol, polyoxyethylene sorbitol and sorbitan esters, microcrystalline cellulose, aluminum methoxide, agar or mixtures of these and the like.

The composition for parenteral injection may comprise a physiologically acceptable sterile aqueous or non-aqueous solution, dispersion, suspension or emulsion, and a sterile powder for reconstitution into a sterile injectable solution or dispersion. Suitable aqueous and non-aqueous carriers, diluents, solvents or excipients include water, ethanol, polyols and suitable mixtures thereof.
Dosage forms of the compounds of the present invention for topical administration include ointments, powders, patches, sprays and inhalants. The active ingredient is mixed under sterile conditions with a physiologically acceptable carrier and any preservatives, buffers, or propellants as may be required.
The compound of the present invention may be administered alone or in combination with other pharmaceutically acceptable compounds.
When a pharmaceutical composition is used, a safe and effective amount of a compound of the present invention is applied to a mammal (e.g., a human) in need of treatment, wherein the dose at the time of administration is a pharmaceutically acceptable effective dose, and for a human of 60 kg body weight, the daily dose is generally 1 to 2000 mg, preferably 50 to 1000 mg. Of course, specific dose should also take into account factors such as route of administration, patient health, etc., which are within the skill scope of a skilled physician.
The main advantages of the present disclosure include:

(a) Unexpectedly, compared to ABX464, the compound of the present disclosure showed excellent *in vitro* activity, and also demonstrated better *in vivo* pharmacokinetic properties, making it more suitable for development as a drug.
(b) The compound of the present disclosure has better pharmaceutical properties.
(c) Compared to ABX464, the compound of the present disclosure has significantly better efficacy in the treatment of inflammatory bowel disease in a mouse model induced by DSS.

The above features mentioned in the present disclosure, or the features mentioned in the examples, may be combined at random. All of the features disclosed in this specification may be used in any composition form and the various features disclosed in the specification may be replaced with any alternative feature that provides the same, equivalent, or similar purpose. Thus, unless otherwise specified, the disclosed features are merely generic examples of equivalent or similar features.
Various specific aspects, features and advantages of the above compounds, methods, and pharmaceutical compositions will be set forth in detail as following. It is to be understood that the following detailed description and examples describe specific embodiments for reference only. Various changes or modifications may occur to those skilled in the art after reading the description of the invention, and such equivalents fall within the scope of the application.
The structures of compounds disclosed herein can be confirmed by conventional methods well known to those skilled in the art. If the present disclosure relates to an absolute configuration of a compound, the absolute configuration can be confirmed by conventional techniques in the field. For example, single crystal X-ray diffraction (SXRD), the diffraction intensity data of the cultivated single crystal is collected using a Bruker D8 venture diffractometer with a light source of CuK$\alpha$ radiation in a scanning mode of $\varphi/\omega$ scan. After collecting the relevant data, the crystal structure is further analyzed by the direct method (Shelxs97) to conform the absolute configuration.
The solvent used in the present invention can be obtained commercially. The compounds of the present disclosure are named according to the conventional naming principles in the art or by ChemDraw® software, and the commercially available compounds use the supplier catalog names.
In all examples, $^1$H-NMR was recorded with a Varian Mercury 400 NMR spectrometer and that chemical shift was expressed as $\delta$ (ppm); Silica gel for separation is 200-300 mesh without specific statement, and the ratio of eluents is volume ratio.
Abbreviations: $CHCl_3$ represents chloroform; Cu represents copper; $Cs_2CO_3$ represents cesium carbonate; EA (EtOAc) represents ethyl acetate; DCM represents dichloromethane; Dioxane represents 1,4-dioxane; DMF represents N,N-dimethylformamide; DMSO represents dimethyl sulfoxide; EtOH represents ethanol; Fe represents iron; h represents hour(s); $K_2CO_3$ represents potassium carbonate; LC-MS represents liquid chromatography-mass spectrometry; MeI ($CH_3I$) represents iodomethane; MeOH represents methanol; MS represents mass spectrometry; $Na_2CO_3$ represents sodium carbonate; NaH represents sodium hydride; NaOH represents sodium hydroxide; NMR represents nuclear magnetic resonance; Pd/C represents palladium on carbon; $Pd(OAc)_2$ represents palladium(II) acetate; PE represents petroleum ether; $POCl_3$ represents phosphorus oxychloride; $PtO_2$ represents platinum(IV) oxide; r. t. represents room temperature; $SOCl_2$ represents thionyl chloride; TEA ($Et_3N$) represents triethylamine; TLC represents thin-layer chromatography; THF represents tetrahydrofuran; $Tf_2O$ represents trifluoromethanesulfonic anhydride; Tol represents toluene; TsCl represents p-toluenesulfonyl chloride; Xantphos represents 4,5-bis(diphenylphosphino)-9,9-dimethylxanthene; AcOH (HOAc) represents acetic acid; CDI represents carbonyldiimidazole; DIEA (DIPEA) represents diisopropylethylamine; $NH_4Cl$ represents ammonium chloride.
The present invention is further described through the following examples. It should be understood that these examples are only used to illustrate the invention, but not intented to impose any limitations on the present invention. The

experimental methods not specified in the following examples are usually carried out under conventional conditions, or according to the conditions recommended by the manufacturer. Unless otherwise stated, percentages and parts are weight percentages and weight parts.

**Preparation Example 1: Synthesis of 3,5-Dichloro-1-methylquinoxalin-2(1*H*)-one (Intermediate 1)**

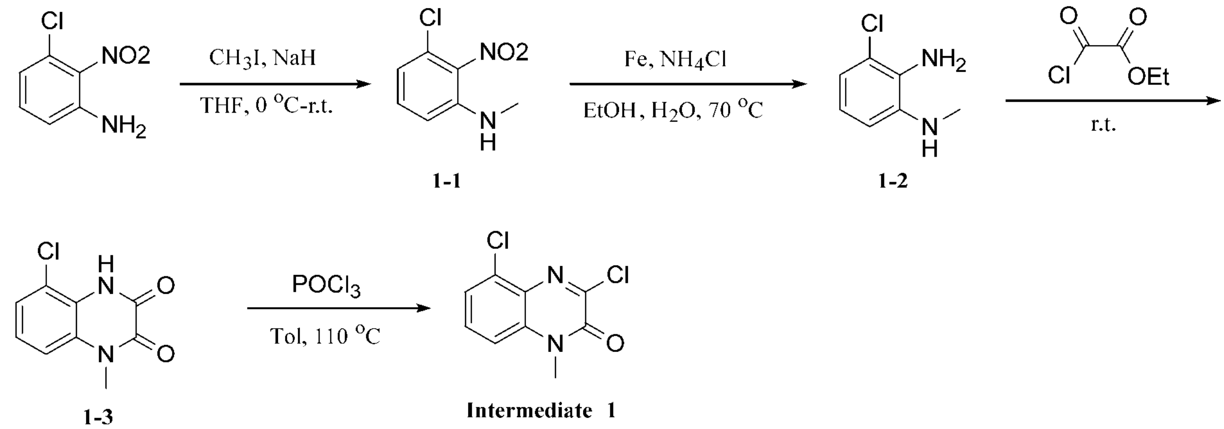


1-1 1-2 1-3 Intermediate 1

**Step 1: Synthesis of Compound 1-1**

To a solution of 3-chloro-2-nitroaniline (3 g, 17.38 mmol) in THF (50 mL) was added NaH (765 mg, 19.12 mmol) slowly at 0 °C. The resulting mixture was stirred for 30 min at 0 °C. Then a solution of MeI (2.47 g, 17.38 mmol) in THF was added dropwise. The reaction was stirred at room temperature until the starting material was consumed. The reaction was then quenched with water and extracted with EA (50 mL*3) and dried over anhydrous $Na_2SO_4$. After filtration, the filtrate was concentrated under reduced pressure. The residue was purified by silica gel column chromatography (PE/EA = 30/1 to 10/1) to afford a red solid **1-1** (1.2 g, yield: 37%), ESI-MS m/z: 186.9 $[M+H]^+$.

**Step 2: Synthesis of Compound 1-2**

To a solution of **compound 1-1** (1.2 g, 6.45 mmol) in EtOH (30 mL) and $H_2O$ (10 mL) was added Fe powder (1.81 g, 32.25 mmol) and $NH_4Cl$ (3.45 g, 64.5 mmol). The resulting mixture was stirred at 70 °C until the starting material is consumed. The reaction was filtrated and then extracted with EA (30 mL*3) and dried over anhydrous $Na_2SO_4$. After filtration, the filtrate was concentrated under reduced pressure. The residue was purified by silica gel column chromatography (PE/EA = 10/1 to 2/1) to afford yellow oil 1-2 (500 mg, yield: 50%), ESI-MS m/z: 156.9 $[M+H]^+$.

**Step 3: Synthesis of Compound 1-3**

**Compound 1-2** (500 mg, 3.21 mmol) was dissolved in ethyl oxalyl monochloride (10 mL). The resulting mixture was stirred at room temperature until the starting material is consumed. The reaction was concentrated under reduced pressure. The residue was purified by silica gel column chromatography (PE/EA = 30/1 to 10/1) to afford a yellow solid **1-3** (320 mg, yield: 47%), ESI-MS m/z: 210.8 $[M+H]^+$.

**Step 4: Synthesis of Intermediate 1**

**Compound 1-3** (320 mg, 1.52 mmol) was dissolved in $POCl_3$ (10 mL). The resulting mixture was stirred at 110 °C until the starting material is consumed. The reaction was concentrated under reduced pressure and then quenched with water (50 mL). The resulting mixture was neutralized with saturated sodium bicarbonate and then extracted with EA (30 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid **intermediate 1** (220 mg, yield: 63%), ESI-MS m/z: 228.8 $[M+H]^+$.

**Preparation Example 2: Synthesis of 3,7-Dichloro-5-fluoro-1-methylquinolizine -2(1*H*)-one (Intermediate 2)**

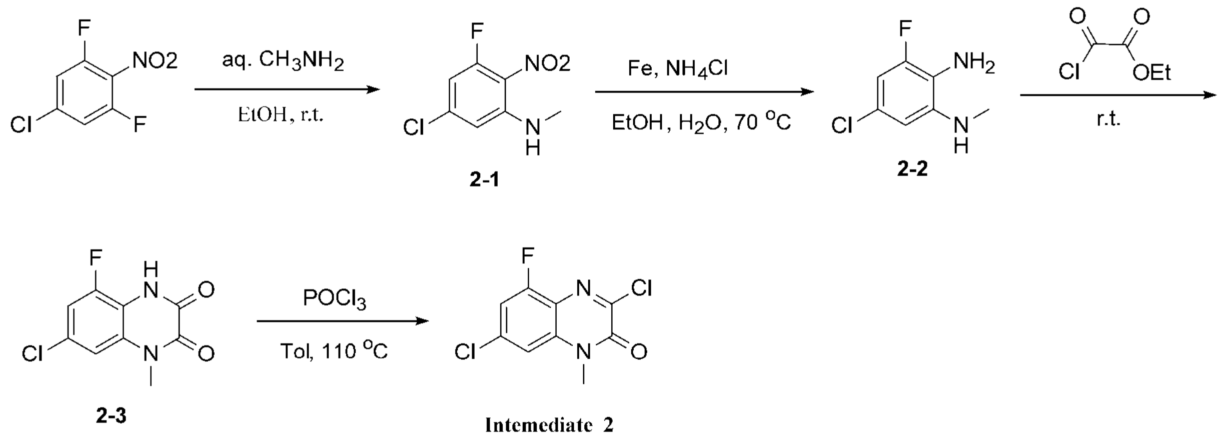


2-1

2-2

2-3

Intemediate 2

**Step 1: Synthesis of Compound 2-1**

To a solution of 4-chloro-2,6-difluoronitrobenzene (2 g, 10.33 mmol) in EtOH (50 mL) was added methylamine water solution (1.07 g, 10.33 mmol, 30%). The resulting mixture was stirred at room temperature until the starting material is consumed. The reaction was concentrated under reduced pressure and then quenched with water (100 mL) and extracted with EA (50 mL*3) and dried over anhydrous $Na_2SO_4$. After filtration, the filtrate was concentrated under reduced pressure. The residue was purified by silica gel column chromatography (PE/EA = 30/1 to 10/1) to afford a yellow solid **2-1** (1.5 g, yield: 71%), ESI-MS m/z: 204.8 $[M+H]^+$.

Using **compound 2-1** as the raw material, **intermediate 2** can be synthesized by a method similar to the synthesis of intermediate 1, ESI-MS m/z: 247.0 $[M+H]^+$.

**Preparation Example 3: Synthesis of 6-Chloroimidazo[1,2-*a*]quinoxalin-4-yl trifluoromethanesulfonate (Intermediate 3)**

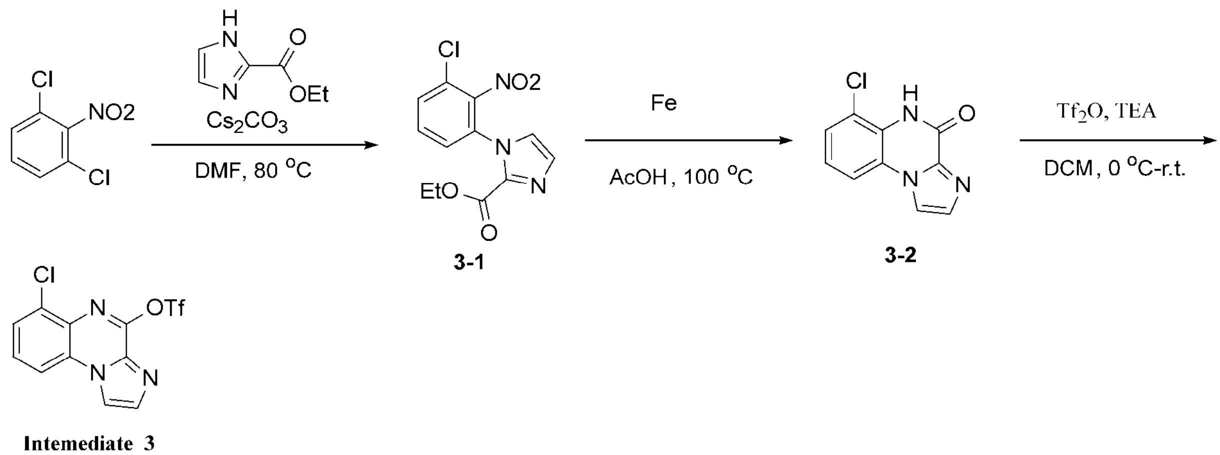


3-1

3-2

Intemediate 3

**Step 1: Synthesis of Compound 3-1**

To a solution of 2,6-dichloronitrobenzene (2 g, 10.42 mmol) in DMF (50 mL) was added imidazole-2-carboxylic acid ethyl ester (1.46 g, 10.42 mmol) and $Cs_2CO_3$ (5.08 g, 15.63 mmol). The resulting mixture was stirred at 80 °C until the starting material is consumed. The reaction was then quenched with water (200 mL) and extracted with EA (50 mL*3) and dried over anhydrous $Na_2SO_4$. After filtration, the filtrate was concentrated under reduced pressure. The residue was purified by silica gel column chromatography (PE/EA = 30/1 to 10/1) to afford a white solid 3-1 (2.7 g, yield: 88%), ESI-MS

m/z: 295.9 [M+H]+.

**Step 2: Synthesis of Compound 3-2**

To a solution of **compound 3-1** (2.7 g, 9.15 mmol) in HOAc (30 mL) was added Fe powder (2.56 g, 45.76 mmol). The resulting mixture was stirred at 100 °C until the starting material is consumed. The reaction was concentrated under reduced pressure to remove HOAc and neutralized with saturated sodium bicarbonate. The resulting mixture was extracted with EA (50 mL*3) and dried over anhydrous $Na_2SO_4$. After filtration, the filtrate was concentrated under reduced pressure. The residue was purified by silica gel column chromatography (PE/EA = 10/1 to 2/1) to afford a yellow solid **3-2** (1.1 g, yield: 55%), ESI-MS m/z: 219.8 [M+H]+.

**Step 3: Synthesis of Intermediate 3**

To a solution of **compound 3-2** (1.1 g, 5.02 mmol) in DCM (30 mL) was added TEA (1.01 g, 10.04 mmol) and $Tf_2O$ (2.12 g, 7.53 mmol) slowly at 0 °C under an argon atmosphere. The resulting mixture was stirred at room temperature until the starting material is consumed. The reaction was then quenched with water (100 mL) and extracted with DCM (50 mL*3) and dried over anhydrous $Na_2SO_4$. After filtration, the filtrate was concentrated under reduced pressure. The residue was purified by silica gel column chromatography (PE/EA = 50/1 to 20/1) to afford a white solid **intermediate 3** (1.3 g, yield: 74%), ESI-MS m/z: 351.8 [M+H]+.

Using imidazole-2-carboxylic acid ethyl ester and corresponding substituted nitrobenzene as raw materials, **intermediate 4-54** can be synthesized by a method similar to intermediate 3.

| Compound | Structure | Name | ESI-MS: [M+H]+ |
|---|---|---|---|
| **Intermediate 4** | 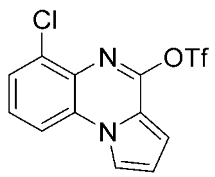 | 6-chloropyrrolo [1,2-*a*] quinoxalin-4-yl trifluoromethanesulfonate | 350.8 |
| **Intermediate 5** | 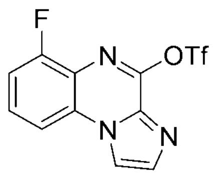 | 6-fluoroimidazo [1,2-*a*] quinoxalin-4-yl trifluoromethanesulfonate | 335.8 |
| **Intermediate 6** | 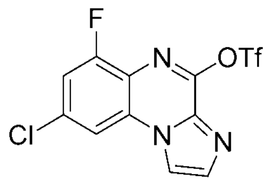 | 8-chloro-6-fluoroimidazo [1,2-*a*] quinoxalin-4-yl trifluoromethanesulfonate | 369.8 |
| **Intermediate 7** | 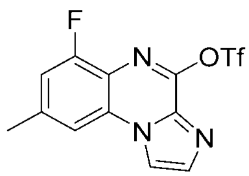 | 6-fluoro-8-methylimidazolo [1,2-*a*] quinoxalin-4-yl trifluoromethanesulfonate | 349.9 |
| **Intermediate 8** | 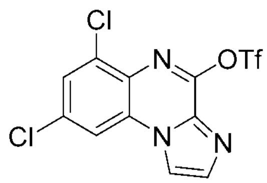 | 6,8-dichloroimidazolo [1,2-*a*] quinoxalin-4-yl trifluoromethanesulfonate | 385.9 |

(continued)

| Compound | Structure | Name | ESI-MS: [M+H]+ |
|---|---|---|---|
| **Intermediate 9** | 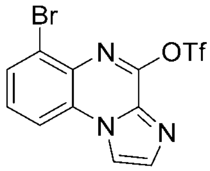 | 6-bromoimidazo [1,2-*a*] quinoxalin-4-yl trifluoromethanesulfonate | 395.9 |
| **Intermediate 10** | 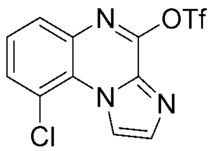 | 9-chloroimidazo [1,2-*a*] quinoxalin-4-yl trifluoromethanesulfonate | 351.8 |
| **Intermediate 11** | 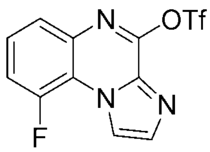 | 9-fluoroimidazo [1,2-*a*] quinoxalin-4-yl trifluoromethanesulfonate | 335.9 |
| **Intermediate 12** | 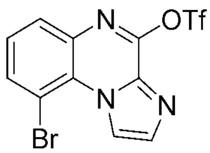 | 9-bromoimidazolo [1,2-*a*] quinoxalin-4-yl trifluoromethanesulfonate | 395.8 |
| **Intermediate 13** | 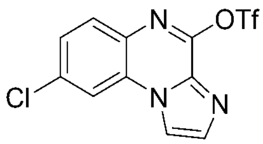 | 8-chloroimidazo [1,2-*a*] quinoxalin-4-yl trifluoromethanesulfonate | 351.8 |
| **Intermediate 14** | 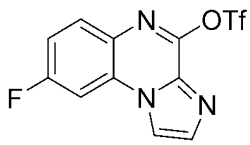 | 8-fluoroimidazo [1,2-*a*] quinoxalin-4-yl trifluoromethanesulfonate | 335.9 |
| **Intermediate 15** | 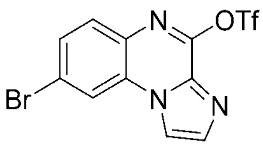 | 8-bromoimidazo [1,2-*a*] quinoxalin-4-yl trifluoromethanesulfonate | 395.8 |
| **Intermediate 16** | 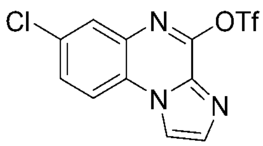 | 7-chloroimidazo [1,2-*a*] quinoxalin-4-yl trifluoromethanesulfonate | 351.8 |
| **Intermediate 17** | 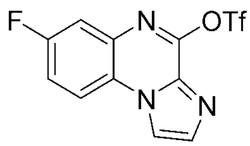 | 7-fluoroimidazo [1,2-*a*] quinoxalin-4-yl trifluoromethanesulfonate | 335.9 |
| **Intermediate 18** | 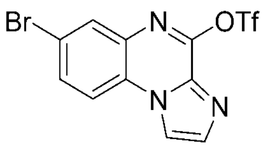 | 7-bromoimidazolo [1,2-*a*] quinoxalin-4-yl trifluor-omethanesulfonate | 395.8 |

(continued)

| Compound | Structure | Name | ESI-MS: [M+H]+ |
|---|---|---|---|
| **Intermediate 19** | 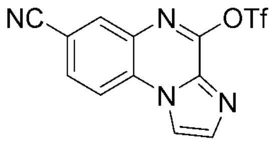 | 7-cyanoimidazolo [1,2-*a*] quinoxalin-4-yl trifluoromethanesulfonate | 342.8 |
| **Intermediate 20** | 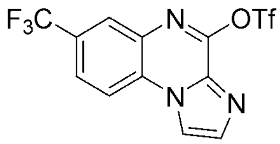 | 7-trifluoromethylimidazolo [1,2-*a*] quinoxalin-4-yl trifluoromethanesulfonate | 385.9 |
| **Intermediate 21** | 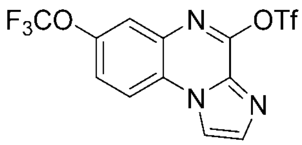 | 7-trifluoromethoxyimidazo [1,2-*a*] quinoxalin-4-yl trifluoromethanesulfonate | 401.9 |
| **Intermediate 22** | 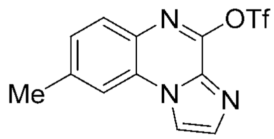 | 8-methylimidazolo [1,2-*a*] quinoxalin-4-yl trifluoromethanesulfonate | 331.8 |
| **Intermediate 23** | 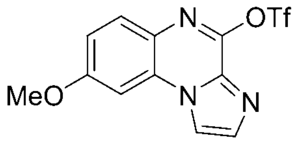 | 8-methoxyimidazo [1,2-*a*] quinoxalin-4-yl trifluoromethanesulfonate | 347.8 |
| **Intermediate 24** | 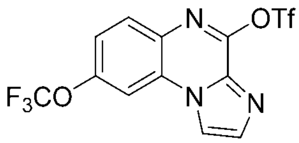 | 8-trifluoromethoxyimidazo [1,2-*a*] quinoxalin-4-yl trifluoromethanesulfonate | 401.9 |
| **Intermediate 25** | 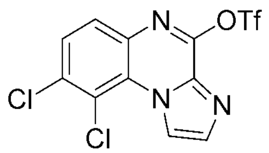 | 8,9-dichloroimidazo [1,2-*a*] quinoxalin-4-yl trifluoromethanesulfonate | 385.8 |
| **Intermediate 26** | 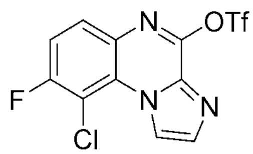 | 9-chloro-8-fluoroimidazo [1,2-*a*] quinoxalin-4-yl trifluoromethanesulfonate | 369.8 |
| **Intermediate 27** | 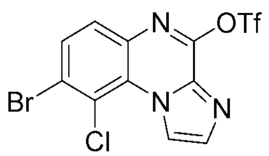 | 8-bromo-7-chloroimidazolo [1,2-*a*] quinoxalin-4-yl trifluoromethanesulfonate | 429.7 |
| **Intermediate 28** | 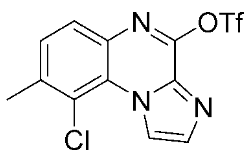 | 9-chloro-8-methylimidazolo [1,2-*a*] quinoxalin-4-yl trifluoromethanesulfonate | 365.9 |

(continued)

| Compound | Structure | Name | ESI-MS: [M+H]+ |
|---|---|---|---|
| **Intermediate 29** | 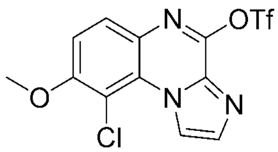 | 9-chloro-8-methoxyimidazo [1,2-*a*] quinoxalin-4-yl trifluoromethanesulfonate | 381.9 |
| **Intermediate 30** | 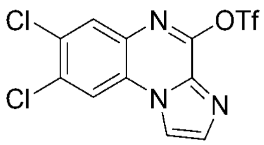 | 7,8-dichloroimidazolo [1,2-*a*] quinoxalin-4-yl trifluoromethanesulfonate | 385.8 |
| **Intermediate 31** | 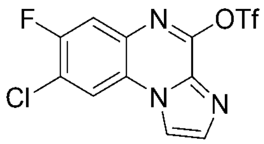 | 8-chloro-7-fluoroimidazo [1,2-*a*] quinoxalin-4-yl trifluoromethanesulfonate | 369.8 |
| **Intermediate 32** | 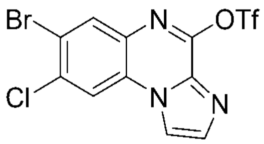 | 7-bromo-8-chloroimidazolo [1,2-*a*] quinoxalin-4-yl trifluoromethanesulfonate | 429.7 |
| **Intermediate 33** | 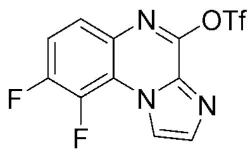 | 8,9-difluoroimidazo [1,2-*a*] quinoxalin-4-yl trifluoromethanesulfonate | 353.9 |
| **Intermediate 34** | 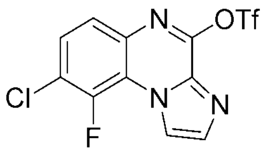 | 8-chloro-9-fluoroimidazo [1,2-*a*] quinoxalin-4-yl trifluoromethanesulfonate | 369.8 |
| **Intermediate 35** | 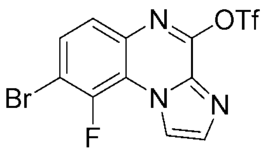 | 8-bromo-9-fluoroimidazolo [1,2-*a*] quinoxalin-4-yl trifluoromethanesulfonate | 413.8 |
| **Intermediate 36** | 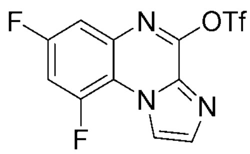 | 7,9-difluoroimidazo [1,2-*a*] quinoxalin-4-yl trifluoromethanesulfonate | 353.9 |
| **Intermediate 37** | 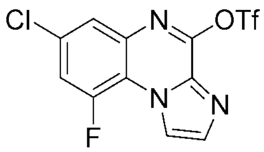 | 7-chloro-9-fluoroimidazo [1,2-*a*] quinoxalin-4-yl trifluoromethanesulfonate | 369.8 |
| **Intermediate 38** | 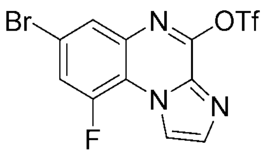 | 7-bromo-9-fluoroimidazolo [1,2-*a*] quinoxalin-4-yl trifluoromethanesulfonate | 413.8 |

(continued)

| Compound | Structure | Name | ESI-MS: [M+H]+ |
|---|---|---|---|
| **Intermediate 39** | 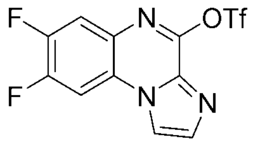 | 7,8-difluoroimidazolo [1,2-*a*] quinoxalin-4-yl trifluoromethanesulfonate | 353.9 |
| **Intermediate 40** | 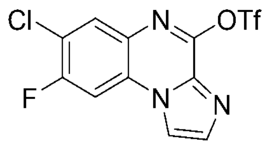 | 7-chloro-8-fluoroimidazo [1,2-*a*] quinoxalin-4-yl trifluoromethanesulfonate | 369.8 |
| **Intermediate 41** | 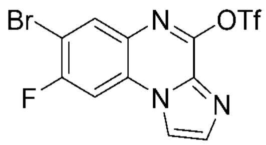 | 7-bromo-8-fluoroimidazolo [1,2-*a*] quinoxalin-4-yl trifluoromethanesulfonate | 413.8 |
| **Intermediate 42** | 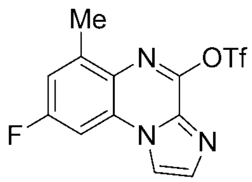 | 8-fluoro-6-methylimidazolo [1,2-*a*] quinoxalin-4-yl trifluoromethanesulfonate | 349.8 |
| **Intermediate 43** | 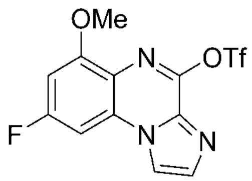 | 8-fluoro-6-methoxyimidazo [1,2-*a*] quinoxalin-4-yl trifluoromethanesulfonate | 365.8 |
| **Intermediate 44** | 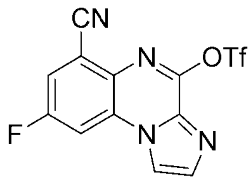 | 8-fluoro-6-cyanoimidazolo [1,2-*a*] quinoxalin-4-yl trifluoromethanesulfonate | 360.8 |
| **Intermediate 45** | 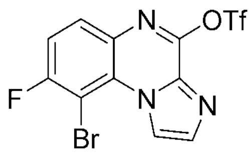 | 9-bromo-8-fluoroimidazo [1,2-*a*] quinoxalin-4-yl trifluoromethanesulfonate | 413.8 |
| **Intermediate 46** | 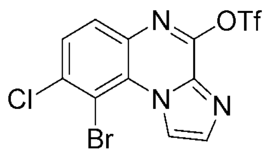 | 9-bromo-8-chloroimidazolo [1,2-*a*] quinoxalin-4-yl trifluoromethanesulfonate | 429.7 |
| **Intermediate 47** | 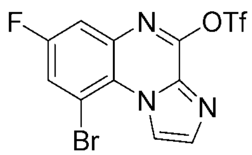 | 9-bromo-7-fluoroimidazolo [1,2-*a*] quinoxalin-4-yl trifluoromethanesulfonate | 413.8 |

(continued)

| Compound | Structure | Name | ESI-MS: [M+H]+ |
|---|---|---|---|
| **Intermediate 48** | 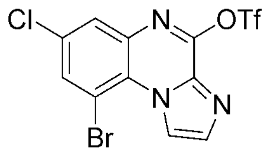 | 9-bromo-7-chloroimidazolo [1,2-*a*] quinoxalin-4-yl trifluoromethanesulfonate | 429.7 |
| **Intermediate 49** | 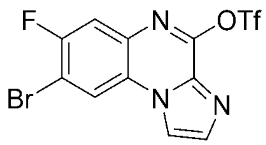 | 8-bromo-7-fluoroimidazolo [1,2-*a*] quinoxalin-4-yl trifluoromethanesulfonate | 413.8 |
| **Intermediate 50** | 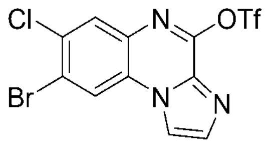 | 8-bromo-7-chloroimidazolo [1,2-*a*] quinoxalin-4-yl trifluoromethanesulfonate | 429.7 |
| **Intermediate 51** | 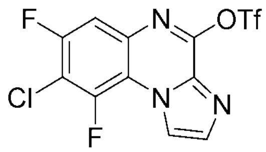 | 8-chloro-7,9-difluoroimidazo [1,2-*a*] quinoxalin-4-yl trifluoromethanesulfonate | 387.8 |
| **Intermediate 52** | 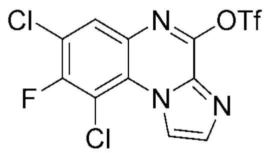 | 7,9-dichloro-8-fluoroimidazo [1,2-*a*] quinoxalin-4-yl trifluoromethanesulfonate | 403.8 |
| **Intermediate 53** | 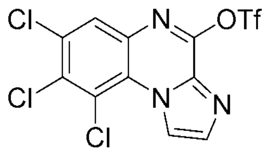 | 7,8,9-trichloroimidazo [1,2-*a*] quinoxalin-4-yl trifluoromethanesulfonate | 419.7 |
| **Intermediate 54** | 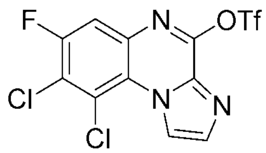 | 8,9-dichloro-7-fluoroimidazo [1,2-*a*] quinoxalin-4-yl trifluoromethanesulfonate | 403.8 |

**Preparation Example 4: Synthesis of 4,6-dichloro-1,2,4-triazolo [4,3-*a*] quinoxaline (Intermediate 55)**

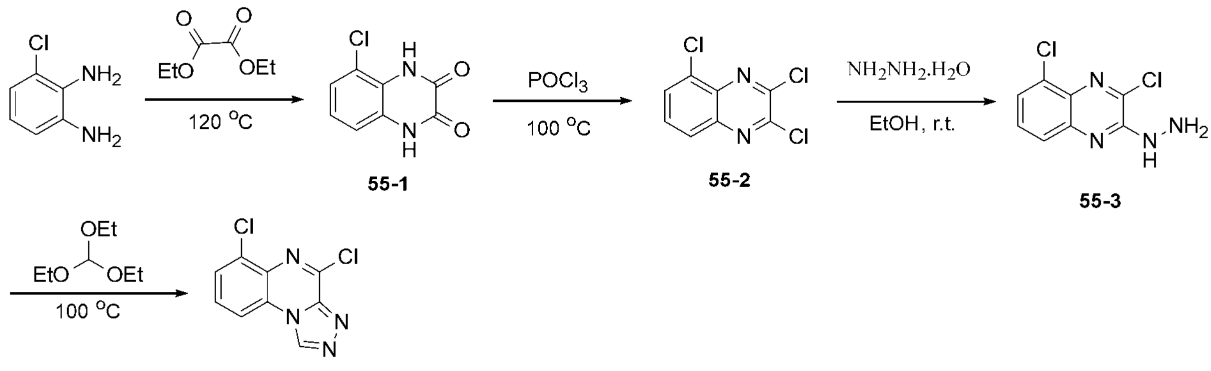


55-1 55-2 55-3

Intermediate 55

**Step 1: Synthesis of Compound 55-1**

To a solution of 3-chloro-phenylenediamine (2 g, 14.03 mmol) in diethyl oxalate (20 mL) was stirred at 120 °C until the starting material was consumed. The reaction was concentrated under reduced pressure. The residue was purified by silica gel column chromatography (PE/EA = 10/1 to 1/1) to afford a light yellow solid 55-1 (2.2 g, yield: 80%), ESI-MS m/z: 196.9 $[M+H]^+$.

**Step 2: Synthesis of Compound 55-2**

**Compound 55-1** (2.2 g, 11.22 mmol) was dissolved in $POCl_3$ (20 mL). The resulting mixture was stirred at 100 °C until the starting material is consumed. The reaction was concentrated under reduced pressure and then quenched with water (50 mL). The resulting mixture was neutralized with saturated sodium bicarbonate and then extracted with EA (30 mL*3) and dried over anhydrous $Na_2SO_4$. After filtration, the filtrate was concentrated under reduced pressure. The residue was purified by silica gel column chromatography (PE/EA = 10/1 to 3/1) to afford a yellow solid **55-2** (1.6 g, yield: 62%), ESI-MS m/z: 232.8 $[M+H]^+$.

**Step 3: Synthesis of Compound 55-3**

To a solution of **compound 55-2** (1.6 g, 6.87 mmol) in EtOH (20 mL) was added hydrazinium hydroxide solution (808 mg, 13.74 mmol, 85%). The resulting mixture was stirred at room temperature until the starting material is consumed. The reaction was concentrated under reduced pressure and then quenched with water (50 mL). The resulting mixture was extracted with EA (30 mL*3) and dried over anhydrous $Na_2SO_4$. After filtration, the filtrate was concentrated under reduced pressure to afford a light yellow solid **55-3** (1.8 g, crude), ESI-MS m/z: 228.9 $[M+H]^+$.

**Step 4: Synthesis of Intermediate 55**

**Compound 55-3** (1.8 g, crude) was dissolved in triethyl orthoformate (20 mL). The resulting mixture was stirred at 100 °C until the starting material is consumed. The reaction was concentrated under reduced pressure and then quenched with water (50 mL). The resulting mixture was extracted with EA (30 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid **55** (1.1 g, yield: 67%), ESI-MS m/z: 238.8 $[M+H]^+$.

**Intermediate 55-3** was used as a common starting material and reacted with triethyl orthoacetate and trifluoroacetic acid, respectively, to afford **Intermediate 56** and **Intermediate 57.**

| Compound | Structure | Name | ESI-MS: $[M+H]^+$ |
|---|---|---|---|
| **Intermediate 56** | 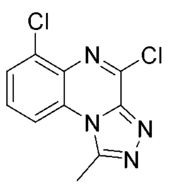 | 4,6-dichloro-1-methyl - [1,2,4] triazolo [4,3-*a*] quinoxaline | 252.9 |

(continued)

| Compound | Structure | Name | ESI-MS: [M+H]+ |
|---|---|---|---|
| **Intermediate 57** | 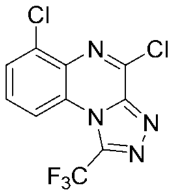 | 4,6-dichloro-1- (trifluoromethyl) - [1,2,4] triazolo [4,3-*a*] quinoxaline | 306.9 |

**Preparation Example 5: Synthesis of 4,6-dichloro-1,2-dihydroimidazo [1,2-*a*] quinoxaline (Intermediate 58)**

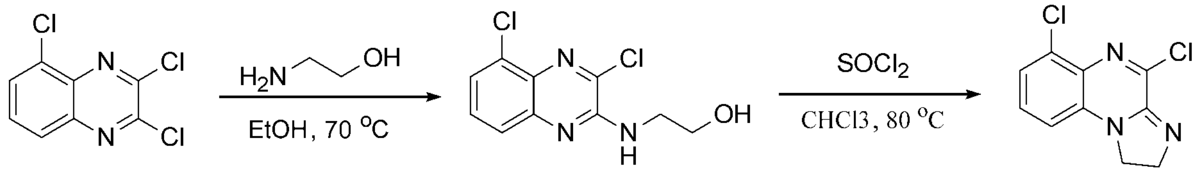


**55-2** **58-1** **Intermediate 58**

**Step 1: Synthesis of Compound 58-1**

To a solution of **55-2** (1 g, 4.31 mmol) in EtOH (20 mL) was added ethanolamine (526 mg, 8.62 mmol). The resulting mixture was stirred at 70 °C until the starting material is consumed. The reaction was concentrated under reduced pressure and diluted with water (50 mL). The resulting mixture was extracted with EA (30 mL*3) and dried over anhydrous $Na_2SO_4$. After filtration, the filtrate was concentrated under reduced pressure. The residue was purified by silica gel column chromatography (PE/EA = 10/1 to 1/1) to afford a red solid **58-1** (950 mg, yield: 86%), ESI-MS m/z: 257.9 $[M+H]^+$.

**Step 2: Synthesis of Intermediate 58**

**Compound 58-1** (950 mg, 3.70 mmol) was dissolved in $CHCl_3$ (20 mL) and $SOCl_2$ (10 mL). The resulting mixture was stirred at 80 °C until the starting material is consumed. The reaction was poured to water (50 mL) and then neutralized with saturated sodium bicarbonate. The resulting mixture was extracted with $CHCl_3$ (30 mL*3) and dried over anhydrous $Na_2SO_4$. After filtration, the filtrate was concentrated under reduced pressure. The residue was purified by silica gel column chromatography (PE/EA = 5/1 to 0/1) to afford a yellow solid **intermediate 58** (500 mg, yield: 57%), ESI-MS m/z: 239.9 $[M+H]^+$.

**Intermediates 59-61** can be synthesized by a method similar to **Intermediate 58,** using variously substituted 1,2-diaminobenzenes as starting materials.

| Compound | Structure | Name | ESI-MS: [M+H]+ |
|---|---|---|---|
| **Intermediate 59** | 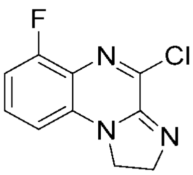 | 4-chloro-6-fluoro-1,2-dihydroimidazo [1,2-*a*] quinoxaline | 223.9 |
| **Intermediate 60** | 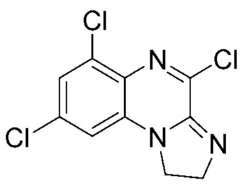 | 4,6,8-trichloro-1,2-dihydroimidazo [1,2-*a*] quinoxaline | 273.9 |

(continued)

| Compound | Structure | Name | ESI-MS: $[M+H]^+$ |
|---|---|---|---|
| **Intermediate 61** | 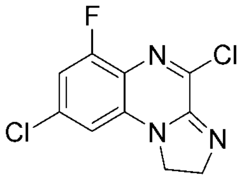 | 4,8-dichloro-6-fluoro-1,2-dihydroimidazo [1,2-*a*] quinoxaline | 257.9 |

**Preparation Example 6: Synthesis of 6-Bromo-1,2-Dihydroimidazolo [1,2-*a*] quinoxalin-4-yl trifluoromethanesulfonate (Intermediate 62)**

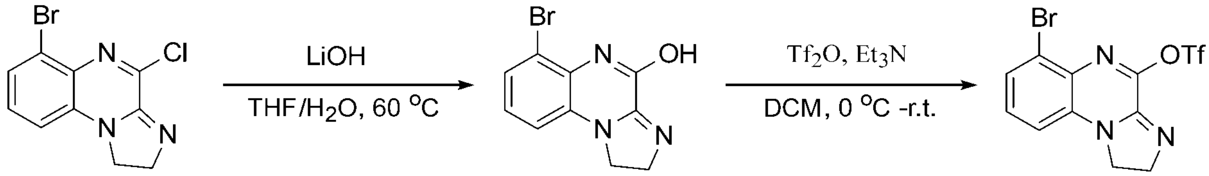


**62-1** **62-2** **Intermediate 62**

**Compound 62-1** can be synthesized by a method similar to **Intermediate 58,** using 3-bromo-1,2-diaminobenzene as a starting material.

**Step 1: Synthesis of Compound 62-2**

To a solution of **62-1** (1 g, 3.51 mmol) in THF/$H_2O$ (20 mL/4 mL) was added LiOH (281 mg, 7.02 mmol). The resulting mixture was stirred at 60 °C until the starting material is consumed. The reaction was then quenched with water (50 mL) and neutralized with 1 M HCl, and then extracted with EA (30 mL*3) and dried over anhydrous $Na_2SO_4$. After filtration, the filtrate was concentrated under reduced pressure to afford a yellow solid **62-2** (910 mg, yield: 97%), ESI-MS m/z: 266.0 $[M+H]^+$.

**Step 2: Synthesis of Intermediate 62**

To a solution of **62-2** (910 mg, 3.42 mmol) in DCM (20 mL) was added TEA (691 mg, 6.84 mmol) and $Tf_2O$ (1.45 g, 5.13 mmol) slowly at 0 °C under an argon atmosphere. The resulting mixture was stirred at room temperature until the starting material is consumed. The reaction was then quenched with water (100 mL) and extracted with DCM (50 mL*3) and dried over anhydrous $Na_2SO_4$. After filtration, the filtrate was concentrated under reduced pressure. The residue was purified by silica gel column chromatography (PE/EA = 10/1 to 3/1) to afford a white solid intermediate 62 (830 mg, yield: 61%), ESI-MS m/z: 397.9 $[M+H]^+$.

**Preparation Example 7: Synthesis of 6-Chloro-1,2,4-triazolo [1,5-*a*] quinoxalin-4-yl trifluoromethanesulfonate (Intermediate 63)**

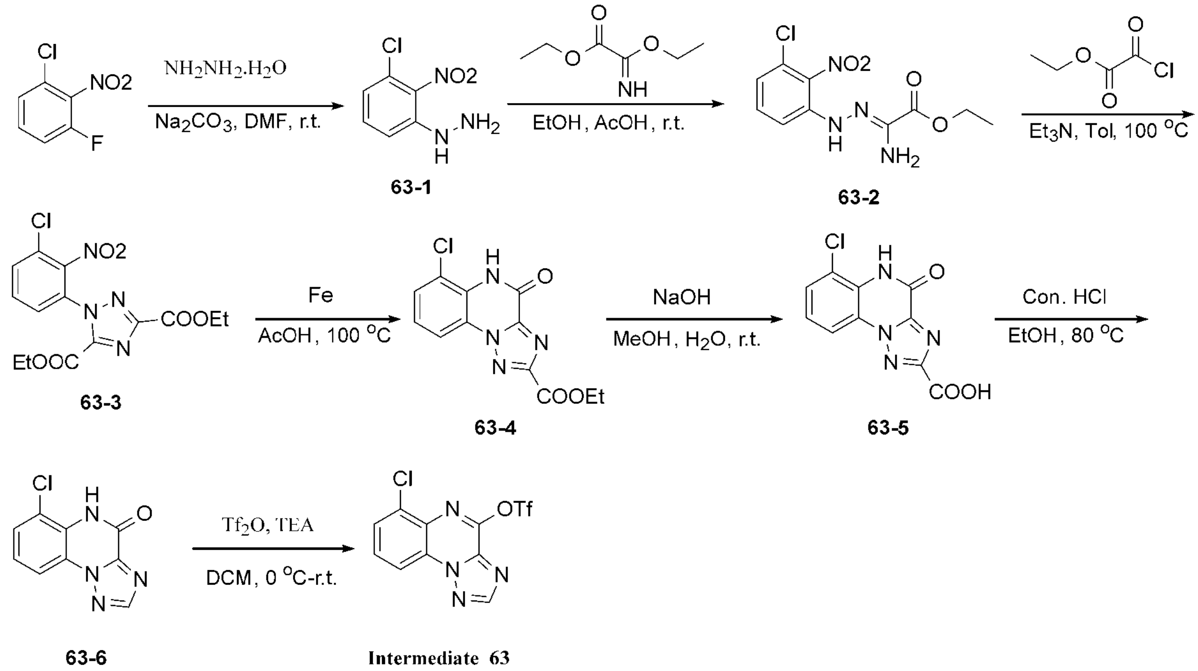


63-1 63-2 63-3 63-4 63-5 63-6 Intermediate 63

**Step 1: Synthesis of Compound 63-1**

To a solution of 2-chloro-6-fluoronitrobenzene (2 g, 11.40 mmol) in DMF (50 mL) was added hydrazinium hydroxide solution (1.34 g, 22.80 mmol) and $Na_2CO_3$ (2.42 g, 22.80 mmol). The resulting mixture was stirred at room temperature until the starting material is consumed. The reaction was quenched with water (50 mL) and extracted with EA (30 mL*3) and dried over anhydrous $Na_2SO_4$. After filtration, the filtrate was concentrated to afford a light red solid **63-1** (1.7 g, yield: 80%), ESI-MS m/z: 187.9 $[M+H]^+$.

**Step 2: Synthesis of Compound 63-2**

To a solution of **63-1** (900 mg, 4.80 mmol) and ethyl 2-ethoxy-2-iminoacetate (835 mg, 5.76 mmol) in EtOH (20 mL) was added HOAc (1 mL). The resulting mixture was stirred at room temperature until the starting material is consumed. The reaction was concentrated under reduced pressure. The reaction was then diluted with water (50 mL) and extracted with EA (30 mL*3) and dried over anhydrous $Na_2SO_4$. After filtration, the filtrate was concentrated under reduced pressure. The residue was purified by silica gel column chromatography (PE/EA = 20/1 to 5/1) to afford a light red solid **63-2** (1.2 g, yield: 88%), ESI-MS m/z: 286.9 $[M+H]^+$.

**Step 3: Synthesis of Compound 63-3**

To a solution of **63-2**(1.2 g, 4.2 mmol) in toluene (30 mL) was added TEA (1.27 g, 12.6 mmol) and ethyl oxalyl monochloride(1.15 g, 8.4 mmol). The resulting mixture was stirred at 110 °C until the starting material is consumed. The reaction was concentrated under reduced pressure. The reaction was then diluted with water (50 mL) and extracted with EA (30 mL*3) and dried over anhydrous $Na_2SO_4$. After filtration, the filtrate was concentrated under reduced pressure. The residue was purified by silica gel column chromatography (PE/EA = 20/1 to 3/1) to afford a yellow solid **63-3** (1.2 g, yield: 77%), ESI-MS m/z: 368.9 $[M+H]^+$.

**Step 4: Synthesis of Compound 63-4**

To a solution of **63-3** (1.2 g, 3.26 mmol) in HOAc (30 mL) was added Fe powder (913 mg, 16.30 mmol). The resulting mixture was stirred at 100 °C until the starting material is consumed. The reaction was filtrated and concentrated under reduced pressure to remove HOAc, then extracted with EA (50 mL*3) and dried over anhydrous $Na_2SO_4$. After filtration, the filtrate was concentrated under reduced pressure. The residue was purified by silica gel column chromatography (PE/EA = 10/1 to 2/1) to afford a yellow solid **63-4** (510 mg, yield: 54%), ESI-MS m/z: 292.9 $[M+H]^+$.

**Step 5: Synthesis of Compound 63-5**

To a solution of **63-4** (510 mg, 1.75 mmol) in MeOH (10 mL) and $H_2O$ (2 mL) was added NaOH (140 mg, 3.50 mmol). The resulting mixture was stirred at room temperature until the starting material is consumed. The reaction was concentrated under reduced pressure and neutralized with 1 M HCl, then extracted with EA (50 mL*3) and dried over anhydrous $Na_2SO_4$. After filtration, the filtrate was concentrated under reduced pressure to afford a yellow solid **63-5** (500 mg, crude), ESI-MS m/z: 264.8 $[M+H]^+$.

**Step 6: Synthesis of Compound 63-6**

To a solution of **63-5** (500 mg, crude) in EtOH (10 mL) was added con.HCl (2 mL). The resulting mixture was stirred at 80 °C until the starting material is consumed. The reaction was concentrated under reduced pressure to remove EtOH and neutralized with saturated sodium bicarbonate, then extracted with EA (50 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid **63-6** (300 mg, yield: 78%), ESI-MS m/z: 220.8 $[M+H]^+$.

**Step 7: Synthesis of Intermediate 63**

To a solution of **63-6** (300 mg, 1.36 mmol) in DCM (10 mL) was added TEA (275 mg, 2.72 mmol) and $Tf_2O$ (575 mg, 2.04 mmol) slowly at 0 °C under an argon atmosphere. The resulting mixture was stirred at room temperature until the starting material is consumed. The reaction was then quenched with water (100 mL) and extracted with DCM (50 mL*3) and dried over anhydrous $Na_2SO_4$. After filtration, the filtrate was concentrated under reduced pressure. The residue was purified by silica gel column chromatography (PE/EA = 10/1 to 3/1) to afford a white solid **intermediate 63** (330 mg, yield: 69%), ESI-MS m/z: 352.8 $[M+H]^+$.

**Preparation Example 8: Synthesis of 6-Chloro-1,2,4-triazolo [1,5-*a*] quinoxalin-4-yl trifluoromethanesulfonate (Intermediate 64)**

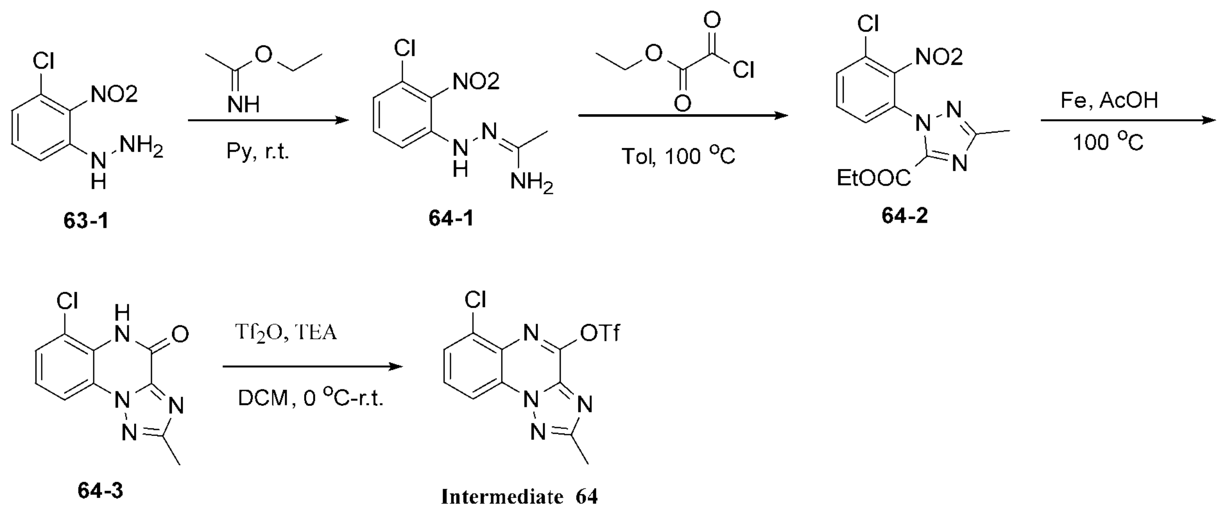


**63-1** **64-1** **64-2**

**64-3** **Intermediate 64**

**Step 1:Synthesis of Compound 64-1**

To a solution of **63-1** (1.5 g, 8.02 mmol) in Py (20 mL) was added ethyl acetimidate hydrochloride (990 mg, 8.02 mmol). The resulting mixture was stirred at room temperature until the starting material is consumed. The reaction was concentrated under reduced pressure and diluted with water (50 mL), then extracted with EA (30 mL*3) and dried over anhydrous $Na_2SO_4$. After filtration, the filtrate was concentrated under reduced pressure. The residue was purified by silica gel column chromatography (PE/EA = 20/1 to 5/1) to afford a light red solid **64-1** (800 mg, yield: 44%), ESI-MS m/z: 228.9 $[M+H]^+$.

**Step 2:Synthesis of Compound 64-2**

To a solution of **64-1**(800 mg, 3.51 mmol) in toluene(20 mL) was added ethyl acetimidate hydrochloride (958 mg, 7.02 mmol). The resulting mixture was stirred at 110 °C until the starting material is consumed. The reaction was concentrated under reduced pressure and diluted with water (50 mL), then extracted with EA (30 mL*3) and dried over anhydrous $Na_2SO_4$. After filtration, the filtrate was concentrated under reduced pressure. The residue was purified by silica gel column chromatography (PE/EA = 10/1 to 2/1) to afford a yellow solid **64-2** (680 mg, yield: 63%), ESI-MS m/z: 310.8 $[M+H]^+$.

**Step 3:Synthesis of Compound 64-3**

To a solution of **64-2** (680 mg, 2.19 mmol) in HOAc (10 mL) was added Fe powder (614 mg, 10.97 mmol). The resulting mixture was stirred at 100 °C until the starting material is consumed. The reaction was filtrated and concentrated under reduced pressure, then extracted with EA (50 mL*3) and dried over anhydrous $Na_2SO_4$. After filtration, the filtrate was concentrated under reduced pressure. The residue was purified by silica gel column chromatography (PE/EA = 10/1 to 2/1) to afford a yellow solid **64-3** (210 mg, yield: 41%), ESI-MS m/z: 234.9 $[M+H]^+$.

**Step 4:Synthesis of Intermediate 64**

To a solution of **64-3** (210 mg, 0.90 mmol) in DCM (10 mL) was added TEA (182 mg, 1.80 mmol) and $Tf_2O$ (381 mg, 1.35 mmol) slowly at 0 °C, the resulting mixture was stirred at room temperature until the starting material is consumed. The reaction was then quenched with water (50 mL) and extracted with DCM (50 mL*3) and dried over anhydrous $Na_2SO_4$. After filtration, the filtrate was concentrated under reduced pressure. The residue was purified by silica gel column chromatography (PE/EA = 10/1 to 3/1) to afford a white solid **intermediate 64** (170 mg, yield: 52%), ESI-MS m/z: 366.8 $[M+H]^+$.

**Preparation Example 9: Synthesis of 7,9-Dichloroimidazolo [1,2-*a*] quinoxalin-4-yl trifluoromethanesulfonate (Intermediate 65)**

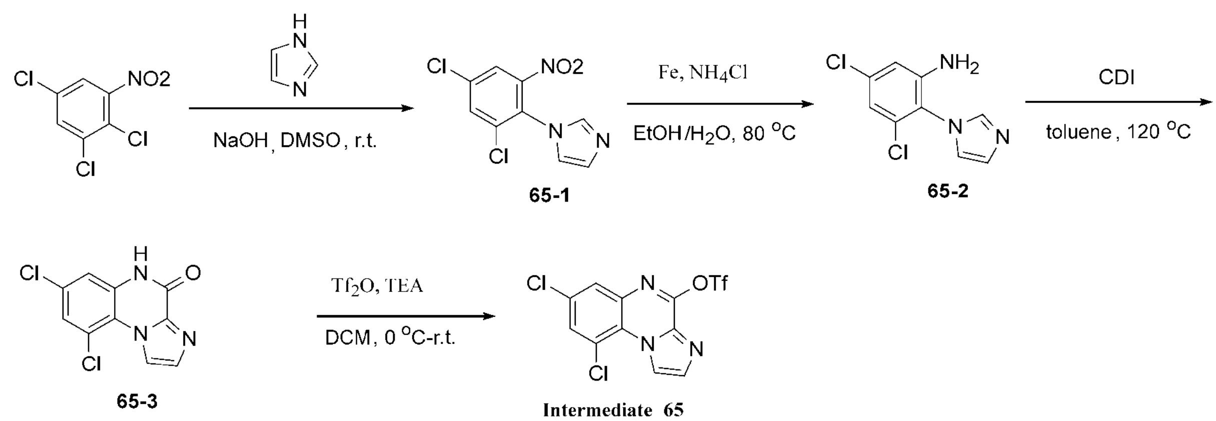


65-1

65-2

65-3

Intermediate 65

**Step 1: Synthesis of Compound 65-1**

To a solution of 2,3,5-trichloronitrobenzene (2.00 g, 8.83 mmol) in DMSO (50 mL) was added imidazole (0.6 g, 8.83 mmol) and NaOH (706 mg, 17.66 mmol). The resulting mixture was stirred at room temperature until the starting material is consumed. The reaction was then quenched with water (100 mL) and extracted with EA (50 mL*3) and dried over anhydrous $Na_2SO_4$. After filtration, the filtrate was concentrated under reduced pressure. The residue was purified by silica gel column chromatography (PE/EA = 30/1 to 10/1) to afford a white solid **65-1** (1.86 g, yield: 82%), ESI-MS m/z: 257.8 $[M+H]^+$.

**Step 2: Synthesis of Compound 65-2**

To a solution of **65-1** (1.86 g, 7.24 mmol) in $EtOH/H_2O$ (10/1, 33 mL) was added Fe powder (2.03 g, 36.2 mmol)

and $NH_4Cl$ (1.94 g, 36.2 mmol). The resulting mixture was stirred at 80 °C until the starting material is consumed. The reaction was filtrated and then extracted with EA (50 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid **65-2** (1.4 g, yield: 85%), ESI-MS m/z: 227.8 $[M+H]^+$.

**Step 3: Synthesis of Compound 65-3**

To a solution of **65-2** (1.4 g, 6.17 mmol) in toluene (30 mL) was added CDI (1.5 g, 9.26 mmol). The resulting mixture was stirred at 120 °C until the starting material is consumed. The reaction was concentrated under reduced pressure and diluted with water (100 mL), then extracted with EA (50 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid **65-3** (1.17 g, yield: 75%), ESI-MS m/z: 253.8 $[M+H]^+$.

**Step 4: Synthesis of Intermediate 65**

To a solution of **65-3** (1.17 g, 4.62 mmol) in DCM (30 mL) was added TEA (934 mg, 9.25 mmol) and $Tf_2O$ (1.56 g, 5.54 mmol) slowly at 0 °C under an argon atmosphere. The resulting mixture was stirred at room temperature until the starting material is consumed. The reaction was then quenched with water (100 mL) and extracted with DCM (50 mL*3) and dried over anhydrous $Na_2SO_4$. After filtration, the filtrate was concentrated under reduced pressure. The residue was purified by silica gel column chromatography (PE/EA = 20/1 to 10/1) to afford a white solid **intermediate 65** (1.46 g, yield: 82%), ESI-MS m/z: 385.7 $[M+H]^+$.

**Intermediates 66-67** can be synthesized by a method similar to **Intermediate 65,** using imidazole and variously substituted nitrobenzene as starting materials.

| Compound | Structure | Name | ESI-MS: $[M+H]^+$ |
|---|---|---|---|
| **Intermediate 66** | 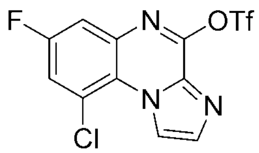 | 9-chloro-7-fluoroimidazo [1,2-*a*] quinoxalin-4-yl trifluoromethanesulfonate | 369.8 |
| **Intermediate 67** | 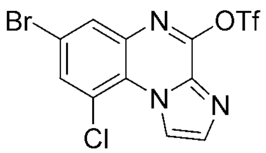 | 7-bromo-9-chloroimidazolo [1,2-*a*] quinoxalin-4-yl trifluoromethanesulfonate | 429.7 |

**Preparation Example 10: Synthesis of 4-chloro-9-fluoro-1,2-dihydroimidazo [1,2-*a*] quinoxaline (Intermediate 68)**

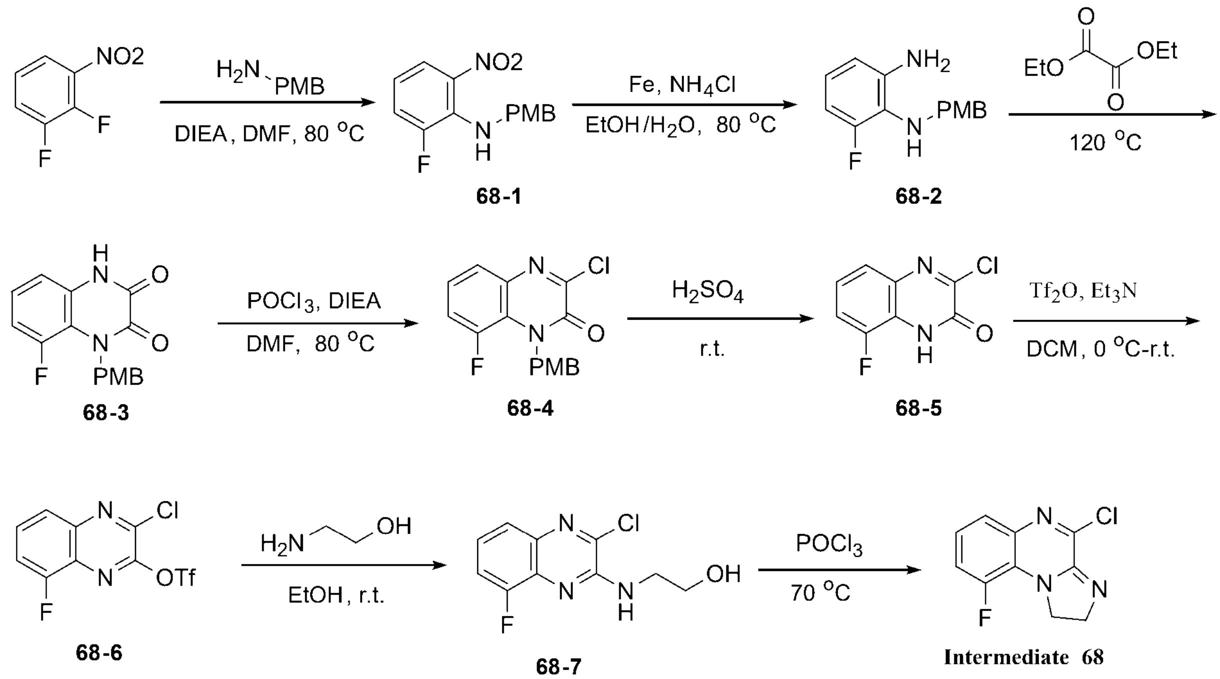

68-1 68-2 68-3 68-4 68-5 68-6 68-7 Intermediate 68

**Step 1: Synthesis of Compound 68-1**

To a solution of 2,3-difluoronitrobenzene (5.00 g, 31.45 mmol) in DMF (100 mL) was added 4-methoxybenzylamine (4.31 g, 31.45 mmol) and DIEA (8.11 g, 62.9 mmol). The resulting mixture was stirred at 80 °C until the starting material is consumed. The reaction was then diluted with water (200 mL) and extracted with EA (50 mL*3) and dried over anhydrous $Na_2SO_4$. After filtration, the filtrate was concentrated under reduced pressure. The residue was purified by silica gel column chromatography (PE/EA = 30/1 to 10/1) to afford a yellow solid **68-1** (5.9 g, yield: 68%), ESI-MS m/z: 276.9 $[M+H]^+$.

**Step 2: Synthesis of Compound 68-2**

To a solution of **68-1** (5.9 g, 21.38 mmol) in EtOH/$H_2O$ (10/1, 110 mL) was added Fe powder (5.99 g, 106.9 mmol) and $NH_4Cl$ (5.72 g, 106.9 mmol). The resulting mixture was stirred at 80 °C until the starting material is consumed. The reaction was filtrated and then extracted with EA (30 mL*3) and dried over anhydrous $Na_2SO_4$. After filtration, the filtrate was concentrated under reduced pressure. The residue was purified by silica gel column chromatography (PE/EA = 30/1 to 10/1) to afford a yellow solid **68-2** (7.62 g, yield: 88%), ESI-MS m/z: 276.9 $[M+H]^+$.

**Step 3: Synthesis of Compound 68-3**

**68-2** (7.62 g, 27.61 mmol) was dissolved in diethyl oxalate (50 mL). The resulting mixture was stirred at 120 °C until the starting material is consumed. The reaction was cooled to room temperature and filtrated to afford a white solid **68-3** (5.72g, yield: 69%), ESI-MS m/z: 300.9 $[M+H]^+$.

**Step 4: Synthesis of Compound 68-4**

To a solution of **68-3** (5.72 g, 19.07 mmol) in DMF (50 mL) was added DIEA (4.92 g, 38.14 mmol) and $POCl_3$ (8.77 g, 57.21 mmol). The resulting mixture was stirred at 80 °C until the starting material is consumed. The reaction was quenched with water (200 mL) and extracted with EA (50 mL*3) and dried over anhydrous $Na_2SO_4$. After filtration, the filtrate was concentrated under reduced pressure. The residue was purified by silica gel column chromatography (PE/EA = 10/1 to 5/1) to afford a yellow solid **68-4** (3.27 g, yield: 54%), ESI-MS m/z: 318.9 $[M+H]^+$.

**Step 5: Synthesis of Compound 68-5**

68-4 (3.27 g, 10.28 mmol) was dissolved in con.$H_2SO_4$ (10 mL). The resulting mixture was stirred at room temperature until the starting material is consumed. The reaction was quenched with water and then extracted with EA (50

mL*3) and dried over anhydrous $Na_2SO_4$. After filtration, the filtrate was concentrated under reduced pressure. The residue was purified by silica gel column chromatography (PE/EA = 5/1 to 1/1) to afford a yellow solid **68-5** (1.32 g, yield: 65%), ESI-MS m/z: 198.8 $[M+H]^+$.

**Step 6: Synthesis of Compound 68-6**

To a solution of **68-5** (1.32 g, 6.67 mmol) in DCM (30 mL) was added TEA (1.35 g, 13.33 mmol) and $Tf_2O$ (2.26 g, 8.00 mmol) slowly at 0 °C under an argon atmosphere. The resulting mixture was stirred at room temperature until the starting material is consumed. The reaction was then quenched with water (50 mL) and extracted with DCM (30 mL*3) and dried over anhydrous $Na_2SO_4$. After filtration, the filtrate was concentrated under reduced pressure. The residue was purified by silica gel column chromatography (PE/EA = 10/1 to 3/1) to afford a yellow solid **68-6** (1.94 g, yield: 88%), ESI-MS m/z: 330.8 $[M+H]^+$.

**Step 7: Synthesis of Compound 68-7**

To a solution of **68-6** (1.94 g, 5.88 mmol) in EtOH (30 mL) was added ethanolamine (717 mg, 11.76 mmol). The resulting mixture was stirred at room temperature until the starting material is consumed. The reaction was concentrated under reduced pressure and diluted with water (200 mL). The resulting mixture was extracted with EA (50 mL*3) and dried over anhydrous $Na_2SO_4$. After filtration, the filtrate was concentrated under reduced pressure. The residue was purified by silica gel column chromatography (PE/EA = 3/1 to 1/1) to afford a yellow solid **68-7** (1.08 g, yield: 76%), ESI-MS m/z: 241.8 $[M+H]^+$.

**Step 8: Synthesis of Intermediate 68**

**68-7** (1.08 g, 4.48 mmol) was dissolved in $POCl_3$ (20 mL). The resulting mixture was stirred at 70 °C until the starting material is consumed. The reaction was concentrated under reduced pressure and quenched with water (100 mL), then extracted with EA (30 mL*3) and dried over anhydrous $Na_2SO_4$. After filtration, the filtrate was concentrated under reduced pressure. The residue was purified by silica gel column chromatography (PE/EA = 5/1 to 0/1) to afford a yellow solid **intermediate 68** (759 mg, yield: 76%), ESI-MS m/z: 223.8 $[M+H]^+$.

**Intermediates 69-74** can be synthesized by a method similar to **Intermediate 68,** using variously substituted nitrobenzene as starting materials.

| Compound | Structure | Name | ESI-MS: $[M+H]^+$ |
|---|---|---|---|
| **Intermediate 69** | 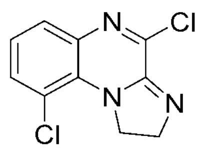 | 4,9-dichloro-1,2- dihydroimidazo [1,2-*a*] quinoxaline | 239.8 |
| **Intermediate 70** | 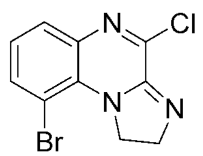 | 4-chloro-9-bromo-1,2-dihydroimidazo [1,2-*a*] quinoxaline | 283.8 |
| **Intermediate 71** | 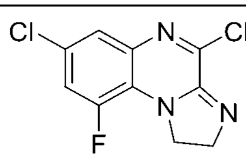 | 4,7-dichloro-9-fluoro-1,2-dihydroimidazo [1,2-*a*] quinoxaline | 257.8 |
| **Intermediate 72** | 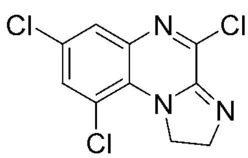 | 4,7,9-trichloro-1,2-dihydroimidazo [1,2-*a*] quinoxalin-4-yl trifluoromethanesulfonate | 273.8 |

(continued)

| Compound | Structure | Name | ESI-MS: $[M+H]^+$ |
|---|---|---|---|
| **Intermediate 73** | 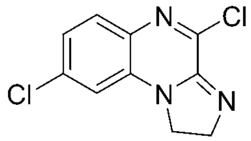 | 4,8-dichloro-1,2- dihydroimidazo [1,2-*a*] quinoxaline | 239.8 |
| **Intermediate 74** | 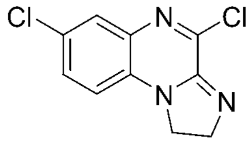 | 4,7-dichloro-1,2- dihydroimidazo [1,2-*a*] quinoxaline | 239.8 |

**Example 1: Synthesis of 5-Chloro-1-methyl-3-((4-(trifluoromethoxy)phenyl)amino) quinoxalin-2(1H)-one (Compound 1)**

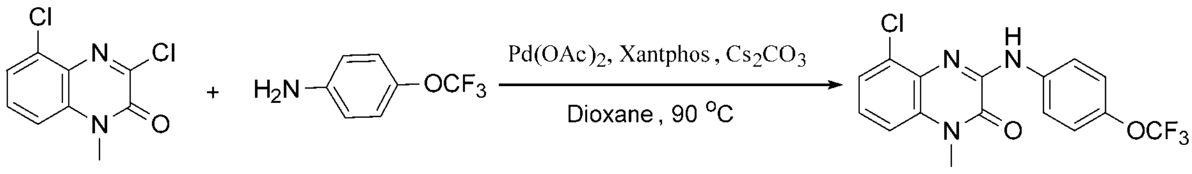


Intermediate 1

1

To a solution of **intermediate 1** (50 mg, 0.22 mmol) and 4- (trifluoromethoxy) aniline (39 mg, 0.22 mmol) in Dioxane (3 mL) was added $Pd(OAc)_2$ (5 mg, 0.022 mmol), Xantphos (26 mg, 0.044 mmol) and $Cs_2CO_3$ (143 mg, 0.44 mmol) under an argon atmosphere. The resulting mixture was stirred at 90 °C until the starting material is consumed. The reaction was diluted with water (20 mL) and extracted with EA (20 mL*3) and dried over anhydrous $Na_2SO_4$. After filtration, the filtrate was concentrated under reduced pressure. The residue was purified by silica gel column chromatography (PE/EA = 10/1 to 1/1) to afford a yellow solid 1 (51 mg, yield: 64%).

$^1$H NMR (400 MHz, DMSO-$d_6$): δ 9.99 (s, 1H), 8.48 (d, *J* = 9.2 Hz, 2H), 7.50-7.45 (m, 2H), 7.39 (d, *J* = 8.7 Hz, 2H), 7.37-7.32 (m, 1H), 3.71 (s, 3H); ESI-MS m/z: 369.9 $[M+H]^+$.

**Examples 2-94: Synthesis of Compounds 2-38 and 41-96**

**Compounds 2-38 and 41-96** can be synthesized by a method similar to **Example 1,** using **Intermediates 1-9, 55-64** and corresponding amines as starting materials.

| Compound | Structure | $^1$H NMR (400 MHz, DMSO-$d_6$) δ | ESI-MS: $[M+H]^+$ |
|---|---|---|---|
| 2 | 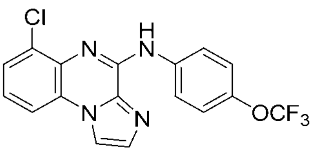 | 10.33 (s, 1H), 8.79 (s, 1H), 8.57 (d, *J* = 9.2 Hz, 2H), 8.23 (d, *J* = 8.2 Hz, 1H), 7.81 (s, 1H), 7.70 (d, *J* = 6.9 Hz, 1H), 7.43 (dd, *J* = 17.2, 8.9 Hz, 3H) | 379.0 |
| 3 | 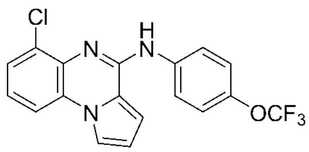 | 10.13 (s, 1H), 8.59 (s, 1H), 8.27 (d, *J* = 9.2 Hz, 2H), 8.03 (d, *J* = 8.2 Hz, 1H), 7.71 (s, 1H), 7.50 (d, *J* = 6.9 Hz, 1H), 7.03 (m, 4H) | 377.9 |

(continued)

| Compound | Structure | $^1H$ NMR (400 MHz, DMSO-$d_6$) δ | ESI-MS: $[M+H]^+$ |
|---|---|---|---|
| **4** | 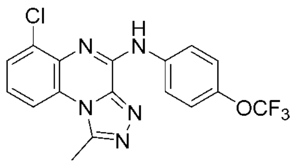 | 10.58 (s, 1H), 10.11 (s, 1H), 8.78 (d,*J* = 2.2 Hz, 1H), 8.26 (dd, *J* = 8.2, 1.1 Hz, 1H), 8.03 (dd, *J* = 8.9, 2.2 Hz, 1H), 7.74 (m, 2H), 7.42 (dd, *J* = 8.4, 7.6 Hz, 1H), 2.68 (s, 3H) | 394.0 |
| **5** | 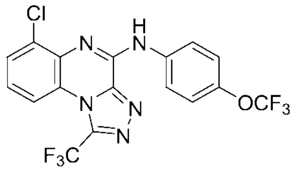 | 10.97 (s, 1H), 8.65 (d, *J* = 2.1 Hz, 1H), 8.04 (dd, *J* = 8.9, 2.1 Hz, 1H), 7.82 (dd, *J* = 15.2, 8.1 Hz, 2H), 7.49 (t, *J* = 8.3 Hz, 1H), 7.41 (m, 2H) | 447.9 |
| **6** | 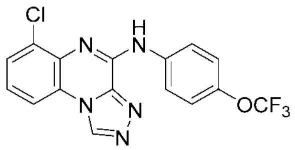 | 10.88 (s, 1H), 10.10 (s, 1H), 8.78 (d, *J* = 2.1 Hz, 1H), 8.25 (dd, *J* = 8.2, 1.1 Hz, 1H), 8.09 (dd, *J* = 8.9, 2.2 Hz, 1H), 7.74 (m, 2H), 7.48 (dd, *J* = 8.4, 7.6 Hz, 2H) | 379.9 |
| **7** | 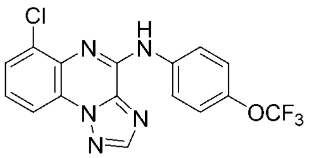 | 10.58 (s, 1H), 10.15 (s, 1H), 8.77 (d, *J* = 2.2 Hz, 1H), 8.20 (dd, *J* = 8.2, 1.3 Hz, 1H), 8.01 (dd, *J* = 8.9, 2.3 Hz, 1H), 7.84 (m, 2H), 7.45 (dd, *J* = 8.5, 7.6 Hz, 2H) | 379.9 |
| **8** | 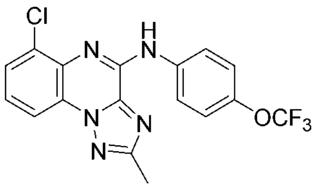 | 10.64 (s, 1H), 8.82 (d, *J* = 2.2 Hz, 1H), 8.17 (dd, *J* = 8.2, 1.3 Hz, 1H), 8.13 (dd, *J* = 8.9, 2.1 Hz, 1H), 7.82 (dd, *J* = 7.9, 1.2 Hz, 1H), 7.55 (m, 3H), 2.60 (s, 3H) | 394.0 |
| **9** | 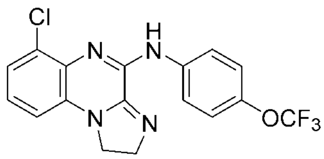 | 9.76 (s, 1H), 8.42 (d, *J* = 9.1 Hz, 2H), 7.37 (d, *J* = 8.6 Hz, 2H), 7.20 (d, *J* = 4.6 Hz, 2H), 6.96-6.84 (m, 1H), 4.27-4.11 (m, 4H) | 381.1 |
| **10** | 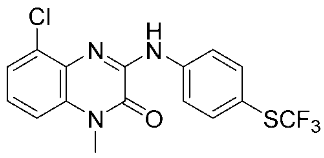 | 10.11 (s, 1H), 8.53 (d, *J* = 8.8 Hz, 2H), 7.72 (d, *J* = 8.7 Hz, 2H), 7.52-7.46 (m, 2H), 7.38 (d, *J* = 7.9 Hz, 1H), 3.71 (s, 3H) | 385.9 |
| **11** | 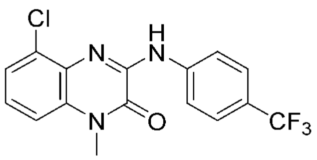 | 10.51 (s, 1H), 9.13 (d, *J* = 8.8 Hz, 2H), 8.52 (d, *J* = 8.7 Hz, 2H), 7.96 (m, 2H), 7.58 (d, *J* = 7.9 Hz, 1H), 3.91 (s, 3H) | 353.9 |
| **12** | 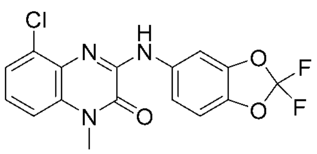 | 10.06 (s, 1H), 8.71 (d, *J* = 2.1 Hz, 1H), 8.06 (dd, *J* = 8.9, 2.2 Hz, 1H), 7.47 (td, *J* = 7.9, 1.1 Hz, 2H), 7.42 (d, *J* = 8.9 Hz, 1H), 7.35 (d, *J* = 8.0 Hz, 1H), 3.70 (s, 3H) | 365.9 |

(continued)

| Compound | Structure | $^1H$ NMR (400 MHz, DMSO-$d_6$) δ | ESI-MS: $[M+H]^+$ |
|---|---|---|---|
| **13** | 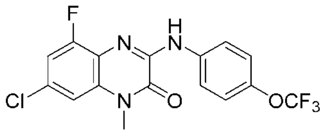 | 10.11 (s, 1H), 8.58 (d, *J* = 9.2 Hz, 2H), 7.55 (m, 1H), 7.39 (m, 3H), 3.81 (s, 3H) | 387.9 |
| **14** | 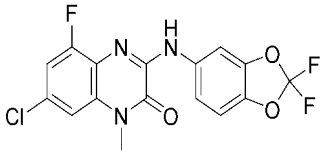 | 10.03 (s, 1H), 8.40 (d, *J* = 2.1 Hz, 1H), 7.97 (dd, *J* = 8.9, 2.1 Hz, 1H), 7.44-7.36 (m, 3H), 3.66 (s, 3H) | 383.9 |
| **15** | 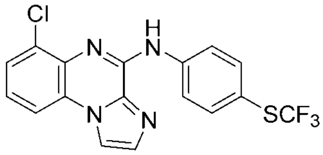 | 10.43 (s, 1H), 8.78 (d, *J* = 1.0 Hz, 1H), 8.60 (d, *J* = 8.8 Hz, 2H), 8.22 (dd, *J* = 8.2, 0.9 Hz, 1H), 7.81 (d, *J* = 1.0 Hz, 1H), 7.74-7.66 (m, 3H), 7.45 (t, *J* = 8.1 Hz, 1H) | 394.9 |
| **16** | 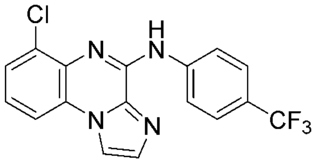 | 10.50 (s, 1H), 8.81 (d, *J* = 1.0 Hz, 1H), 8.65 (d, *J* = 8.7 Hz, 2H), 8.24 (d, *J* = 7.4 Hz, 1H), 7.82 (d, *J* = 0.9 Hz, 1H), 7.72 (dd, *J* = 16.5, 7.9 Hz, 3H), 7.46 (t, *J* = 8.1 Hz, 1H) | 363.0 |
| **17** | 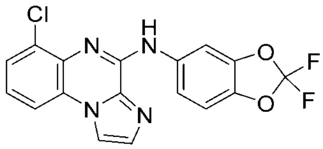 | 10.33 (s, 1H), 8.80 (d, *J* = 2.1 Hz, 1H), 8.74 (d, *J* = 1.0 Hz, 1H), 8.20-8.16 (m, 1H), 8.05 (dd, *J* = 8.9, 2.1 Hz, 1H), 7.77 (d, *J* = 1.0 Hz, 1H), 7.65 (dd, *J* = 7.9, 0.9 Hz, 1H), 7.43-7.38 (m, 2H) | 374.9 |
| **18** | 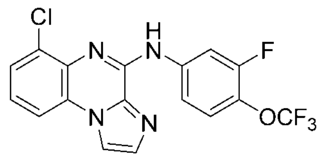 | 10.53 (s, 1H), 8.89 (dd, *J* = 14.2, 2.4 Hz, 1H), 8.78 (d, *J* = 0.9 Hz, 1H), 8.21 (d, *J* = 8.3 Hz, 1H), 8.16 (d, *J* = 8.4 Hz, 1H), 7.80 (d, *J* = 0.9 Hz, 1H), 7.68 (d, *J* = 7.9 Hz, 1H), 7.56 (t, *J* = 9.0 Hz, 1H), 7.45 (t, *J* = 8.1 Hz, 1H) | 396.9 |
| **19** | 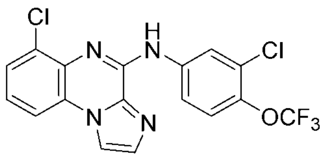 | 10.54 (s, 1H), 9.16 (d, *J* = 2.6 Hz, 1H), 8.79 (d, *J* = 1.1 Hz, 1H), 8.29 (dd, *J* = 9.1, 2.6 Hz, 1H), 8.23 (dd, *J* = 8.3, 1.0 Hz, 1H), 7.81 (d, *J* = 1.1 Hz, 1H), 7.70 (dd, *J* = 7.9, 1.0 Hz, 1H), 7.60-7.56 (m, 1H), 7.46 (t, *J* = 8.1 Hz, 1H) | 412.8 |
| **20** | 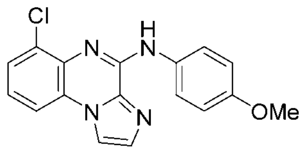 | 10.13 (s, 1H), 8.70 (s, 1H), 8.27 (d, *J* = 9.1 Hz, 2H), 8.03 (d, *J* = 8.1 Hz, 1H), 7.71 (s, 1H), 7.60 (d, *J* = 6.7 Hz, 1H), 7.43 (m, 3H), 3.93 (s, 3H) | 325.1 |
| **21** | 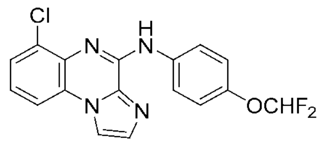 | 10.23 (s, 1H), 8.75 (s, 1H), 8.47 (d, *J* = 9.2 Hz, 2H), 8.13 (d, *J* = 8.1 Hz, 1H), 7.81 (s, 1H), 7.60 (d, *J* = 6.9 Hz, 1H), 7.43-7.01 (m, 4H) | 361.1 |

(continued)

| Compound | Structure | $^1$H NMR (400 MHz, DMSO-$d_6$) δ | ESI-MS: [M+H]$^+$ |
|---|---|---|---|
| **22** | 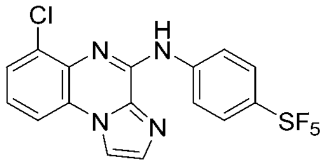 | 10.43 (s, 1H), 8.89 (s, 1H), 8.67 (d, *J* = 9.2 Hz, 2H), 8.33 (d, *J* = 8.4 Hz, 1H), 7.91 (s, 1H), 7.60 (m, 1H), 7.43 (m, 3H) | 420.8 |
| **23** | 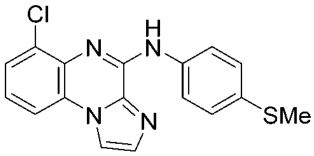 | 10.03 (s, 1H), 8.50 (s, 1H), 8.17 (d, *J* = 9.1 Hz, 2H), 7.83 (d, *J* = 8.1 Hz, 1H), 7.51 (s, 1H), 7.20 (d, *J* = 6.7 Hz, 1H), 7.03 (m, 3H), 2.45 (s, 3H) | 340.9 |
| **24** | 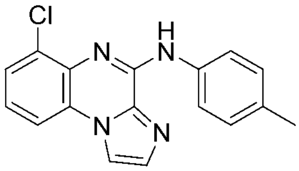 | 10.23 (s, 1H), 8.70 (s, 1H), 8.27 (m, 2H), 7.99 (d, *J* = 8.1 Hz, 1H), 7.71 (s, 1H), 7.50 (d, *J* = 6.9 Hz, 1H), 7.33 (m, 3H), 2.15 (s, 3H) | 309.1 |
| **25** | 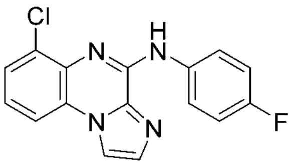 | 10.43 (s, 1H), 8.90 (s, 1H), 8.47 (m, 2H), 8.29 (d, *J* = 8.2 Hz, 1H), 7.91 (s, 1H), 7.70 (d, *J* = 6.6 Hz, 1H), 7.43 (m, 3H) | 313.1 |
| **26** | 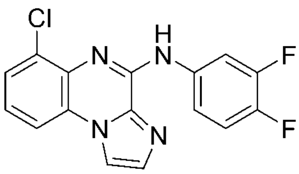 | 10.63 (s, 1H), 9.10 (s, 1H), 8.67 (m, 2H), 8.39 (d, *J* = 8.6 Hz, 1H), 7.99 (s, 1H), 7.75 (d, *J* = 6.7 Hz, 1H), 7.63 (m, 2H) | 331.1 |
| **27** | 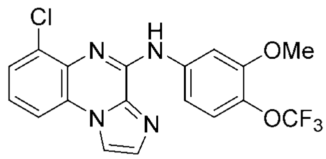 | 10.33 (s, 1H), 8.70 (d, *J* = 2.1 Hz, 1H), 8.54 (d, *J* = 1.0 Hz, 1H), 8.26 (m, 1H), 8.01 (dd, *J* = 8.9, 2.1 Hz, 1H), 7.72 (d, *J* = 1.0 Hz, 1H), 7.60 (dd, *J* = 7.9, 0.9 Hz, 1H), 7.38 (m, 2H), 3.68 (s, 3H) | 409.1 |
| **28** | 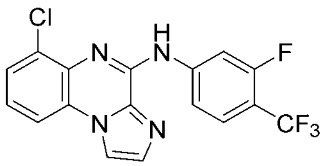 | 10.75 (s, 1H), 8.91-8.79 (m, 2H), 8.25 (d, *J* = 8.2 Hz, 2H), 7.83 (s, 1H), 7.75 (dd, *J* = 19.5, 8.3 Hz, 2H), 7.49 (t, *J* = 8.1 Hz, 1H) | 381.1 |
| **29** | 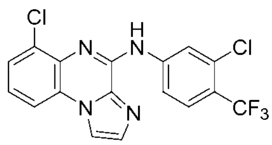 | 10.72 (s, 1H), 9.18 (s, 1H), 8.82 (s, 1H), 8.37 (d, *J* = 9.8 Hz, 1H), 8.25 (d, *J* = 8.2 Hz, 1H), 7.84 (d, *J* = 7.5 Hz, 2H), 7.72 (d, *J* = 7.8 Hz, 1H), 7.49 (t, *J* = 8.1 Hz, 1H) | 396.8 |
| **30** | 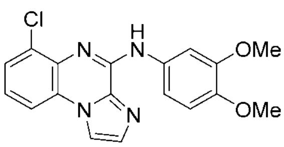 | 10.03 (s, 1H), 8.50 (d, *J* = 2.1 Hz, 1H), 8.24 (d, *J* = 1.2 Hz, 1H), 8.06 (m, 1H), 7.81 (dd, *J* = 8.9, 2.1 Hz, 1H), 7.42 (d, *J* = 1.0 Hz, 1H), 7.20 (dd, *J* = 7.9, 0.9 Hz, 1H), 7.08 (m, 2H), 3.78 (s, 3H), 3.68 (s, 3H) | 355.1 |

(continued)

| Compound | Structure | $^1H$ NMR (400 MHz, DMSO-$d_6$) δ | ESI-MS: $[M+H]^+$ |
|---|---|---|---|
| **31** | 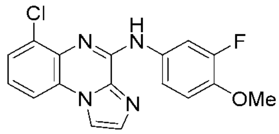 | 10.13 (s, 1H), 9.01 (s, 1H), 8.57 (m, 2H), 8.19 (d, *J* = 8.6 Hz, 1H), 7.79 (s, 1H), 7.55 (d, *J*= 6.7 Hz, 1H), 7.33 (m, 2H), 3.88 (s, 3H) | 343.1 |
| **32** | 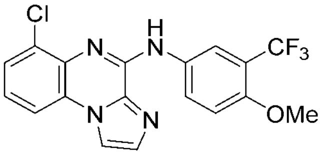 | 10.33 (s, 1H), 9.31 (s, 1H), 8.87 (m, 2H), 8.49 (d, *J* = 8.6 Hz, 1H), 7.89 (s, 1H), 7.65 (d, *J* = 6.7 Hz, 1H), 7.43 (m, 2H), 4.08 (s, 3H) | 393.1 |
| **33** | 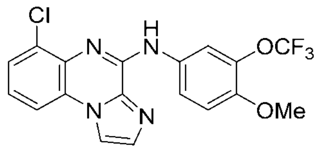 | 10.03 (s, 1H), 9.11 (s, 1H), 8.77 (m, 2H), 8.29 (d, *J* = 8.6 Hz, 1H), 7.81 (s, 1H), 7.62 *(d, J* = 6.7 Hz, 1H), 7.33 (m, 2H), 3.68 (s, 3H) | 409.1 |
| **34** | 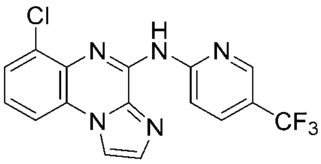 | 11.03 (s, 1H), 10.29 (s, 1H), 9.27 (m, 2H), 8.93 (d, *J* = 8.4 Hz, 1H), 8.31 (s, 1H), 8.05 (d, *J* = 6.9 Hz, 1H), 7.83 (m, 2H) | 364.1 |
| **35** | 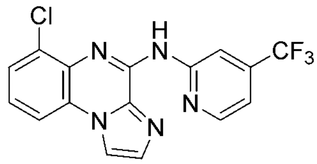 | 11.13 (s, 1H), 10.19 (s, 1H), 9.37 (m, 2H), 8.83 (d, *J* = 8.4 Hz, 1H), 8.37 (s, 1H), 8.25 (d, *J* = 6.9 Hz, 2H), 7.93 (s, 1H) | 364.1 |
| **36** | 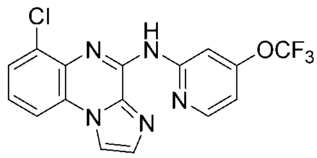 | 10.53 (s, 1H), 9.69 (s, 1H), 9.07 (m, 2H), 8.53 (d, *J* = 8.4 Hz, 1H), 8.17 (s, 1H), 7.89 (d, *J* = 6.9 Hz, 2H), 7.63 (s, 1H) | 380.1 |
| **37** | 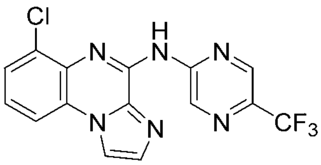 | 11.13 (s, 1H), 10.39 (s, 1H), 9.37 (m, 2H), 8.95 (d, *J* = 8.4 Hz, 1H), 8.39 (s, 1H), 8.25 (d, *J* = 6.9 Hz, 1H), 7.93 (s, 1H) | 365.1 |
| **38** | 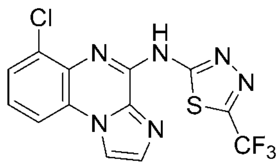 | 12.13 (s, 1H), 9.79 (s, 1H), 8.78 (d, *J* = 8.4 Hz, 1H), 8.31 (s, 1H), 8.10 (d, *J* = 6.5 Hz, 1H), 7.73 (m, 1H) | 370.8 |
| **41** | 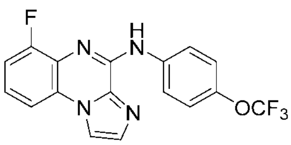 | 10.21 (s, 1H), 8.75 (s, 1H), 8.39 (d, *J* = 9.1 Hz, 2H), 8.05 (d, *J* = 7.9 Hz, 1H), 7.79 (s, 1H), 7.47-7.31 (m, 4H) | 363.1 |

(continued)

| Compound | Structure | $^1$H NMR (400 MHz, DMSO-$d_6$) δ | ESI-MS: [M+H]$^+$ |
|---|---|---|---|
| **42** | 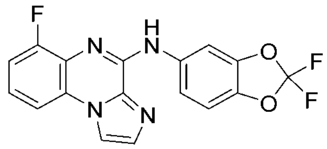 | 10.29 (s, 1H), 8.76 (s, 1H), 8.55 (d, *J* = 2.1 Hz, 1H), 8.09-7.97 (m, 2H), 7.79 (s, 1H), 7.51-7.30 (m, 3H) | 359.1 |
| **43** | 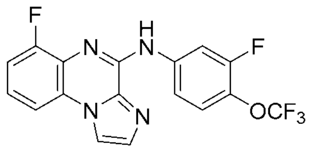 | 8.79 (d, *J* = 1.1 Hz, 1H), 8.69 (d, *J* = 2.5 Hz, 1H), 8.66 (d, *J* = 2.5 Hz, 1H), 8.15-8.06 (m, 2H), 7.82 (d, *J* = 1.0 Hz, 1H), 7.59 (t, *J* = 8.8 Hz, 1H), 7.51-7.38 (m, 2H) | 380.9 |
| **44** | 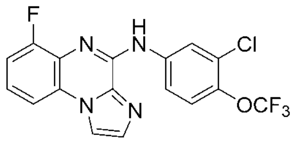 | 10.46 (s, 1H), 8.86 (d, *J* = 2.5 Hz, 1H), 8.78 (s, 1H), 8.28 (dd, *J* = 9.1, 2.6 Hz, 1H), 8.07 (d, *J* = 8.0 Hz, 1H), 7.81 (s, 1H), 7.59 (d, *J* = 9.0 Hz, 1H), 7.50-7.35 (m, 2H) | 396.9 |
| **45** | 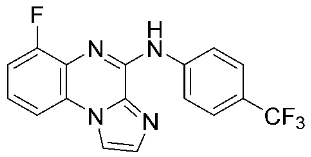 | 10.41 (s, 1H), 8.79 (s, 1H), 8.51 (d,*J* = 8.6 Hz, 2H), 8.08 (d, *J* = 8.1 Hz, 1H), 7.82 (s, 1H), 7.74 (d, *J* = 8.7 Hz, 2H), 7.45 (dt, *J* = 18.7, 8.2 Hz, 2H) | 347.1 |
| **46** | 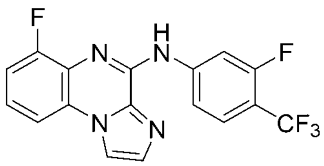 | 10.66 (s, 1H), 8.79 (s, 1H), 8.65 (d, *J*= 14.5 Hz, 1H), 8.20 (d, *J* = 8.6 Hz, 1H), 8.08 (d, *J* = 8.1 Hz, 1H), 7.77 (dd,*J* = 21.8, 13.1 Hz, 2H), 7.52-7.35 (m, 2H) | 365.1 |
| **47** | 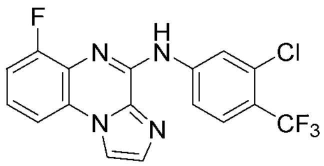 | 10.64 (d, *J* = 5.1 Hz, 1H), 8.89 (s, 1H), 8.80 (d, *J* = 2.7 Hz, 1H), 8.36 (d, *J* = 8.8 Hz, 1H), 8.12-8.05 (m, 1H), 7.84 (dd, *J* = 11.1, 2.2 Hz, 2H), 7.53 - 7.37 (m, 2H) | 381.1 |
| **48** | 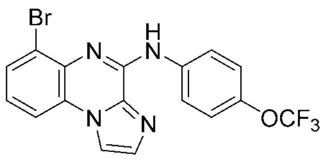 | 10.35 (s, 1H), 8.79 (s, 1H), 8.60 (d, *J* = 9.2 Hz, 2H), 8.26 (d, *J* = 8.2 Hz, 1H), 7.85 (d, *J* = 7.8 Hz, 1H), 7.81 (s, 1H), 7.42-7.34 (m, 3H) | 422.9 |
| **49** | 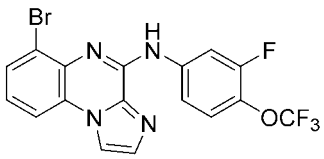 | 10.56 (s, 1H), 8.95 (dd, *J* = 14.2, 2.5 Hz, 1H), 8.80 (s, 1H), 8.28 (d, *J* = 8.2 Hz, 1H), 8.21 (d, *J* = 9.8 Hz, 1H), 7.88 (d, *J* = 7.9 Hz, 1H), 7.82 (s, 1H), 7.57 (t, *J* = 8.9 Hz, 1H), 7.41 (t, *J* = 8.0 Hz, 1H) | 440.9 |
| **50** | 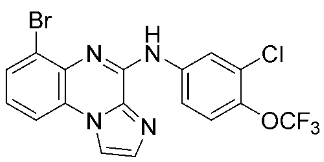 | 10.55 (s, 1H), 9.23 (d, *J* = 2.6 Hz, 1H), 8.80 (s, 1H), 8.35-8.23 (m, 2H), 7.88 (d, *J* = 7.8 Hz, 1H), 7.82 (d, *J* = 1.0 Hz, 1H), 7.58 (d, *J* = 10.0 Hz, 1H), 7.40 (t, *J* = 8.1 Hz, 1H) | 456.9 |

(continued)

| Compound | Structure | $^1$H NMR (400 MHz, DMSO-$d_6$) δ | ESI-MS: $[M+H]^+$ |
|---|---|---|---|
| **51** | 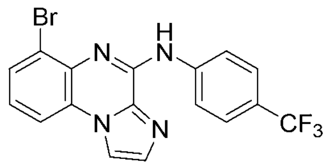 | 10.47 (s, 1H), 8.80 (s, 1H), 8.69 (d, *J* = 8.6 Hz, 2H), 8.27 (d, *J* = 8.2 Hz, 1H), 7.87 (d, *J* = 7.8 Hz, 1H), 7.82 (s, 1H), 7.74 (d, *J* = 8.7 Hz, 2H), 7.40 (t, *J* = 8.0 Hz, 1H) | 407.0 |
| **52** | 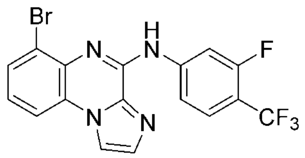 | 10.74 (s, 1H), 8.91 (d, *J* = 14.9 Hz, 1H), 8.81 (s, 1H), 8.29 (d, *J* = 8.3 Hz, 2H), 7.89 (d, *J* = 7.2 Hz, 1H), 7.84 (s, 1H), 7.76 (t, *J* = 8.7 Hz, 1H), 7.43 (t, *J* = 8.1 Hz, 1H) | 424.9 |
| **53** | 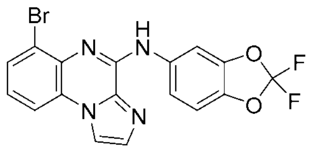 | 10.23 (s, 1H), 8.70 (d, *J* = 2.2 Hz, 1H), 8.64 (d, *J* = 1.1 Hz, 1H), 8.16 (m, 1H), 7.85 (dd, *J* = 8.8, 2.1 Hz, 1H), 7.67 (d, *J* = 1.0 Hz, 1H), 7.55 (m, 1H), 7.38 (m, 2H) | 418.9 |
| **54** | 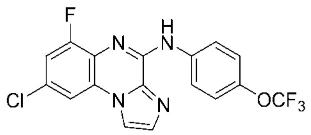 | 10.32 (s, 1H), 8.79 (d, *J* = 1.2 Hz, 1H), 8.39-8.33 (m, 2H), 8.31 (d, *J* = 1.8 Hz, 1H), 7.80 (d, *J* = 1.1 Hz, 1H), 7.59 (dd, *J* = 10.3, 2.1 Hz, 1H), 7.40 (d, *J* = 8.6 Hz, 2H) | 396.9 |
| **55** | 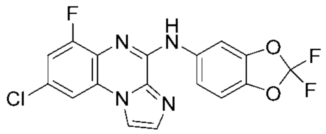 | 10.34 (s, 1H), 8.79 (d, *J* = 1.3 Hz, 1H), 8.49 (d, *J* = 2.1 Hz, 1H), 8.32-8.28 (m, 1H), 7.99 (dd, *J* = 8.9, 2.1 Hz, 1H), 7.79 (d, *J* = 1.2 Hz, 1H), 7.59 (dd, *J* = 10.3, 2.2 Hz, 1H), 7.43 (d, *J* = 8.9 Hz, 1H) | 392.9 |
| **56** | 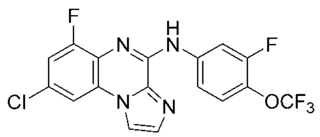 | 10.28 (s, 1H), 8.57 (s, 1H), 8.38 (d, *J* = 13.8 Hz, 1H), 8.08 (s, 1H), 7.87 (d, *J* = 8.9 Hz, 1H), 7.58 (s, 1H), 7.35 (t, *J* = 10.5 Hz, 2H) | 414.9 |
| **57** | 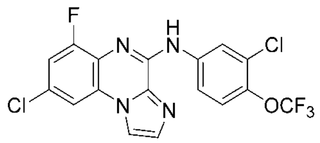 | 10.52 (s, 1H), 8.81 (d, *J* = 2.4 Hz, 2H), 8.32 (d, *J* = 1.7 Hz, 1H), 8.25 (dd, *J* = 9.1, 2.6 Hz, 1H), 7.81 (d, *J* = 1.1 Hz, 1H), 7.60 (ddd, *J* = 7.2, 6.0, 1.6 Hz, 2H) | 430.9 |
| **58** | 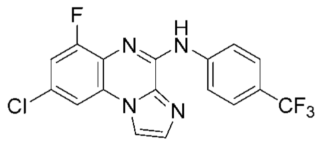 | 10.42 (s, 1H), 8.80 (d, *J* = 0.9 Hz, 1H), 8.47 (d, *J* = 8.6 Hz, 2H), 8.30 (s, 1H), 7.81 (d, *J* = 1.0 Hz, 1H), 7.73 (d, *J* = 8.7 Hz, 2H), 7.58 (dd, *J* = 10.3, 1.9 Hz, 1H) | 380.9 |
| **59** | 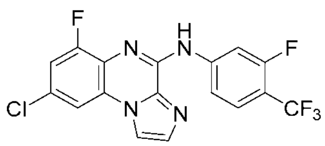 | 10.71 (s, 1H), 8.81 (s, 1H), 8.59 (d,*J* = 14.5 Hz, 1H), 8.32 (s, 1H), 8.17 (d, *J* = 8.5 Hz, 1H), 7.82 (s, 1H), 7.75 (t, *J* = *8.7* Hz, 1H), 7.61 (d, *J* = 10.2 Hz, 1H) | 398.9 |
| **60** | 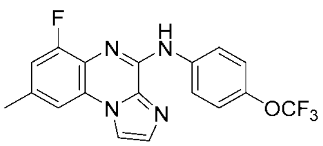 | 9.42 (s, 1H), 7.80 (d, *J* = 0.9 Hz, 1H), 7.67 (m, 2H), 7.30 (s, 1H), 7.11 (d, *J* = 1.0 Hz, 1H), 7.03 (d, *J* = 8.7 Hz, 2H), 6.88 (m, 1H), 2.22 (s, 3H) | 377.1 |

(continued)

| Compound | Structure | $^1H$ NMR (400 MHz, DMSO-$d_6$) δ | ESI-MS: $[M+H]^+$ |
|---|---|---|---|
| **61** | 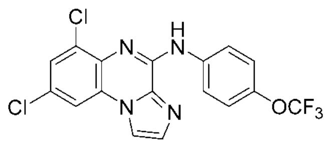 | 10.37 (s, 1H), 8.80 (d, *J* = 1.0 Hz, 1H), 8.51 (d, *J* = 9.2 Hz, 2H), 8.44 (d, *J* = *2.2* Hz, 1H), 7.79 (d, *J* = 2.7 Hz, 2H), 7.39 (d, *J* = 8.7 Hz, 2H) | 412.9 |
| **62** | 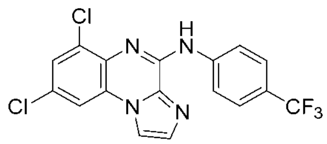 | 10.54 (s, 1H), 8.84 (s, 1H), 8.62 (d, *J* = 8.6 Hz, 2H), 8.47 (d, *J* = 2.1 Hz, 1H), 7.83 (s, 2H), 7.75 (d, *J* = 8.7 Hz, 2H) | 396.9 |
| **63** | 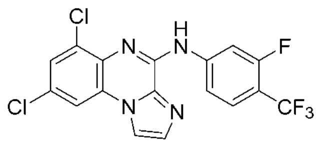 | 10.71 (s, 1H), 8.79 (d, *J* = 1.0 Hz, 1H), 8.75 (d, *J* = 14.9 Hz, 1H), 8.41 (d, *J* = 2.2 Hz, 1H), 8.20 (d, *J* = 8.7 Hz, 1H), 7.79 (d, *J* = 1.0 Hz, 1H), 7.76 (d, *J* = 2.2 Hz, 1H), 7.72 (t, *J* = 8.7 Hz, 1H) | 414.9 |
| **64** | 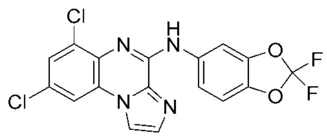 | 10.22 (s, 1H), 8.69 (m, 1H), 8.39 (d, *J* = 2.0 Hz, 1H), 8. 28 (m, 1H), 7.89 (m, 1H), 7.65 *(d, J* = 1.1 Hz, 1H), 7.47 (dd, *J* = 10.3, 2.1 Hz, 1H), 7.33 (d, *J* = 8.8 Hz, 1H) | 408.9 |
| **65** | 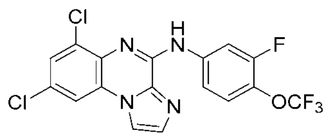 | 10.23 (s, 1H), 8.52 (s, 1H), 8.36 (d, *J* = 13.1 Hz, 1H), 8.05 (s, 1H), 7.86 (d, *J* = 8.8 Hz, 1H), 7.52 (s, 1H), 7.31 (m, 2H) | 430.9 |
| **66** | 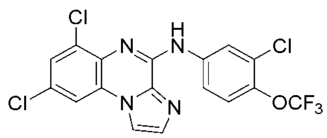 | 10.61 (s, 1H), 9.10 (d, *J* = *2.5* Hz, 1H), 8.83 (s, 1H), 8.47 (d, *J* = 1.9 Hz, 1H), 8.29 (dd, *J* = 9.1, 2.5 Hz, 1H), 7.82 (s, 2H), 7.59 (d, *J* = 9.1 Hz, 1H) | 446.9 |
| **67** | 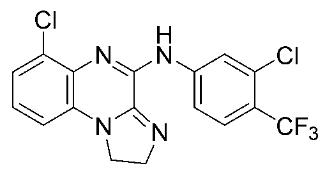 | 10.07 (s, 1H), 9.02 (s, 1H), 8.26 (d, *J* = 8.6 Hz, 1H), 7.80 (d, *J* = 8.9 Hz, 1H), 7.26-7.13 (m, 2H), 6.86 (dd, *J* = 7.6, 1.1 Hz, 1H), 4.15 (s, 4H) | 399.0 |
| **68** | 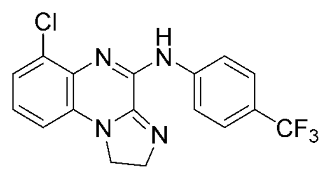 | 9.82 (s, 1H), 8.52 (d, *J* = 8.6 Hz, 2H), 7.71 (d, *J* = 8.6 Hz, 2H), 7.22-7.13 (m, 2H), 6.85 (dd, *J* = 7.3, 1.8 Hz, 1H), 4.16 (s, 4H) | 365.1 |
| **69** | 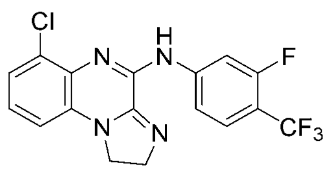 | 10.10 (s, 1H), 8.72 (d, *J* = 14.4 Hz, 1H), 8.15 (d, *J* = 8.3 Hz, 1H), 7.72 (t, *J* = 8.7 Hz, 1H), 7.24-7.15 (m, 2H), 6.86 (dd, *J* = 7.6, 1.3 Hz, 1H), 4.15 (s, 4H) | 382.9 |
| **70** | 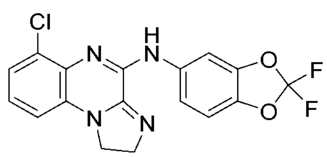 | 9.51 (s, 1H), 8.29 (d, *J* = 2.1 Hz, 1H), 7.86 (dd, *J* = 8.9, 2.2 Hz, 1H), 7.39-7.31 (m, 2H), 7.19 (dd, *J* = 8.0, 1.5 Hz, 1H), 6.96 (t, *J* = 7.9 Hz, 1H), 4.22 (s, 4H) | 377.1 |

(continued)

| Compound | Structure | $^1H$ NMR (400 MHz, DMSO-$d_6$) $\delta$ | ESI-MS: $[M+H]^+$ |
|---|---|---|---|
| **71** | 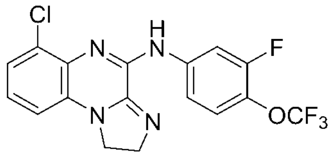 | 9.89 (s, 1H), 8.73 (dd, *J* = 14.1, 2.3 Hz, 1H), 8.05 (d, *J* = 9.1 Hz, 1H), 7.52 (t, *J* = 9.0 Hz, 1H), 7.19 -7.11 (m, 2H), 6.82 (dd, *J* = 7.3, 1.7 Hz, 1H), 4.12 (s, 4H) | 399.1 |
| **72** | 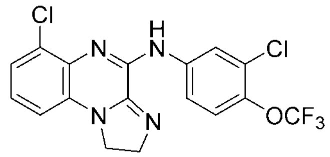 | 9.90 (s, 1H), 9.00 (d, *J* = 2.5 Hz, 1H), 8.17 (dd, *J* = 9.1, 2.5 Hz, 1H), 7.53 (d, *J* = 8.4 Hz, 1H), 7.20-7.12 (m, 2H), 6.83 (dd, *J* = 7.2, 1.9 Hz, 1H), 4.13 (s, 4H) | 414.9 |
| **73** | 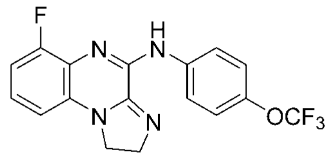 | 9.57 (s, 1H), 8.27 (d, *J* = 9.1 Hz, 2H), 7.36 (d, *J* = 8.7 Hz, 2H), 7.16 (dt, *J* = 13.7, 6.9 Hz, 1H), 6.97-6.84 (m, 1H), 6.69 (d, *J* = 7.7 Hz, 1H), 4.13 (s, 4H) | 365.1 |
| **74** | 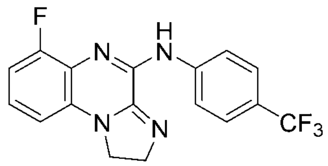 | 9.71 (s, 1H), 8.38 (d, *J* = 8.6 Hz, 2H), 7.70 (d, *J* = 8.7 Hz, 2H), 7.19 (td, *J* = 8.2, 5.6 Hz, 1H), 6.94-6.84 (m, 1H), 6.71 (d, *J* = 8.1 Hz, 1H), 4.23-4.08 (m, 4H) | 349.1 |
| **75** | 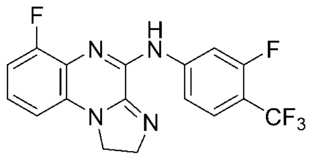 | 10.18 (s, 1H), 8.62 (d, *J* = 14.1 Hz, 1H), 8.29 (d, *J* = 8.8 Hz, 1H), 7.89 (m, 1H), 7.46 (dd, *J* = 7.8, 1.3 Hz, 1H), 7.29 (dd, *J* = 8.0, 1.3 Hz, 1H), 7.18 (m, 1H), 4.46 (s, 4H) | 367.0 |
| **76** | 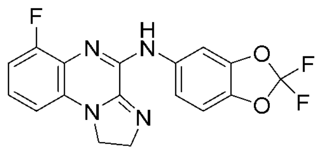 | 10.11 (s, 1H), 8.59 (d, *J* = 2.1 Hz, 1H), 8.06 (m, 1H), 7.69 (m, 2H), 7.31 (m, 1H), 7.16 (t, *J* = 7.9 Hz, 1H), 4.27 (s, 4H) | 360.9 |
| **77** | 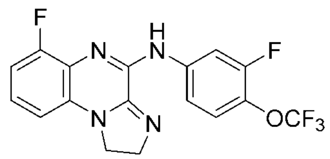 | 10.12 (s, 1H), 8.58 (m, 1H), 8.12 (d, *J* = 9.0 Hz, 1H), 7.63 (t, *J* = 8.7 Hz, 1H), 7.47 (dd, *J* = 7.8, 1.3 Hz, 1H), 7.24 (dd, *J* =8.0, 1.3 Hz, 1H), 7.05 (t, *J* = 7.9 Hz, 1H), 4.19 (s, 4H) | 383.0 |
| **78** | 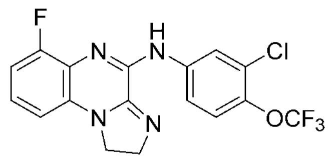 | 10.12 (s, 1H), 8.72 (d, *J* = 2.6 Hz, 1H), 8.50 (dd, *J* = 9.0, 2.6 Hz, 1H), 7.94 (dd, *J* = 9.1, 1.2 Hz, 1H), 7.51 (dd, *J* = 7.9, 1.4 Hz, 1H), 7.32 (dd, *J* = 8.0, 1.4 Hz, 1H), 7.12 (m, 1H), 4.37 (s, 4H) | 399.0 |
| **79** | 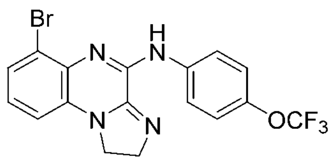 | 9.67 (s, 1H), 8.46 (d, *J* = 9.2 Hz, 2H), 7.35 (d, *J* = 8.7 Hz, 2H), 7.30 (d, *J* = 7.9 Hz, 1H), 7.09 (t, *J* = 8.0 Hz, 1H), 6.87 (d, *J* = 8.0 Hz, 1H), 4.14 (s, 4H) | 424.9 |

(continued)

| Compound | Structure | $^1H$ NMR (400 MHz, DMSO-$d_6$) δ | ESI-MS: $[M+H]^+$ |
|---|---|---|---|
| **80** | 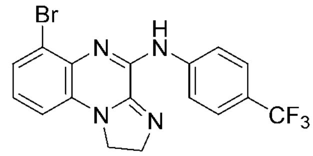 | 9.82 (s, 1H), 8.55 (d, *J* = 8.6 Hz, 2H), 7.70 (d, *J* = 8.7 Hz, 2H), 7.31 (d, *J* = 7.9 Hz, 1H), 7.11 (t, *J* = 8.0 Hz, 1H), 6.88 (d, *J* = 8.0 Hz, 1H), 4.15 (s, 4H) | 409.0 |
| **81** | 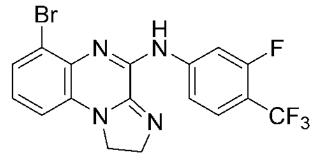 | 10.09 (s, 1H), 8.75 (d, *J* = 14.5 Hz, 1H), 8.17 (d, *J* = 8.5 Hz, 1H), 7.71 (t, *J* = 8.6 Hz, 1H), 7.32 (d, *J* = 7.9 Hz, 1H), 7.14 (t, *J* = 8.0 Hz, 1H), 6.88 (d, *J* = 7.9 Hz, 1H), 4.14 (s, 4H) | 427.0 |
| **82** | 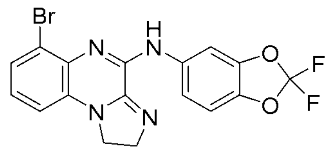 | 9.61 (s, 1H), 8.22 (d, *J* = 2.0 Hz, 1H), 7.81 (dd, *J* = 8.9, 2.1 Hz, 1H), 7.31 (m, 2H), 7.14 (dd, *J* = 8.0, 1.3 Hz, 1H), 6.91 (t, *J* = 7.8 Hz, 1H), 4.17 (s, 4H) | 421.0 |
| **83** | 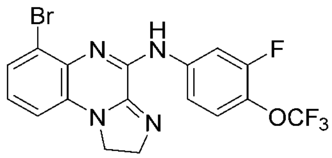 | 9.92 (s, 1H), 8.79 (d, *J* = 12.5 Hz, 1H), 8.09 (d, *J* = 9.1 Hz, 1H), 7.53 (t, *J* = 9.0 Hz, 1H), 7.32 (d, *J* = 8.0 Hz, 1H), 7.12 (t, *J* = 8.0 Hz, 1H), 6.88 (d, *J* = 8.0 Hz, 1H), 4.15 (s, 4H) | 443.0 |
| **84** | 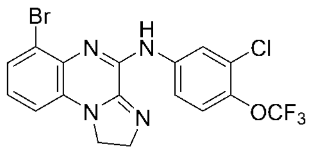 | 9.92 (s, 1H), 9.08 (s, 1H), 8.19 (dd, *J* = 9.1, 2.6 Hz, 1H), 7.54 (d, *J* = 9.0 Hz, 1H), 7.32 (d, *J* = 8.0 Hz, 1H), 7.12 (t, *J* = 8.0 Hz, 1H), 6.88 (d, *J* = 8.0 Hz, 1H), 4.15 (s, 4H) | 458.9 |
| **85** | 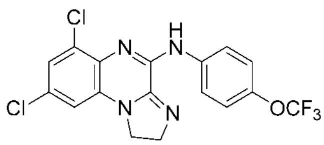 | 9.75 (s, 1H), 8.38 (d, *J* = 9.1 Hz, 2H), 7.35 (d, *J* = 8.9 Hz, 2H), 7.20 (d, *J* = 2.1 Hz, 1H), 6.92 (d, *J* = 2.1 Hz, 1H), 4.12 (s, 4H) | 414.9 |
| **86** | 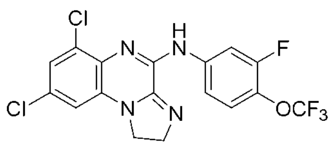 | 10.25 (s, 1H), 8.73 (d, *J* = 13.1 Hz, 1H), 8.44 (d, *J* = 8.8 Hz, 1H), 7.82 (d, *J* = 8.6 Hz, 1H), 7.30 (d, *J* = 2.0 Hz, 1H), 7.12 (d, *J* = 2.1 Hz, 1H), 4.27 (s, 4H) | 432.9 |
| **87** | 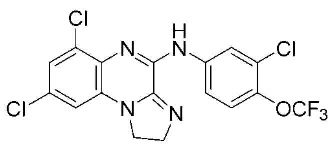 | 9.99 (s, 1H), 8.94 (d, *J* = 2.5 Hz, 1H), 8.17 (dd, *J* = 9.1, 2.6 Hz, 1H), 7.54 (d, *J* = 8.7 Hz, 1H), 7.22 (d, *J* = 2.1 Hz, 1H), 6.94 (d, *J* = 2.1 Hz, 1H), 4.13 (s, 4H) | 448.8 |
| **88** | 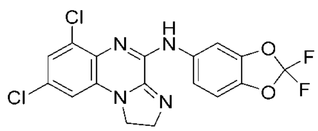 | 10.12 (s, 1H), 8.59 (m, 1H), 8.19 (m, 1H), 8. 02 (m, 1H), 7.47 (d, *J* = 2.1 Hz, 1H), 7.33 (d, *J* = 2.2 Hz, 1H), 4.15 (s, 4H) | 410.8 |
| **89** | 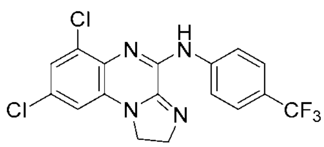 | 9.89 (s, 1H), 8.48 (d, *J* = 8.6 Hz, 2H), 7.70 (d, *J* = 8.6 Hz, 2H), 7.22 (d, *J* = 2.1 Hz, 1H), 6.94 (d, *J* = 2.1 Hz, 1H), 4.13 (s, 4H) | 398.9 |

(continued)

| Compound | Structure | $^1H$ NMR (400 MHz, DMSO-$d_6$) $\delta$ | ESI-MS: $[M+H]^+$ |
|---|---|---|---|
| **90** | 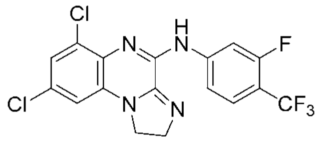 | 10.16 (s, 1H), 8.64 (d, *J* = 14.4 Hz, 1H), 8.13 (d, *J* = 8.8 Hz, 1H), 7.71 (t, *J* = 8.6 Hz, 1H), 7.23 (d, *J* = 2.0 Hz, 1H), 6.95 (d, *J*= *2.0* Hz, 1H), 4.13 (s, 4H) | 416.9 |
| **91** | 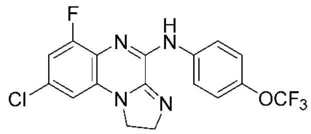 | 9.68 (s, 1H), 8.25 (d, *J* = 9.1 Hz, 2H), 7.37 (d, *J* = 8.5 Hz, 2H), 7.05 (d, *J* = 10.2 Hz, 1H), 6.84 (s, 1H), 4.14 (s, 4H) | 399.0 |
| **92** | 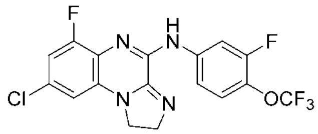 | 9.89 (s, 1H), 8.47 (dd, *J* = 13.7, 2.5 Hz, 1H), 8.00 (d, *J* = 9.1 Hz, 1H), 7.53 (t, *J* = 9.0 Hz, 1H), 7.04 (dd, *J* = 10.2, 2.1 Hz, 1H), 6.82 (s, 1H), 4.12 (s, 4H) | 416.9 |
| **93** | 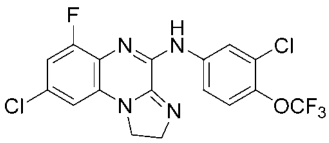 | 9.89 (s, 1H), 8.68 (d, *J* = 2.6 Hz, 1H), 8.15 (dd, *J* = 9.1, 2.6 Hz, 1H), 7.55 (d, *J* = 8.1 Hz, 1H), 7.05 (dd, *J* = 10.2, 2.1 Hz, 1H), 6.84 (s, 1H), 4.13 (s, 4H) | 432.9 |
| **94** | 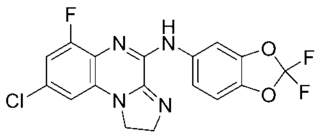 | 10.22 (s, 1H), 8.67 (m, 1H), 8.29 (m, 1H), 8. 11 (m, 1H), 7.55 (m, 1H), 7.43 (d, *J* = 2.2 Hz, 1H), 4.31 (s, 4H) | 394.9 |
| **95** | 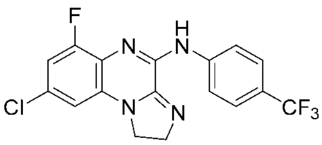 | 9.80 (s, 1H), 8.34 (d, *J* = 8.6 Hz, 2H), 7.70 (d, *J* = 8.7 Hz, 2H), 7.05 (dd, *J* = 10.2, 2.1 Hz, 1H), 6.83 (s, 1H), 4.13 (s, 4H) | 382.9 |
| **96** | 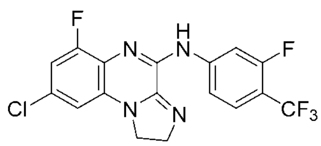 | 10.13 (s, 1H), 8.51 (d, *J* = 14.5 Hz, 1H), 8.14 (d, *J* = 8.9 Hz, 1H), 7.78 (t, *J* = 8.7 Hz, 1H), 7.12 (dd, *J* = 10.2, 2.1 Hz, 1H), 6.90 (s, 1H), 4.19 (s, 4H) | 400.9 |

**Example 95: Synthesis of 6-Chloro-N - (2,2-difluoro-2,3-dihydrobenzofuran-5-yl) imidazolo [1,2-*a*] quinoxalin-4-amine (Compound 39)**

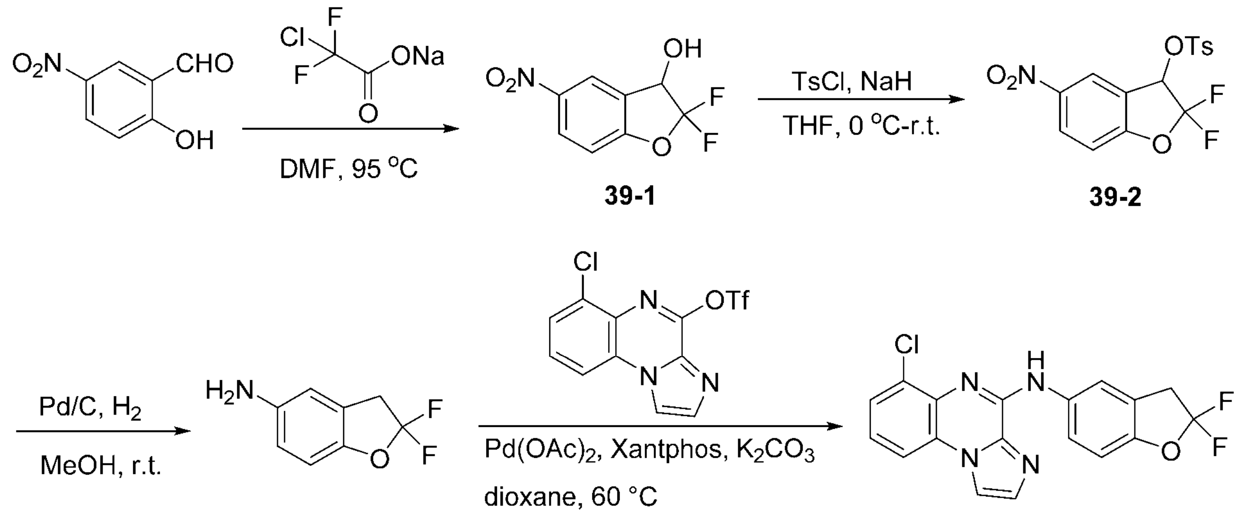


39-1

39-2

39-3

39

**Step 1: Synthesis of Intermediate 39-1**

To a reaction flask was added DMF (30 mL). Under argon atmosphere, a solution of 2-hydroxy-5-nitrobenzaldehyde (500 mg, 2.99 mmol) and sodium difluoro(chloro) acetate (2.28 g, 14.96 mmol) in DMF (10 mL) was added dropwise at 95 °C with stirring. The mixture was stirred at 95 °C for 30 minutes, and the reaction was monitored by **LC-MS** until complete consumption of the starting material. The mixture was poured into water (50 mL) and with EA (30 mL*3) and dried over anhydrous $Na_2SO_4$. After filtration, the filtrate was concentrated under reduced pressure. The residue was purified by silica gel column chromatography (PE/EA = 30/1 to 10/1) to afford a yellow solid **intermediate 39-1** (409 mg, yield: 63%), ESI-MS m/z: 217.9 $[M+H]^+$.

**Step 2: Synthesis of Intermediate 39-2**

To a solution of **39-1** (409 mg, 1.88 mmol) in THF (30 mL) was added NaH (765 mg, 19.12 mmol) slowly at 0 °C under argon atmosphere The resulting mixture was stirred for 30 min at 0 °C. Then TsCl (430 mg, 2.25 mmol) was added dropwise. The reaction was stirred at room temperature until the starting material is consumed. The reaction was then quenched with water (50 mL) and extracted with EA (30 mL*3) and dried over anhydrous $Na_2SO_4$. After filtration, the filtrate was concentrated under reduced pressure. The residue was purified by silica gel column chromatography (PE/EA = 30/1 to 10/1) to afford a yellow solid **intermediate 39-2** (520 mg, yield: 74%), ESI-MS m/z: 372.1 $[M+H]^+$.

**Step 3: Synthesis of Intermediate 39-3**

To a solution of **39-2** (520 mg, 1.4 mmol) in THF (30 mL) was added Pd/C (50 mg, 10%). The reaction was stirred at room temperature under a hydrogen atmosphere until the starting material is consumed. The reaction was filtrated and then the filtrate was concentrated under reduced pressure. The residue was purified by silica gel column chromatography (PE/EA = 10/1 to 1/1) to afford a white solid **intermediate 39-3** (195 mg, yield: 60%), ESI-MS m/z: 172.2 $[M+H]^+$.

**Step 4:Synthesis of Compound 39**

**Compound 39** can be synthesized by a method similar to Example **1,** using **intermediate 3** and **intermediate 39-3** as materials.

$^1H$ NMR (400 MHz, DMSO-$d_6$) δ: 10.13 (s, 1H), 8.71 (d, *J* = 2.2 Hz, 1H), 8.64 (d, *J* = 1.1 Hz, 1H), 8.15 (m, 1H), 8.01 (m, 1H), 7.57 (d, *J* = 1.1 Hz, 1H), 7.55 (m, 1H), 7.31 (m, 2H), 3.63 (t, *J* = 13.2 Hz, 2H); ESI-MS m/z: 373.1 $[M+H]^+$.

**Example 96: Synthesis of 6-Chloro-N - (2,2,3,3-tetrafluoro-2,3-dihydrobenzofuran -5-yl) imidazolo [1,2-*a*] quinoxalin-4-amine (Compound 40)**

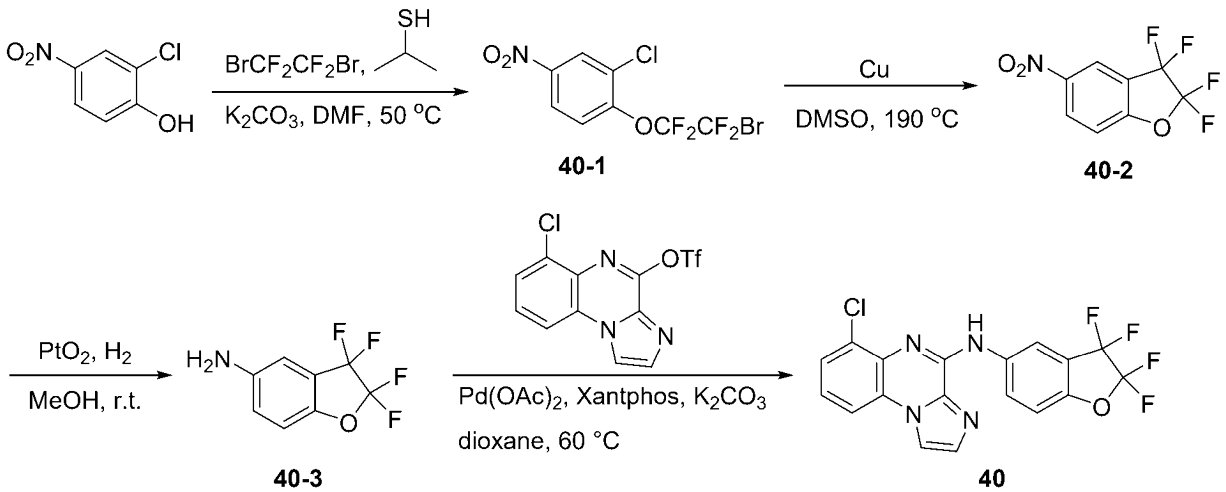


**Step 1: Synthesis of Intermediate 40-1**

To a solution of 2-chloro-4-nitrophenol (500 mg, 2.88 mmol) in DMF (30 mL) was added 2-propanethiol (110 mg, 1.44 mmol), 1,2-dibromotetrafluoroethane (1.12 g, 4.32 mmol) and $K_2CO_3$ (476 mg, 3.45 mmol), and the mixture was heated at 50 °C for 48 hours. The reaction was monitored by LC-MS until complete consumption of the starting material. The mixture was poured into 2 N NaOH aqueous solution (50 mL) and extracted with EA (30 mL*3) and dried over anhydrous $Na_2SO_4$. After filtration, the filtrate was concentrated under reduced pressure. The residue was purified by silica gel column chromatography (PE/EA = 100/1 to 20/1) to afford yellow oil **intermediate 40-1** (426 mg, yield: 42%), ESI-MS m/z: 351.8 $[M+H]^+$.

**Step 2: Synthesis of Intermediate 40-2**

To a solution of **40-1** (426 mg, 1.21 mmol) in DMSO (30 mL) was added Copper powder (387 mg, 6.05 mmol), and the mixture was heated at 190 °C in a sealed tube for 6 hours. The reaction was monitored by LC-MS until complete consumption of the starting material. The mixture was poured into water and extracted with EA (30 mL*3) and dried over anhydrous $Na_2SO_4$. After filtration, the filtrate was concentrated under reduced pressure. The residue was purified by silica gel column chromatography (PE/EA = 50/1 to 10/1) to afford a yellow solid **intermediate 40-2** (236 mg, yield: 82%), ESI-MS m/z: 237.9 $[M+H]^+$.

**Step 3: Synthesis of Intermediate 40-3**

To a solution of **40-2** (236 mg, 1 mmol) in MeOH (30 mL) was added $PtO_2$ (20 mg). The reaction was stirred at room temperature under a hydrogen atmosphere until the starting material is consumed. The reaction was filtrated and then the filtrate was concentrated under reduced pressure to afford colorless oil **intermediate 40-3** (220 mg, crude), ESI-MS m/z: 207.9 $[M+H]^+$.

**Step 4: Synthesis of Compound 40**

**Compound 40** can be synthesized by a method similar to **Example 1,** using **intermediate 3** and **intermediate 40-3** as materials.

$^1H$ NMR (400 MHz, DMSO-$d_6$) δ: 10.23 (s, 1H), 8.75 (m, 2H), 8.35 (m, 1H), 8.21 (m, 1H), 7.77 (d, $J$ = 1.2 Hz, 1H), 7.56 (m, 1H), 7.35 (m, 2H); ESI-MS m/z: 408.9 $[M+H]^+$.

**Examples 97-249: Synthesis of Compounds B1-B153**

**Compounds B1-B153** can be synthesized by a method similar to **Example 1,** using **Intermediates 10-54, 65-74** and corresponding amines as starting materials.

| Compound | Structure | Name | ESI-MS $[M+H]^+$ |
|---|---|---|---|
| **B1** | 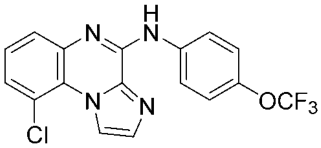 | 9-chloro-*N* - (4- (trifluoromethoxy) phenyl) imidazolo [1,2-*a*] quinoxalin-4-amine | 378.9 |
| **B2** | 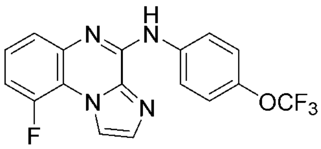 | 9-fluoro-*N* - (4- (trifluoromethoxy) phenyl) imidazolo [1,2-*a*] quinoxalin-4-amine | 362.9 |
| **B3** | 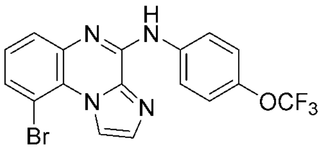 | 9-bromo-*N* - (4- (trifluoromethoxy) phenyl) imidazolo [1,2-*a*] quinoxalin-4-amine | 422.8 |
| **B4** | 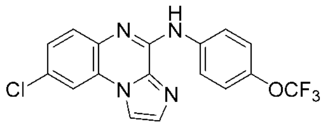 | 8-chloro-*N* - (4- (trifluoromethoxy) phenyl) imidazolo [1,2-*a*] quinoxalin-4-amine | 378.9 |
| **B5** | 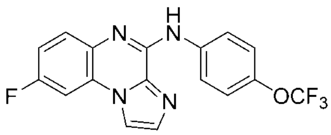 | 8-fluoro-*N* - (4- (trifluoromethoxy) phenyl) imidazolo [1,2-*a*] quinoxalin-4-amine | 362.9 |
| **B6** | 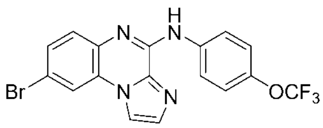 | 8-bromo-*N* - (4- (trifluoromethoxy) phenyl) imidazolo [1,2-*a*] quinoxalin-4-amine | 422.8 |
| **B7** | 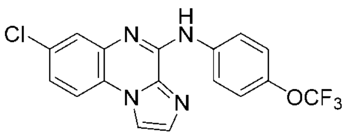 | 7-chloro-*N* - (4- (trifluoromethoxy) phenyl) imidazolo [1,2-*a*] quinoxalin-4-amine | 378.9 |
| **B8** | 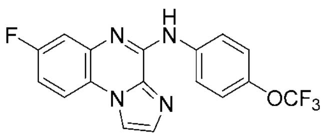 | 7-fluoro-*N*-(4-(trifluoromethoxy) phenyl) imidazolo [1,2-*a*] quinoxalin-4-amine | 362.9 |
| **B9** | 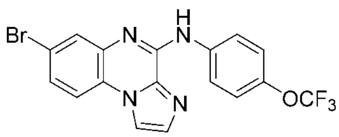 | 7-bromo-*N* - (4- (trifluoromethoxy) phenyl) imidazolo [1,2-*a*] quinoxalin-4-amine | 422.8 |
| **B10** | 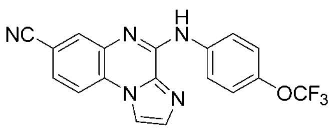 | 7-cyano-*N* - (4- (trifluoromethoxy) phenyl) imidazolo [1,2-*a*] quinoxalin-4-amine | 369.9 |
| **B11** | 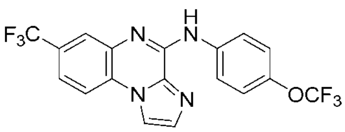 | 7-trifluoromethyl-*N* - (4-(trifluoromethoxy) phenyl) imidazolo [1,2-*a*] quinoxalin-4-amine | 412.9 |

(continued)

| Compound | Structure | Name | ESI-MS $[M+H]^+$ |
|---|---|---|---|
| **B12** | 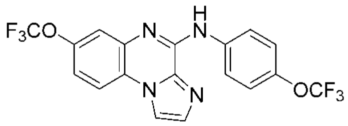 | 7-trifluoromethoxy-*N* - (4-(trifluoromethoxy) phenyl) imidazolo [1,2-*a*] quinoxalin-4-amine | 428.9 |
| **B13** | 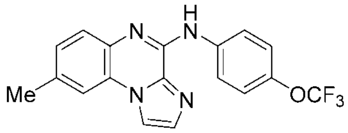 | 8-methyl-*N* - (4- (trifluoromethoxy) phenyl) imidazolo [1,2-*a*] quinoxalin-4-amine | 358.9 |
| **B14** | 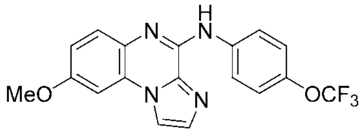 | 8-methoxy-*N* - (4- (trifluoromethoxy) phenyl) imidazolo [1,2-*a*] quinoxalin-4-amine | 374.9 |
| **B15** | 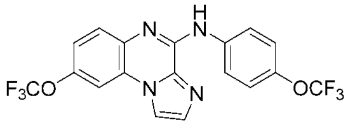 | 8-trifluoromethoxy-*N* - (4-(trifluoromethoxy) phenyl) imidazolo [1,2-*a*] quinoxalin-4-amine | 428.9 |
| **B16** | 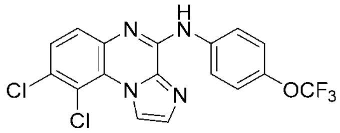 | 8,9-dichloro-*N* - (4- (trifluoromethoxy) phenyl) imidazolo [1,2-*a*] quinoxalin-4-amine | 412.8 |
| **B17** | 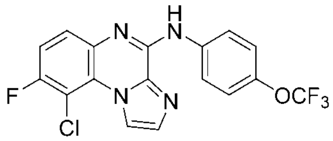 | 9-chloro-8-fluoro-*N* - (4-(trifluoromethoxy) phenyl) imidazolo [1,2-*a*] quinoxalin-4-amine | 396.8 |
| **B18** | 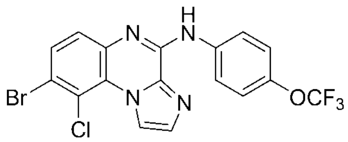 | 8-bromo-9-chloro-*N* - (4-(trifluoromethoxy) phenyl) imidazolo [1,2-*a*] quinoxalin-4-amine | 456.8 |
| **B19** | 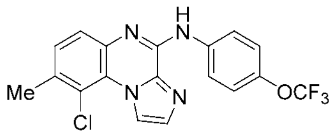 | 9-chloro-8-methyl-*N* - (4-(trifluoromethoxy) phenyl) imidazolo [1,2-*a*] quinoxalin-4-amine | 392.9 |
| **B20** | 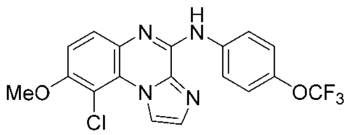 | 9-chloro-8-methoxy-*N* - (4-(trifluoromethoxy) phenyl) imidazolo [1,2-*a*] quinoxalin-4-amine | 408.9 |
| **B21** | 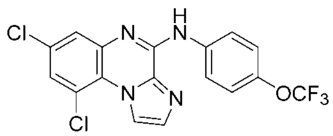 | 7,9-dichloro-*N* - (4- (trifluoromethoxy) phenyl) imidazolo [1,2-*a*] quinoxalin-4-amine | 412.8 |
| **B22** | 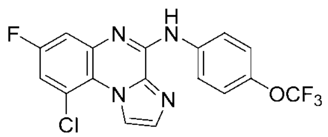 | 9-chloro-7-fluoro-*N* - (4-(trifluoromethoxy) phenyl) imidazolo [1,2-*a*] quinoxalin-4-amine | 396.8 |

(continued)

| Compound | Structure | Name | ESI-MS [M+H]+ |
|---|---|---|---|
| **B23** | 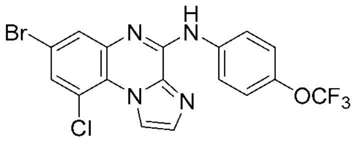 | 7-bromo-9-Chloro-*N* - (4-(trifluoromethoxy) phenyl) imidazolo [1,2-*a*] quinoxalin-4-amine | 456.8 |
| **B24** | 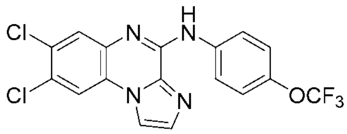 | 7,8-dichloro-*N* - (4- (trifluoromethoxy) phenyl) imidazolo [1,2-*a*] quinoxalin-4-amine | 412.8 |
| **B25** | 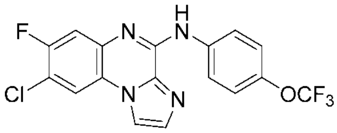 | 8-chloro-7-fluoro-*N* - (4-(trifluoromethoxy) phenyl) imidazolo [1,2-*a*] quinoxalin-4-amine | 396.8 |
| **B26** | 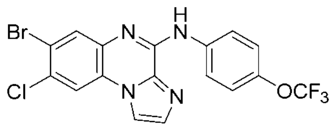 | 7-bromo-8-chloro-*N* - (4-(trifluoromethoxy) phenyl) imidazolo [1,2-*a*] quinoxalin-4-amine | 456.8 |
| **B27** | 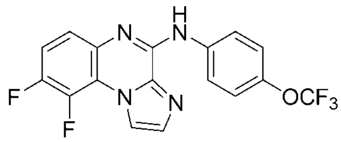 | 8,9-difluoro-*N* - (4- (trifluoromethoxy) phenyl) imidazolo [1,2-*a*] quinoxalin-4-amine | 380.9 |
| **B28** | 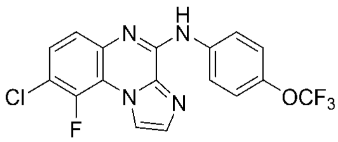 | 8-chloro-9-fluoro-*N* - (4-(trifluoromethoxy) phenyl) imidazolo [1,2-*a*] quinoxalin-4-amine | 396.8 |
| **B29** | 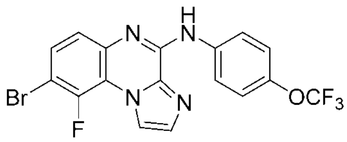 | 8-bromo-9-fluoro-*N* - (4-(trifluoromethoxy) phenyl) imidazolo [1,2-*a*] quinoxalin-4-amine | 440.8 |
| **B30** | 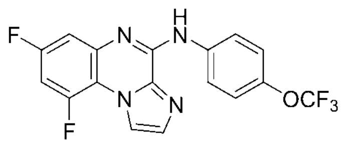 | 7,9-difluoro-*N* - (4- (trifluoromethoxy) phenyl) imidazolo [1,2-*a*] quinoxalin-4-amine | 380.9 |
| **B31** | 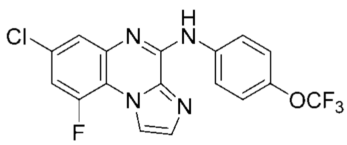 | 7-chloro-9-fluoro-*N* - (4-(trifluoromethoxy) phenyl) imidazolo [1,2-*a*] quinoxalin-4-amine | 396.8 |
| **B32** | 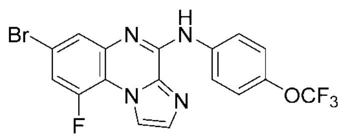 | 7-bromo-9-fluoro-*N* - (4-(trifluoromethoxy) phenyl) imidazolo [1,2-*a*] quinoxalin-4-amine | 440.8 |

(continued)

| Compound | Structure | Name | ESI-MS [M+H]+ |
|---|---|---|---|
| **B33** | 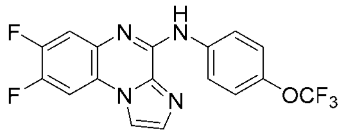 | 7,8-difluoro-*N* - (4- (trifluoromethoxy) phenyl) imidazolo [1,2-*a*] quinoxalin-4-amine | 380.9 |
| **B34** | 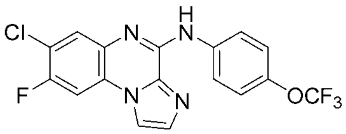 | 7-chloro-8-fluoro-*N* - (4-(trifluoromethoxy) phenyl) imidazolo [1,2-*a*] quinoxalin-4-amine | 396.8 |
| **B35** | 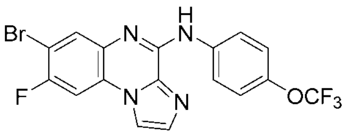 | 7-bromo-8-fluoro-*N* - (4-(trifluoromethoxy) phenyl) imidazolo [1,2-*a*] quinoxalin-4-amine | 440.8 |
| **B36** | 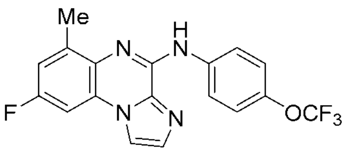 | 8-fluoro-6-methyl-*N* - (4-(trifluoromethoxy) phenyl) imidazolo [1,2-*a*] quinoxalin-4-amine | 376.9 |
| **B37** | 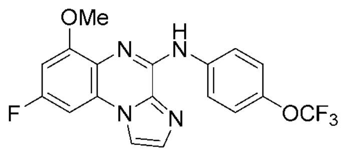 | 8-fluoro-6-methoxy-*N* - (4-(trifluoromethoxy) phenyl) imidazolo [1,2-*a*] quinoxalin-4-amine | 392.9 |
| **B38** | 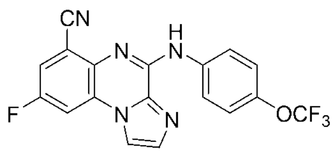 | 8-fluoro-6-Cyano-*N* - (4-(trifluoromethoxy) phenyl) imidazolo [1,2-*a*] quinoxalin-4-amine | 387.9 |
| **B39** | 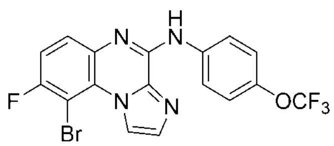 | 9-bromo-8-fluoro-*N* - (4-(trifluoromethoxy) phenyl) imidazolo [1,2-*a*] quinoxalin-4-amine | 440.8 |
| **B40** | 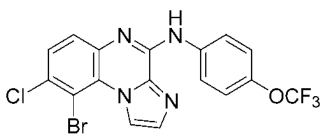 | 9-bromo-8-chloro-*N* - (4-(trifluoromethoxy) phenyl) imidazolo [1,2-*a*] quinoxalin-4-amine | 456.8 |
| **B41** | 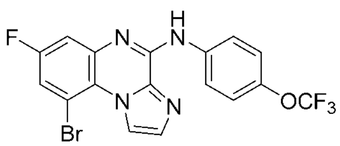 | 9-bromo-7-fluoro-*N* - (4-(trifluoromethoxy) phenyl) imidazolo [1,2-*a*] quinoxalin-4-amine | 440.8 |
| **B42** | 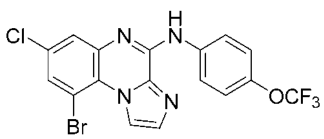 | 9-bromo-7-chloro-*N* - (4-(trifluoromethoxy) phenyl) imidazolo [1,2-*a*] quinoxalin-4-amine | 456.8 |

(continued)

| Compound | Structure | Name | ESI-MS $[M+H]^+$ |
|---|---|---|---|
| **B43** | 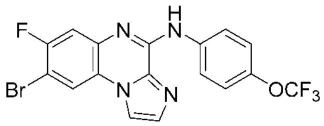 | 8-bromo-7-fluoro-*N* - (4-(trifluoromethoxy) phenyl) imidazolo [1,2-*a*] quinoxalin-4-amine | 440.8 |
| **B44** | 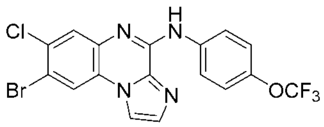 | 8-bromo-7-chloro-*N* - (4-(trifluoromethoxy) phenyl) imidazolo [1,2-*a*] quinoxalin-4-amine | 456.8 |
| **B45** | 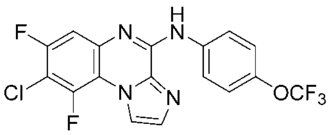 | 8-chloro-7,9-difluoro-*N* - (4-(trifluoromethoxy) phenyl) imidazolo [1,2-*a*] quinoxalin-4-amine | 414.8 |
| **B46** | 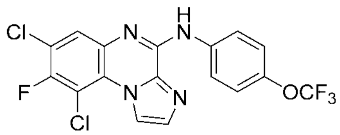 | 7,9-dichloro-8-fluoro-*N* - (4-(trifluoromethoxy) phenyl) imidazolo [1,2-*a*] quinoxalin-4-amine | 430.8 |
| **B47** | 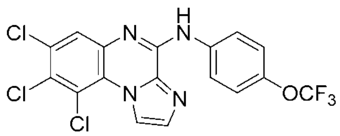 | 7,8,9-trichloro-*N* - (4- (trifluoromethoxy) phenyl) imidazolo [1,2-*a*] quinoxalin-4-amine | 446.8 |
| **B48** | 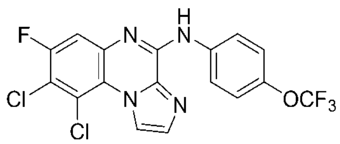 | 8,9-dichloro-7-fluoro-*N* - (4-(trifluoromethoxy) phenyl) imidazolo [1,2-*a*] quinoxalin-4-amine | 430.8 |
| **B49** | 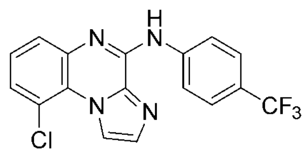 | 9-chloro-*N* - (4- (trifluoromethyl) phenyl) imidazolo [1,2-*a*] quinoxalin-4-amine | 362.9 |
| **B50** | 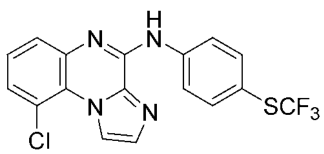 | 9-chloro-*N* - (4- (trifluoromethylthio) phenyl) imidazolo [1,2-*a*] quinoxalin-4-amine | 394.8 |
| **B51** | 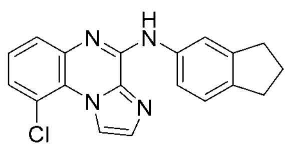 | 9-chloro-*N* - (2,3-dihydro-1H-inden-5-yl) imidazolo [1,2-*a*] quinoxalin-4-amine | 334.9 |
| **B52** | 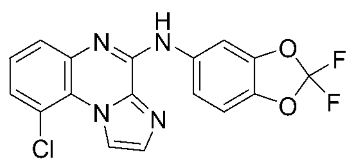 | 9-chloro-*N* - (2,2-difluorobenzo [*d*] [1,3] dihydroxy-5-yl) imidazolo [1,2-*a*] quinoxalin-4-amine | 374.8 |

(continued)

| Compound | Structure | Name | ESI-MS $[M+H]^+$ |
|---|---|---|---|
| **B53** | 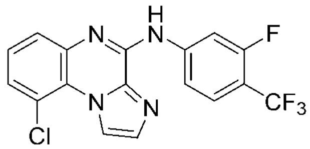 | 9-chloro-*N* - (3-Fluoro-4-(trifluoromethyl) phenyl) imidazolo [1,2-*a*] quinoxalin-4-amine | 380.9 |
| **B54** | 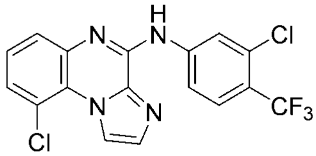 | 9-chloro-*N* - (3-chloro-4-(trifluoromethyl) phenyl) imidazolo [1,2-*a*] quinoxalin-4-amine | 396.8 |
| **B55** | 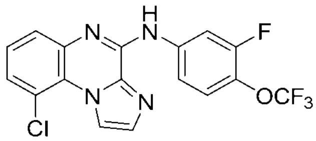 | 9-chloro-*N* - (3-Fluoro-4-(trifluoromethoxy) phenyl) imidazolo [1,2-*a*] quinoxalin-4-amine | 396.8 |
| **B56** | 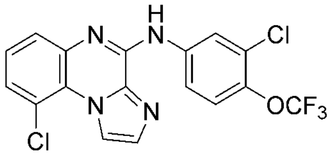 | 9-chloro-*N* - (3-chloro-4-(trifluoromethoxy) phenyl) imidazolo [1,2-*a*] quinoxalin-4-amine | 412.8 |
| **B57** | 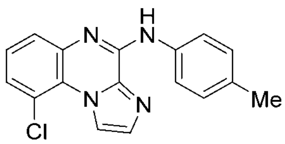 | 9-chloro-*N* - (p-toluene) imidazolo [1,2-*a*] quinoxaline-4-amine | 308.9 |
| **B58** | 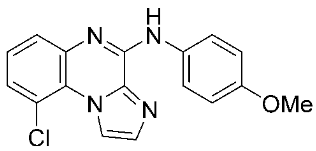 | 9-chloro-*N* - (4- (methoxy) phenyl) imidazolo [1,2-*a*] quinoxalin-4-amine | 324.9 |
| **B59** | 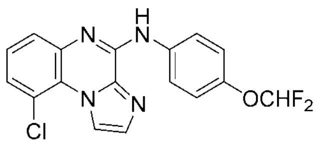 | 9-chloro-*N* - (4- (difluoromethoxy) phenyl) imidazolo [1,2-*a*] quinoxalin-4-amine | 360.9 |
| **B60** | 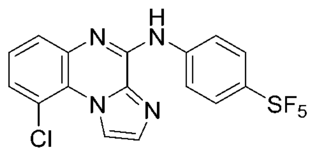 | 9-chloro-*N* - (4- (thiopentafluoride) phenyl) imidazolo [1,2-*a*] quinoxalin-4-amine | 420.8 |
| **B61** | 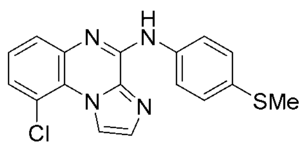 | 9-chloro-*N* - (4- (methylthio) phenyl) imidazolo [1,2-*a*] quinoxalin-4-amine | 340.9 |
| **B62** | 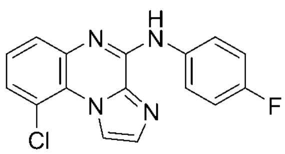 | 9-chloro-*N* - (p-fluorophenyl) imidazolo [1,2-*a*] quinoxalin-4-amine | 312.9 |

(continued)

| Compound | Structure | Name | ESI-MS [M+H]+ |
|---|---|---|---|
| **B63** | 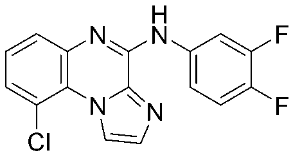 | 9-chloro-*N* - (3,4-difluorophenyl) imidazolo [1,2-*a*] quinoxalin-4-amine | 330.9 |
| **B64** | 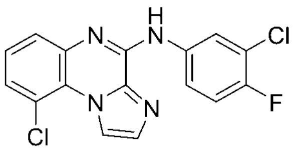 | 9-chloro-*N* - (3-chloro-4-fluorophenyl) imidazolo [1,2-*a*] quinoxalin-4-amine | 346.8 |
| **B65** | 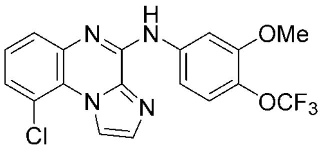 | 9-chloro-*N* - (3-methoxy-4-(trifluoromethoxy) phenyl) imidazolo [1,2-*a*] quinoxalin-4-amine | 408.9 |
| **B66** | 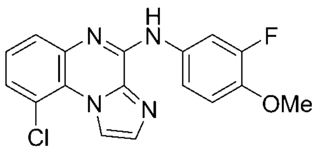 | 9-chloro-*N* - (3-fluoro-4-methoxyphenyl) Imidazolo [1,2-*a*] quinoline-4-amine | 342.9 |
| **B67** | 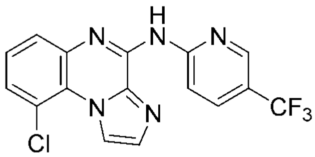 | 9-chloro-*N* - (5- (trifluoromethyl) pyridin-2-yl) imidazolo [1,2-*a*] quinoxalin-4-amine | 363.9 |
| **B68** | 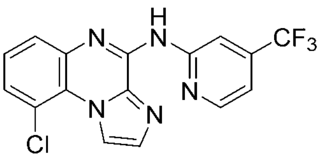 | 9-chloro-*N* - (4- (trifluoromethyl) pyridin-2-yl) imidazolo [1,2-*a*] quinoxalin-4-amine | 363.9 |
| **B69** | 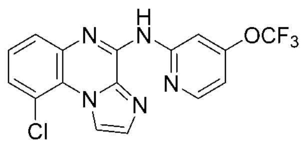 | 9-chloro-*N* - (4- (trifluoromethoxy) pyridin-2-yl) imidazolo [1,2-*a*] quinoxalin-4-amine | 379.8 |
| **B70** | 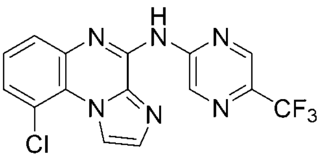 | 9-chloro-*N* - (5- (trifluoromethyl) pyrazin-2-yl) imidazolo [1,2-*a*] quinoxalin-4-amine | 364.9 |
| **B71** | 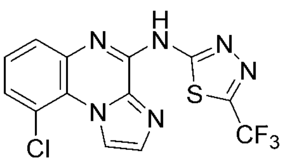 | *N* - (9-chloroimidazo [1,2-*a*] quinoxalin-4-yl) -5-(trifluoromethyl) -1,3,4-thiadiazole-2-amine | 370.8 |

(continued)

| Compound | Structure | Name | ESI-MS $[M+H]^+$ |
|---|---|---|---|
| **B72** | 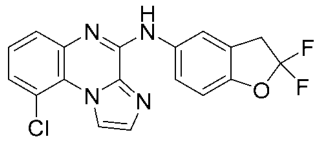 | 9-chloro-*N* - (2,2-difluoro-2,3-dihydrobenzofuran-5-yl ) imidazolo [1,2-*a*] quinoxalin-4-amine | 373.1 |
| **B73** | 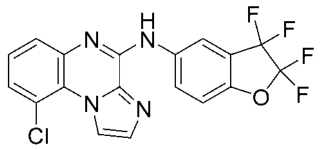 | 9-chloro-*N* - (2,2,3,3-tetrafluoro-2,3-dihydrobenzofura n-5-yl) imidazolo [1,2-*a*] quinoxalin-4-amine | 409.0 |
| **B74** | 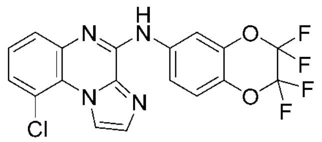 | 9-chloro-*N* - (2,2,3,3-tetrafluoro-2,3-dihydrobenzo *[b]* [1,4] dioxolan-6-yl) imidazolo [1,2-*a*] quinoxalin-4-amine | 424.8 |
| **B75** | 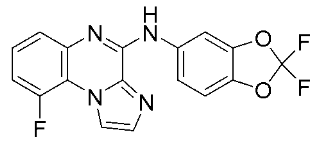 | 9-fluoro-*N* - (2,2-difluorobenzo [d] [1,3] dihydroxy-5-yl) imidazolo [1,2-*a*] quinoxalin-4-amine | 358.9 |
| **B76** | 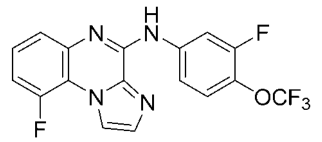 | 9-fluoro-*N* - (3-fluoro-4-(trifluoromethoxy) phenyl) imidazolo [1,2-*a*] quinoxalin-4-amine | 380.9 |
| **B77** | 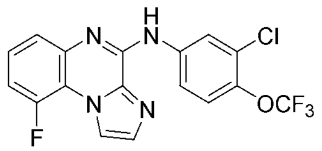 | 9-fluoro-*N* - (3-chloro-4-(trifluoromethoxy) phenyl) imidazolo [1,2-*a*] quinoxalin-4-amine | 396.8 |
| **B78** | 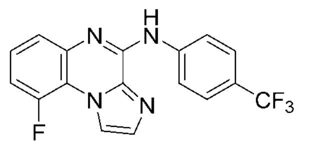 | 9-fluoro-*N* - (4- (trifluoromethyl) phenyl) imidazolo [1,2-*a*] quinoxalin-4-amine | 346.9 |
| **B79** | 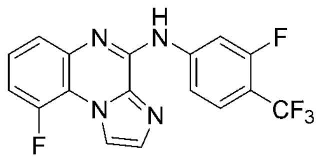 | 9-fluoro-*N* - (3-fluoro-4-(trifluoromethyl) phenyl) imidazolo [1,2-*a*] quinoxalin-4-amine | 364.9 |
| **B80** | 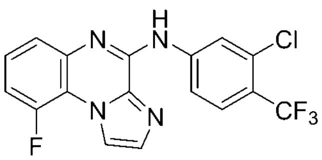 | 9-fluoro-*N* - (3-chloro-4-(trifluoromethyl) phenyl) imidazolo [1,2-*a*] quinoxalin-4-amine | 380.9 |
| **B81** | 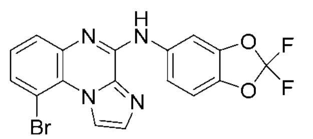 | 9-bromo-*N* - (2,2-difluorobenzo [*d*] [1,3] dihydroxy-5-yl) imidazolo [1,2-*a*] quinoxalin-4-amine | 418.8 |

(continued)

| Compound | Structure | Name | ESI-MS $[M+H]^+$ |
|---|---|---|---|
| **B82** | 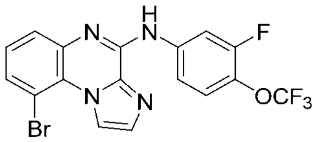 | 9-bromo-*N* - (3-fluoro-4-(trifluoromethoxy) phenyl) imidazolo [1,2-*a*] quinoxalin-4-amine | 440.8 |
| **B83** | 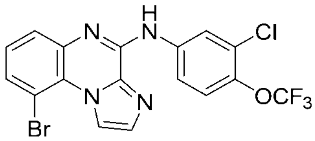 | 9-bromo-*N* - (3-chloro-4-(trifluoromethoxy) phenyl) imidazolo [1,2-*a*] quinoxalin-4-amine | 456.8 |
| **B84** | 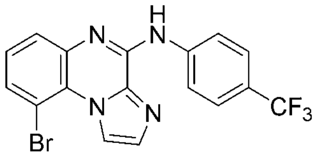 | 9-bromo-*N* - (4- (trifluoromethyl) phenyl) imidazolo [1,2-*a*] quinoxalin-4-amine | 406.8 |
| **B85** | 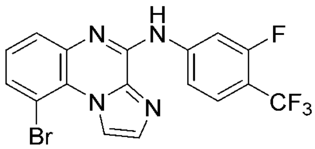 | 9-bromo-*N* - (3-fluoro-4-(trifluoromethyl) phenyl) imidazolo [1,2-*a*] quinoxalin-4-amine | 424.8 |
| **B86** | 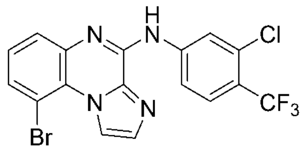 | 9-bromo-*N* - (3-chloro-4-(trifluoromethyl) phenyl) imidazolo [1,2-*a*] quinoxalin-4-amine | 440.8 |
| **B87** | 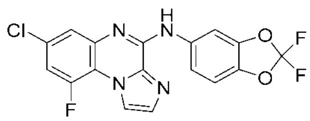 | 7-chloro-9-fluoro-*N* - (2,2-difluorobenzo [*d*] [1,3] dihydroxy-5-yl) imidazolo [1,2-*a*] quinoxalin-4-amine | 392.8 |
| **B88** | 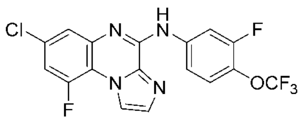 | 7-chloro-9-fluoro-*N* - (3-fluoro-4-(trifluoromethoxy) phenyl) imidazolo [1,2-*a*] quinoxalin-4-amine | 414.8 |
| **B89** | 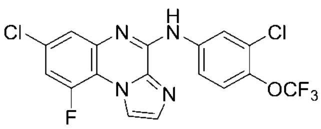 | 7-chloro-9-fluoro-*N* - (3-chloro-4-(trifluoromethoxy) phenyl) imidazolo [1,2-*a*] quinoxalin-4-amine | 430.8 |
| **B90** | 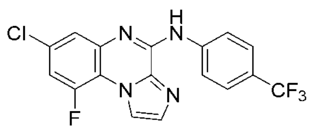 | 7-chloro-9-fluoro-*N* - (4-(trifluoromethyl) phenyl) imidazolo [1,2-*a*] quinoxalin-4-amine | 380.9 |
| **B91** | 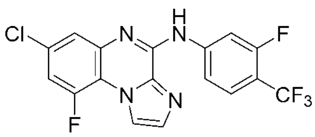 | 7-chloro-9-fluoro-*N* - (3-fluoro-4-(trifluoromethyl) phenyl) imidazolo [1,2-*a*] quinoxalin-4-amine | 398.8 |

(continued)

| Compound | Structure | Name | ESI-MS $[M+H]^+$ |
|---|---|---|---|
| **B92** | 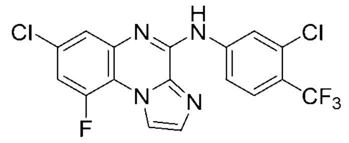 | 7-chloro-9-fluoro-*N* - (3-chloro-4-(trifluoromethyl) phenyl) imidazolo [1,2-*a*] quinoxalin-4-amine | 414.8 |
| **B93** | 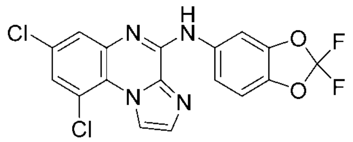 | 7,9-dichloro-*N* - (2,2-difluorobenzo [*d*] [1,3] dihydroxy-5-yl) imidazolo [1,2-*a*] quinoxalin-4-amine | 408.8 |
| **B94** | 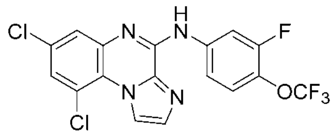 | 7,9-dichloro-*N* - (3-fluoro-4-(trifluoromethoxy) phenyl) imidazolo [1,2-*a*] quinoxalin-4-amine | 430.8 |
| **B95** | 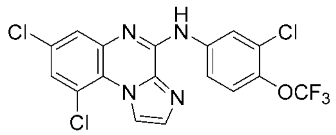 | 7,9-dichloro-*N* - (3-chloro-4-(trifluoromethoxy) phenyl) imidazolo [1,2-*a*] quinoxalin-4-amine | 446.8 |
| **B96** | 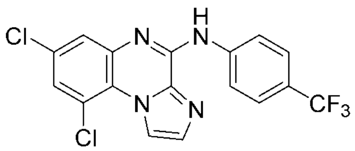 | 7,9-dichloro-*N* - (4- (trifluoromethyl) phenyl) imidazolo [1,2-*a*] quinoxalin-4-amine | 396.8 |
| **B97** | 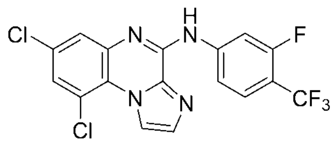 | 7,9-dichloro-*N* - (3-fluoro-4-(trifluoromethyl) phenyl) imidazolo [1,2-*a*] quinoxalin-4-amine | 414.8 |
| **B98** | 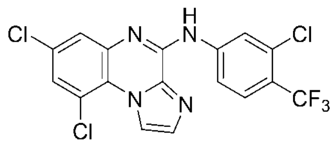 | 7,9-dichloro-*N* - (3-chloro-4-(trifluoromethyl) phenyl) imidazolo [1,2-*a*] quinoxalin-4-amine | 430.8 |
| **B99** | 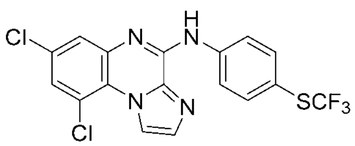 | 7,9-dichloro-*N* - (4- (trifluoromethylthio) phenyl) imidazolo [1,2-*a*] quinoxalin-4-amine | 428.8 |
| **B100** | 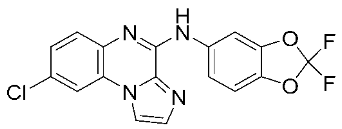 | 8-chloro-*N* - (2,2-difluorobenzo [*d*] [1,3] dihydroxy-5-yl) imidazolo [1,2-*a*] quinoxalin-4-amine | 374.8 |
| **B101** | 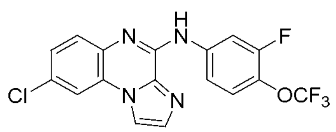 | 8-chloro-*N* - (3-fluoro-4-(trifluoromethoxy) phenyl) imidazolo [1,2-*a*] quinoxalin-4-amine | 396.8 |

(continued)

| Compound | Structure | Name | ESI-MS $[M+H]^+$ |
|---|---|---|---|
| **B102** | 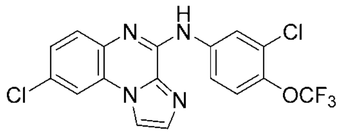 | 8-chloro-*N* - (3-chloro-4-(trifluoromethoxy) phenyl) imidazolo [1,2-*a*] quinoxalin-4-amine | 412.8 |
| **B103** | 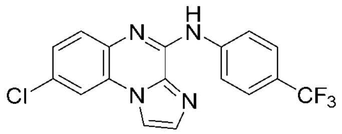 | 8-chloro-*N* - (4- (trifluoromethyl) phenyl) imidazolo [1,2-*a*] quinoxalin-4-amine | 362.9 |
| **B104** | 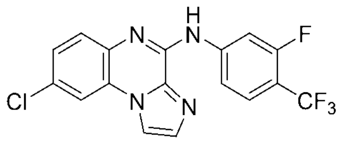 | 8-chloro-*N* - (3-fluoro-4-(trifluoromethyl) phenyl) imidazolo [1,2-*a*] quinoxalin-4-amine | 380.9 |
| **B105** | 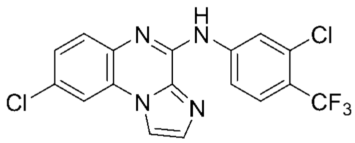 | 8-chloro-*N* - (3-chloro-4-(trifluoromethyl) phenyl) imidazolo [1,2-*a*] quinoxalin-4-amine | 396.8 |
| **B106** | 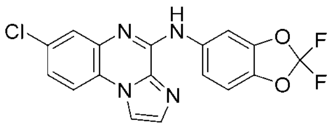 | 7-chloro-*N* - (2,2-difluorobenzo [*d*] [1,3] dihydroxy-5-yl) imidazolo [1,2-*a*] quinoxalin-4-amine | 374.8 |
| **B107** | 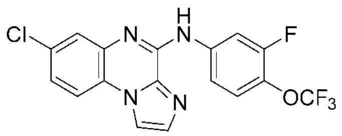 | 7-chloro-*N* - (3-fluoro-4-(trifluoromethoxy) phenyl) imidazolo [1,2-*a*] quinoxalin-4-amine | 396.8 |
| **B108** | 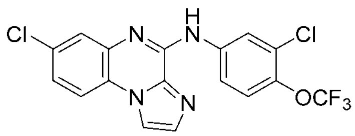 | 7-chloro-*N* - (3-chloro-4-(trifluoromethoxy) phenyl) imidazolo [1,2-*a*] quinoxalin-4-amine | 412.8 |
| **B109** | 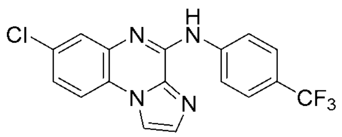 | 7-chloro-*N* - (4- (trifluoromethyl) phenyl) imidazolo [1,2-*a*] quinoxalin-4-amine | 362.9 |
| **B110** | 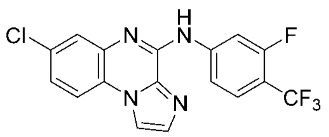 | 7-chloro-N - (3-fluoro-4-(trifluoromethyl) phenyl) imidazolo [1,2-*a*] quinoxalin-4-amine | 380.9 |
| **B111** | 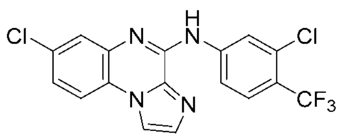 | 7-chloro-*N* - (3-chloro-4-(trifluoromethyl) phenyl) imidazolo [1,2-*a*] quinoxalin-4-amine | 396.8 |

(continued)

| Compound | Structure | Name | ESI-MS [M+H]+ |
|---|---|---|---|
| **B112** | 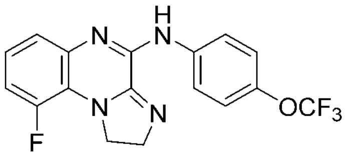 | 9-fluoro-*N* - (4- (trifluoromethoxy) phenyl) -1,2-dihydroimidazo [1,2-*a*] quinoxalin-4-amine | 364.9 |
| **B113** | 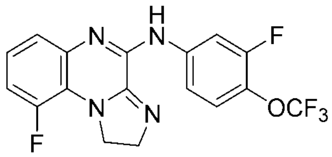 | 9-fluoro-*N* - (3-fluoro-4-(trifluoromethoxy) phenyl) -1,2-dihydroimidazo [1,2-*a*] quinoxalin-4-amine | 382.9 |
| **B114** | 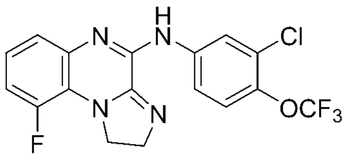 | N - (3-chloro-4- (trifluoromethoxy) phenyl) -9-fluoro-1,2-dihydroimidazo [1,2-*a*] quinoxalin-4-amine | 398.9 |
| **B115** | 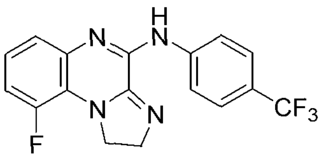 | 9-fluoro-*N* - (4- (trifluoromethyl) phenyl) -1,2-dihydroimidazo [1,2-*a*] quinoxalin-4-amine | 348.9 |
| **B116** | 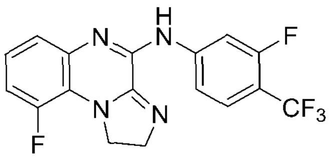 | 9-fluoro-*N* - (3-fluoro-4-(trifluoromethyl) phenyl) -1,2-dihydroimidazo [1,2-*a*] quinoxalin-4-amine | 366.9 |
| **B117** | 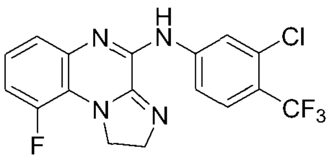 | N - (3-chloro-4- (trifluoromethyl) phenyl) -9-fluoro-1,2-dihydroimidazo [1,2-*a*] quinoxalin-4-amine | 382.9 |
| **B118** | 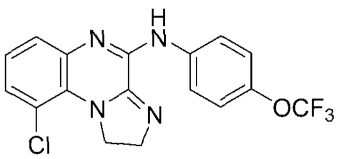 | 9-chloro-*N* - (4- (trifluoromethoxy) phenyl) -1,2-dihydroimidazo [1,2-*a*] quinoxalin-4-amine | 380.9 |
| **B119** | 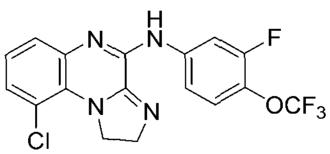 | 9-chloro-*N* - (3-fluoro-4-(trifluoromethoxy) phenyl) -1,2-dihydroimidazo [1,2-*a*] quinoxalin-4-amine | 398.9 |
| **B120** | 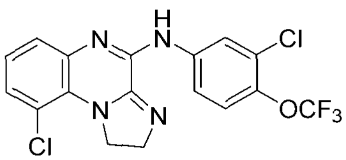 | 9-chloro-*N* - (3-chloro-4-(trifluoromethoxy) phenyl) -1,2-dihydroimidazo [1,2-*a*] quinoxalin-4-amine | 414.8 |

(continued)

| Compound | Structure | Name | ESI-MS $[M+H]^+$ |
|---|---|---|---|
| **B121** | 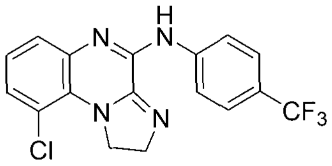 | 9-chloro-*N* - (4- (trifluoromethyl) phenyl) -1,2-dihydroimidazo [1,2-*a*] quinoxalin-4-amine | 364.9 |
| **B122** | 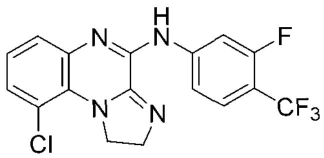 | 9-chloro-*N* - (3-fluoro-4-(trifluoromethyl) phenyl) -1,2-dihydroimidazo [1,2-*a*] quinoxalin-4-amine | 382.9 |
| **B123** | 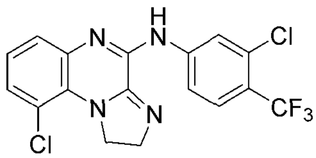 | 9-chloro-*N* - (3-chloro-4-(trifluoromethyl) phenyl) -1,2-dihydroimidazo [1,2-*a*] quinoxalin-4-amine | 398.8 |
| **B124** | 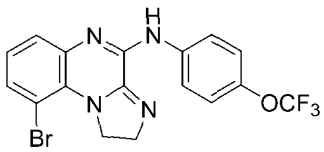 | 9-bromo-*N* - (4- (trifluoromethoxy) phenyl) -1,2-dihydroimidazo [1,2-*a*] quinoxalin-4-amine | 424.8 |
| **B125** | 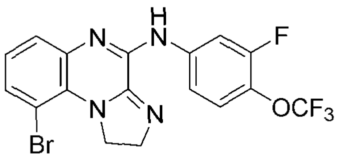 | 9-bromo-*N* - (3-fluoro-4-(trifluoromethoxy) phenyl) -1,2-dihydroimidazo [1,2-a] quinoxalin-4-amine | 442.8 |
| **B126** | 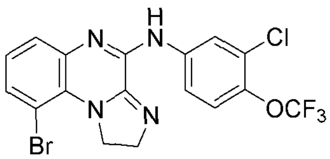 | 9-bromo-*N* - (3-chloro-4-(trifluoromethoxy) phenyl) -1,2-dihydroimidazo [1,2-*a*] quinoxalin-4-amine | 458.8 |
| **B127** | 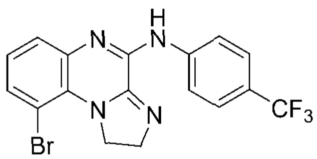 | 9-bromo-*N* - (4- (trifluoromethyl) phenyl) -1,2-dihydroimidazo [1,2-*a*] quinoxalin-4-amine | 408.8 |
| **B128** | 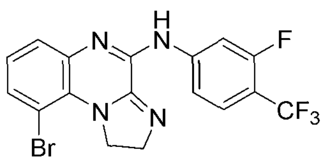 | 9-bromo-*N* - (3-fluoro-4-(trifluoromethyl) phenyl) -1,2-dihydroimidazo [1,2-*a*] quinoxalin-4-amine | 426.8 |
| **B129** | 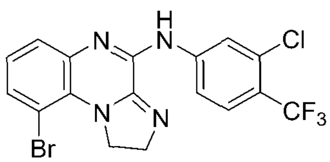 | 9-bromo-*N* - (3-chloro-4-(trifluoromethyl) phenyl) -1,2-dihydroimidazo [1,2-*a*] quinoxalin-4-amine | 442.8 |

(continued)

| Compound | Structure | Name | ESI-MS [M+H]+ |
|---|---|---|---|
| **B130** | 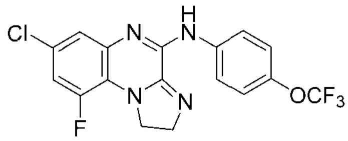 | 7-chloro-9-fluoro-*N* - (4-(trifluoromethoxy) phenyl) -1,2-dihydroimidazo [1,2-*a*] quinoxalin-4-amine | 398.9 |
| **B131** | 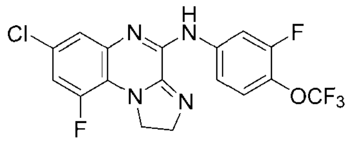 | 7-chloro-9-fluoro-*N* - (3-fluoro-4-(trifluoromethoxy) phenyl) -1,2-dihydroimidazo [1,2-*a*] quinoxalin-4-amine | 416.9 |
| **B132** | 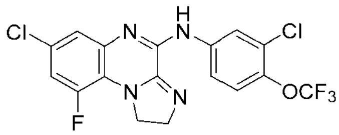 | 7-chloro-*N* - (3-chloro-4-(trifluoromethoxy) phenyl) -9-fluoro-1,2-dihydroimidazo [1,2-*a*] quinoxalin-4-amine | 432.8 |
| **B133** | 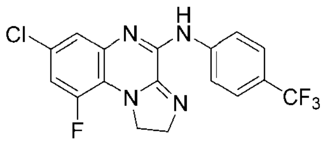 | 7-chloro-9-fluoro-*N* - (4-(trifluoromethyl) phenyl) -1,2-dihydroimidazo [1,2-*a*] quinoxalin-4-amine | 382.9 |
| **B134** | 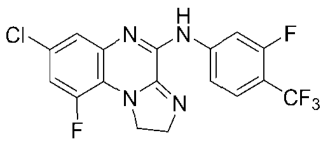 | 7-chloro-9-fluoro-*N* - (3-fluoro-4-(trifluoromethyl) phenyl) -1,2-dihydroimidazo [1,2-*a*] quinoxalin-4-amine | 400.9 |
| **B135** | 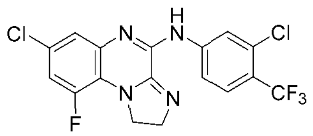 | 7-chloro-*N* - (3-chloro-4-(trifluoromethyl) phenyl) -9-fluoro-1,2-dihydroimidazo [1,2-*a*] quinoxalin-4-amine | 416.8 |
| **B136** | 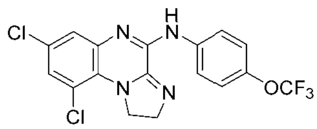 | 7,9-dichloro-*N* - (4- (trifluoromethoxy) phenyl) -1,2-dihydroimidazo [1,2-*a*] quinoxalin-4-amine | 414.8 |
| **B137** | 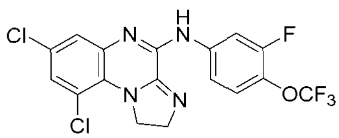 | 7,9-dichloro-*N* - (3-fluoro-4-(trifluoromethoxy) phenyl) -1,2-dihydroimidazo [1,2-*a*] quinoxalin-4-amine | 432.8 |
| **B138** | 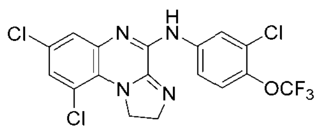 | 7,9-dichloro-*N* - (3-chloro-4-(trifluoromethoxy) phenyl) -1,2-dihydroimidazo [1,2-*a*] quinoxalin-4-amine | 448.8 |
| **B139** | 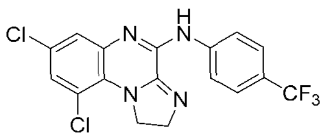 | 7,9-dichloro-*N* - (4- (trifluoromethyl) phenyl) -1,2-dihydroimidazo [1,2-*a*] quinoxalin-4-amine | 398.8 |

(continued)

| Compound | Structure | Name | ESI-MS [M+H]+ |
|---|---|---|---|
| **B140** | 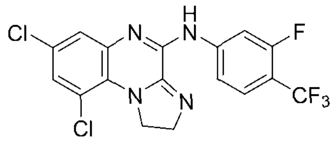 | 7,9-dichloro-*N* - (3-fluoro-4-(trifluoromethyl) phenyl) -1,2-dihydroimidazo [1,2-*a*] quinoxalin-4-amine | 416.8 |
| **B141** | 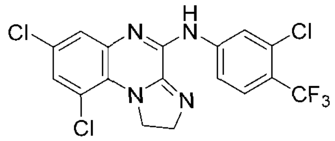 | 7,9-dichloro-*N* - (3-chloro-4-(trifluoromethyl) phenyl) -1,2-dihydroimidazo [1,2-*a*] quinoxalin-4-amine | 432.8 |
| **B142** | 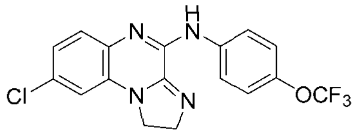 | 8-chloro-*N* - (4- (trifluoromethoxy) phenyl) -1,2-dihydroimidazo [1,2-*a*] quinoxalin-4-amine | 380.9 |
| **B143** | 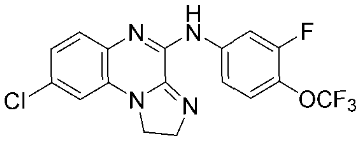 | 8-chloro-*N* - (3-fluoro-4-(trifluoromethoxy) phenyl)-1,2-dihydroimidazo [1,2-*a*] quinoxalin-4-amine | 398.9 |
| **B144** | 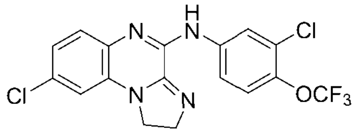 | 8-chloro-*N* - (3-chloro-4-(trifluoromethoxy) phenyl) -1,2-dihydroimidazo [1,2-*a*] quinoxalin-4-amine | 414.8 |
| **B145** | 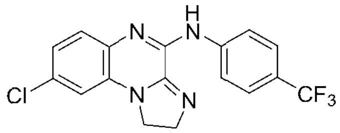 | 8-chloro-*N* - (4- (trifluoromethyl) phenyl) -1,2-dihydroimidazo [1,2-*a*] quinoxalin-4-amine | 364.9 |
| **B146** | 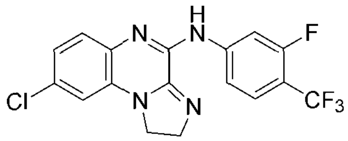 | 8-chloro-*N* - (3-Fluoro-4-(trifluoromethyl) phenyl) -1,2-dihydroimidazo [1,2-*a*] quinoxalin-4-amine | 382.9 |
| **B147** | 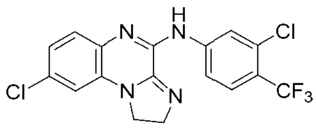 | 8-chloro-*N* - (3-chloro-4-(trifluoromethyl) phenyl) -1,2-dihydroimidazo [1,2-*a*] quinoxalin-4-amine | 398.8 |
| **B148** | 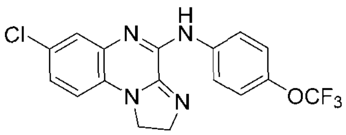 | 7-chloro-*N* - (4- (trifluoromethoxy) phenyl) -1,2-dihydroimidazo [1,2-*a*] quinoxalin-4-amine | 380.9 |
| **B149** | 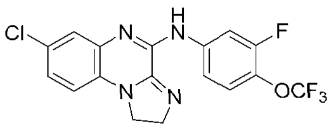 | 7-chloro-*N* - (3-fluoro-4-(trifluoromethoxy) phenyl) -1,2-dihydroimidazo [1,2-*a*] quinoxalin-4-amine | 398.9 |

(continued)

| Compound | Structure | Name | ESI-MS $[M+H]^+$ |
|---|---|---|---|
| **B150** | 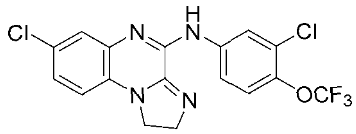 | 7-chloro-*N* - (3-chloro-4-(trifluoromethoxy) phenyl) -1,2-dihydroimidazo [1,2-*a*] quinoxalin-4-amine | 414.8 |
| **B151** | 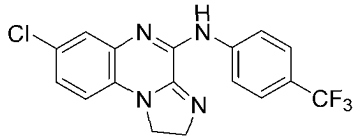 | 7-chloro-*N* - (4- (trifluoromethyl) phenyl) -1,2-dihydroimidazo [1,2-*a*] quinoxalin-4-amine | 364.9 |
| **B152** | 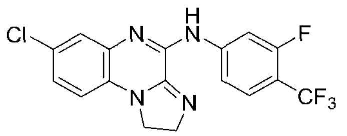 | 7-chloro-*N* - (3-fluoro-4-(trifluoromethyl) phenyl) -1,2-dihydroimidazo [1,2-*a*] quinoxalin-4-amine | 382.9 |
| **B153** | 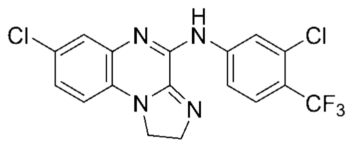 | 7-chloro-*N* - (3-chloro-4-(trifluoromethyl) phenyl) -1,2-dihydroimidazo [1,2-*a*] quinoxalin-4-amine | 398.8 |

**Example 250: Induction of miR-124 Expression in PBMC Cells**

This study evaluated the ability of the compound of the present invention to influence miR-124 transcriptional activity *in vitro.* The experiment analyzed miR-124 expression in human PBMCs (Sailybio) using quantitative PCR (qPCR).

Thawed human PBMCs (1 mL) were added to 19 mL of culture medium (RPMI1640 + 10% hFBS + 1‰ 2-mercaptoethanol) and the PBMCs were incubated in a cell culture incubator for 30 minutes. The PBMCs were collected and the cells ($3.0*10^6$ cells/well/3 mL) were resuspended in medium containing PMA (10 ng/mL; Invivogen) and ionomycin (200 ng/mL; Invivogen). The cells were incubated in a 6-well plate for 48 hours. Diluted compound was added to the cells to achieve a final concentration of 0.1 μM. After 72 hours, the samples were centrifuged and the cells were collected.

Total miRNA was extracted from the cells using the MiPure Cell/Tissue miRNA Kit (Vazyme) according to the manufacturer's instructions, followed by purification. The purified samples were treated with genomic DNA elimination buffer, and reverse transcription was performed using a GeneAmp PCR System. The resulting cDNA was subjected to quantitative real-time PCR (qPCR) analysis on a QuantStudio™ 7 Flex Real-Time PCR System. Data analysis was performed using the QuantStudio 7 software with default settings to automatically determine Ct values, and the results were exported. In this assay, miR-191 was used as the reference gene, and the DMSO-treated group served as the control. The relative expression of miR-124 was calculated using the following formula:

$$\Delta Ct = Ct\ (target\ gene) - Ct\ (reference\ gene)$$

$$\Delta\Delta Ct = \Delta Ct\ (treated\ group) - \Delta Ct\ (control\ group)$$

$$Relative\ mRNA\ expression = 2^{\wedge}(-\Delta\Delta Ct)$$

Table 1. Effects of Test Compounds 0.1 μM) on miR-124 Expression in PBMCs

| Compound | Fold change *vs* DMSO | Compound | Fold change *vs* DMSO | Compound | Fold change *vs* DMSO |
|---|---|---|---|---|---|
| **1** | 16.04 | **41** | 24.38 | **67** | 28.83 |
| **2** | 16.48 | **42** | 14.07 | **68** | 29.53 |
| **6** | 11.93 | **43** | 18.94 | **69** | 22.87 |
| **9** | 26.88 | **44** | 17.49 | **70** | 16.44 |
| **10** | 15.39 | **45** | 17.85 | **71** | 25.33 |
| **11** | 18.42 | **46** | 9.99 | **72** | 18.96 |
| **12** | 21.98 | **47** | 9.86 | **73** | 8.59 |
| **13** | 20.90 | **48** | 20.43 | **74** | 31.61 |
| **14** | 24.72 | **49** | 19.53 | **79** | 17.82 |
| **15** | 12.66 | **50** | 18.49 | **80** | 19.78 |
| **16** | 13.37 | **51** | 23.12 | **81** | 15.27 |
| **17** | 18.99 | **52** | 22.29 | **83** | 18.33 |
| **18** | 17.60 | **54** | 14.87 | **84** | 15.54 |
| **19** | 21.21 | **55** | 22.29 | **85** | 10.43 |
| **21** | 15.54 | **56** | 17.16 | **87** | 15.81 |
| **22** | 15.27 | **58** | 27.29 | **89** | 24.32 |
| **28** | 10.40 | **59** | 20.51 | **91** | 27.56 |
| **29** | 9.32 | **61** | 12.13 | **92** | 18.78 |
| **34** | 14.73 | **62** | 15.52 | **95** | 26.99 |
| **39** | 16.75 | **63** | 18.44 | **96** | 17.70 |
| **40** | 17.47 | **66** | 22.69 | | |
| **B1** | 43.82 | **B67** | 23.41 | **B110** | 25.53 |
| **B2** | 37.35 | **B75** | 29.53 | **B111** | 22.49 |
| **B3** | 23.46 | **B76** | 18.49 | **B118** | 28.81 |
| **B4** | 33.15 | **B77** | 23.12 | **B119** | 25.37 |
| **B5** | 31.28 | **B78** | 14.87 | **B120** | 22.64 |
| **B6** | 21.97 | **B79** | 22.29 | **B121** | 29.53 |
| **B7** | 32.03 | **B80** | 17.16 | **B122** | 27.33 |
| **B8** | 33.48 | **B87** | 15.84 | **B123** | 24.26 |
| **B9** | 22.62 | **B88** | 20.06 | **B130** | 25.57 |
| **B16** | 33.39 | **B89** | 22.31 | **B131** | 27.01 |
| **B17** | 31.93 | **B90** | 24.90 | **B132** | 27.59 |
| **B18** | 22.65 | **B91** | 25.71 | **B133** | 26.32 |
| **B21** | 35.25 | **B92** | 27.08 | **B134** | 27.54 |
| **B22** | 32.54 | **B93** | 19.08 | **B135** | 24.93 |
| **B23** | 25.21 | **B94** | 26.35 | **B136** | 34.11 |
| **B24** | 26.68 | **B95** | 24.77 | **B137** | 31.13 |
| **B25** | 23.43 | **B96** | 23.21 | **B138** | 29.94 |

(continued)

| Compoun d | Fold change *vs* DMSO | Compo und | Fold change *vs* DMSO | Compou nd | Fold change *vs* DMSO |
|---|---|---|---|---|---|
| **B30** | 22.49 | **B97** | 26.20 | **B139** | 40.58 |
| **B31** | 24.42 | **B98** | 22.91 | **B140** | 38.37 |
| **B32** | 19.73 | **B99** | 17.63 | **B141** | 36.20 |
| **B49** | 32.05 | **B100** | 19.57 | **B142** | 25.59 |
| **B50** | 21.34 | **B101** | 24.74 | **B143** | 28.36 |
| **B52** | 30.23 | **B102** | 27.33 | **B144** | 27.75 |
| **B53** | 36.78 | **B103** | 25.52 | **B145** | 30.02 |
| **B54** | 34.07 | **B104** | 28.13 | **B146** | 28.73 |
| **B55** | 36.33 | **B105** | 27.78 | **B147** | 27.91 |
| **B56** | 40.72 | **B106** | 16.33 | **B148** | 29.35 |
| **B59** | 25.39 | **B107** | 24.41 | **B149** | 34.69 |
| **B60** | 24.78 | **B108** | 22.47 | **B150** | 26.37 |
| **B61** | 19.86 | **B109** | 26.38 | **ABX464** | 13.51/16.17 |
| Note: The test results for compound **ABX464** represent data obtained from two independent experiments. | | | | | |

As shown in the table above, compared with the positive control ABX464, the compounds of the present invention demonstrate significantly stronger activity in inducing miR-124 expression in PBMCs.

**Example 251: *In Vivo* Pharmacokinetic Evaluation in Mice**

The compound was administered orally (p.o.) at a dose of 10 mg/kg. The test formulation was prepared at a final concentration of 0.4 mg/mL in 5% DMSO + 45% PEG400 + 50% aqueous solution or suspension. Male ICR mice (9 per group) were sampled at three discontinuous time points (n=3 mice/time point). Approximately 80 μL of blood was collected via orbital or cardiac puncture at 5 min, 15 min, 30 min, 1 h, 2 h, 4 h, 8 h, and 24 h post-dose. All whole blood samples were collected in EDTA $K_2$-containing tubes. After centrifugation at 4°C (4000 rpm, 10 min), plasma was collected and stored below -40°C for subsequent analysis. Compound concentrations in plasma were determined by liquid chromatography-tandem mass spectrometry (LC-MS/MS), and pharmacokinetic parameters were derived from the plasma concentration-time curves.

**Table 2. Pharmacokinetic parameters of compounds 2, 18, 19, 43, 67, 68, 70, 71, 89, B1, B4, B7, B54, B118, B121, B139 and ABX464 in mice**

| Compound | Administration route | Dose (mg/kg) | $t_{1/2}$ (h) | $T_{max}$ (h) | $C_{max}$ (ng/mL) | AUC $_{0-t}$ (ng.h/L) |
|---|---|---|---|---|---|---|
| **2** | po | 10 | 3.45 | 4.0 | 207 | 933 |
| **18** | po | 10 | 3.78 | 2.0 | 365 | 1841 |
| **19** | po | 10 | 3.36 | 4.0 | 358 | 1638 |
| **43** | po | 10 | 3.87 | 2.0 | 348 | 1682 |
| **67** | po | 10 | 3.29 | 4.0 | 403 | 2033 |
| **68** | po | 10 | 2.17 | 0.5 | 243 | 879 |
| **70** | po | 10 | 2.32 | 0.5 | 357 | 1427 |
| **71** | po | 10 | 3.41 | 4.0 | 274 | 1369 |
| **89** | po | 10 | 3.46 | 2.0 | 454 | 1864 |
| **B1** | po | 10 | 2.58 | 1.0 | 450 | 3586 |
| **B4** | po | 10 | 3.26 | 2.0 | 271 | 2849 |

(continued)

| Compound | Administration route | Dose (mg/kg) | $t_{½}$ (h) | $T_{max}$ (h) | $C_{max}$ (ng/mL) | AUC $_{0-t}$ (ng.h/L) |
|---|---|---|---|---|---|---|
| **B7** | po | 10 | 3.05 | 2.0 | 378 | 3235 |
| **B54** | po | 10 | 1.96 | 0.5 | 346 | 1332 |
| **B118** | po | 10 | 2.47 | 2.0 | 269 | 1875 |
| **B121** | po | 10 | 2.12 | 1.0 | 378 | 1948 |
| **B139** | po | 10 | 3.36 | 2.0 | 375 | 3264 |
| **ABX464** | po | 10 | 1.08 | 0.5 | 168 | 420 |

As shown in the above table, the compounds of the present invention exhibit favorable oral absorption characteristics. Key pharmacokinetic parameters including maximum plasma concentration ($C_{max}$), area under the curve ($AUC_{0-t}$), and mean residence time (MRT) were superior to those of the reference compound ABX464. These optimal absorption properties are particularly significant for enhancing drug efficacy, reducing dosage requirements, and lowering treatment costs.

Further experimental studies confirmed that other inventive compounds also demonstrate excellent oral bioavailability, with their $C_{max}$, $AUC_{0-1}$, and MRT values consistently outperforming ABX464.

**Example 252: Preventive and Therapeutic Effects of Compounds on Inflammatory Bowel Disease (IBD) in Mice**

This study evaluated the preventive and therapeutic effects of the compounds of the present invention on dextran sulfate sodium (DSS)-induced IBD in mice. The experiment utilized female C57BL/6 mice (purchased from Beijing Vital River Laboratory Animal Technology Co., Ltd.) to establish the DSS-induced IBD model.

1. Methods and Materials

1.1 Experimental Animals

Female C57BL/6 mice (6-8 weeks old) were purchased from Beijing Vital River Laboratory Animal Technology Co., Ltd. Upon arrival, the mice were housed in individually ventilated cages (IVC) under specific pathogen-free (SPF) conditions, with 2-6 mice per cage. They were provided with ad libitum access to food and water and maintained at a temperature of 20-26°C (68-79°F), relative humidity of 40-70%, and a 12-hour light/dark cycle (lights on from 08:00 to 20:00). After a 7-day acclimatization period, experiments were initiated.

1.2 Reagents and Equipment

Dextran sulfate sodium (DSS, MW = 36,000-50,000): MP Biomedicals, Cat. No. 160110, Lot No. S3045;
Dimethyl sulfoxide (DMSO): Sinopharm Chemical Reagent Co., Ltd., Cat. No. 30072418, Lot No. 20230309;
Polyethylene glycol-15 hydroxystearate (Solutol HS-15): Sigma, Cat. No. 42966, Lot No. BCCJ5912;
Zoletil (Tiletamine-Zolazepam): Virbac, Lot No. 8V4RA;
Xylazine: Tokyo Chemical Industry (TCI), Lot No. R834H-MB;
Balance: METTLER TOLEDO, Model AL104;
Centrifuge: Thermo Fisher Scientific, Model 21R;

1.3 Experimental Design and Methods

1.3.1 Drug Preparation

Test compounds and ABX464 vehicle: 5% DMSO + 10% Solutol HS-15 + 85% saline, stored at 4°C.
DSS solution: 2% (w/v) DSS in autoclaved drinking water, prepared fresh before use.

1.3.2 Experimental Groups and Procedures

The mice were randomly allocated into eight groups according to body weight: normal control group (negative control), model group (DSS group), ABX464 treatment group, test compound groups (administered with Compounds 19, 43, 59, 84, and 89 of the present invention at 40 mg/kg, p.o., q.d.)

| Group | Experiment design | N | Dose |
|---|---|---|---|
| Normal control | 10 days normal water | 5 | vehicle |
| Model group | 7 days 2% DSS + 3 days normal water | 5 | vehicle |
| ABX464 | 7 days 2% DSS + 3 days normal water | 5 | 40 mg/kg |
| Compound 19 | 7 days 2% DSS + 3 days normal water | 5 | 40 mg/kg |
| Compound 43 | 7 days 2% DSS + 3 days normal water | 5 | 40 mg/kg |
| Compound 59 | 7 days 2% DSS + 3 days normal water | 5 | 40 mg/kg |
| Compound 84 | 7 days 2% DSS + 3 days normal water | 5 | 40 mg/kg |
| Compound 89 | 7 days 2% DSS + 3 days normal water | 5 | 40 mg/kg |

Following a 7-day acclimation period, the model group and compound treatment groups were administered 2% DSS aqueous solution ad libitum for 7 days starting from Day 0, followed by 3 days of normal water consumption. All groups received daily oral gavage of their respective vehicles or test compounds for 10 consecutive days beginning on the trial commencement date. During the experimental period, body weight changes in mice were monitored and recorded daily, the Disease Activity Index (DAI) was scored each day with the total score calculated on the final day, and on the last experimental day all animals were humanely euthanized followed by colon collection and length measurement.

1.3.3 Statistical Analysis

The experimental data were expressed as mean ± standard error of the mean (mean ± S.E.M.). Statistical analysis was performed using GraphPad Prism or SPSS software with appropriate statistical methods. A value of $P < 0.05$ was considered statistically significant.

2. Results

2.1 Effects of the Compounds of the Present Invention on Body Weight in DSS-Induced IBD Mice

The body weight results are shown in Figure 1. Compared with the normal control group, mice in the model group exhibited significant weight loss starting from day 5, with over 10% reduction by day 10. In contrast to the model group, all compound-treated groups showed weight recovery beginning on day 7. By day 10: Compounds 19, 59 and 84 of the present invention demonstrated weight gain percentages of 0.16%, 2.12% and 0.38%, respectively; Compounds 43 and 89 showed weight loss percentages of 2.22% and 1.85%, respectively. Notably, compared with ABX464 (5.37% weight loss), all tested compounds of the present invention displayed superior ameliorative effects on body weight reduction.

2.2 Effects of the Compounds of the Present Invention on DAI in DSS-Induced IBD Mice

The results of DAI scores are presented in Table 3. Compared with the normal control group, the model group showed significantly elevated DAI scores, reaching a total of 14.5 points. In contrast, the compound-treated groups (19, 43, 59, 84, and 89) of the present invention demonstrated total DAI scores of 8.33, 10.03, 8.57, 8.47, and 10.33 points, respectively, indicating marked reductions compared to the model group. Notably, compounds 19, 59, and 84 exhibited statistically significant reductions in total DAI scores by 42.55%, 40.90%, and 41.59%, respectively. Furthermore, when compared with ABX464 (which showed only a 5.72% reduction), the compounds of the present invention achieved substantially greater decreases in total DAI scores, demonstrating superior efficacy in improving DAI scores relative to the reference drug ABX464.

**Table 3. Total Disease Activity Index (DAI) Scores**

| Group | Normal control | Model group | ABX464 | Compo und 19 | Compo und 43 | Compou nd 59 | Compo und 84 | Compo und 89 |
|---|---|---|---|---|---|---|---|---|
| DAI | 0.67 ± 0.37 | 14.50 ± 0.87 | 13.67 ± 1.31 | 8.33 ± 1.80 | 10.03 ± 2.11 | 8.57 ± 1.10 | 8.47 ± 0.55 | 10.33 ± 0.94 |

2.3 Effects of the Compounds of the Present Invention on Colon Length in DSS-Induced IBD Mice

The colon length measurement results are shown in Table 4. Compared with the normal control group, the model group exhibited significant colon shortening, reaching only 77.32% of the normal length, indicating successful establishment of the IBD model. In contrast, administration of the compounds of the present invention (19, 43, 59, 84, and 89) significantly increased colon length to 96.27%, 99.69%, 100%, 96.89%, and 99.07% of normal control values, respectively (all $P<0.05$ versus model group). These results demonstrate that the compounds effectively ameliorated DSS-induced colonic damage, with near-complete restoration to normal physiological length. Furthermore, compared with ABX464 treatment, the compounds of the present invention showed comparable or slightly superior efficacy in improving colon length (particularly Compound 59, which completely restored normal colon length).

**Table 4. Colon length of mice (cm)**

| Group | Normal control | Model group | ABX4 64 | Compo und 19 | Compo und 43 | Compo und 59 | Compo und 84 | Compo und 89 |
|---|---|---|---|---|---|---|---|---|
| Colon length (cm) | 6.44 ± 0.13 | 4.98 ± 0.21 | 6.22 ± 0.12 | 6.20 ± 0.20 | 6.42 ± 0.11 | 6.48 ± 0.10 | 6.24 ± 0.17 | 6.38 ± 0.12 |

3. Conclusions

This study evaluated the preventive and therapeutic effects of the inventive compounds on DSS-induced IBD in mice. The experimental model was successfully established, with significant changes observed in key evaluation indicators, including body weight, Daily Disease Activity Index (DAI), and colon length. The final results demonstrated that the inventive compounds exhibited favorable pharmacological effects on body weight, DAI, and colon length in mice. Notably, their efficacy in improving body weight and DAI was significantly superior to that of the reference drug ABX464. Therefore, the inventive compounds show promising preventive and therapeutic effects against DSS-induced IBD in mice.

Further experiments confirmed that other compounds of the present invention (e.g., B7, B83, B97, and B108) also displayed preventive and therapeutic effects on DSS-induced IBD in mice. These compounds demonstrated favorable pharmacological effects on body weight, DAI, and colon length, all of which outperformed the reference drug ABX464.

All references mentioned in the present disclosure are cited as references in this application, just as each reference is cited separately. In addition, it should be understood that after reading the above lecture content of the present disclosure, those skilled in the art can make various changes or modifications to the present disclosure, and these equivalent forms also fall within the scope of the claims attached to the present disclosure.

## Claims

1. A compound having the structure shown in Formula (1), or an isomer, crystal form, pharmaceutically acceptable salt, hydrate, or solvate thereof:

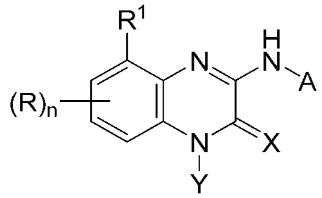

**(1)**

wherein:

n is 0, 1, 2 or 3;
each R is independently selected from the group consisting of: halogen, CN, nitro, $C_{1-3}$ alkyl, $C_{1-3}$ haloalkyl, $C_{1-3}$ alkoxy or $C_{1-3}$ haloalkoxy;
$R^1$ is selected from the group consisting of: H, CN, $C_{1-3}$ alkyl, $C_{1-3}$ alkoxy or halogen;
A is selected from the group consisting of: 6-10 membered aryl, 5-7 membered heteroaryl, benzo 5-7-membered cycloalkyl or benzo 5-7-membered heterocyclyl, wherein the aryl, heteroaryl, benzo cycloalkyl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$haloalkoxy, $C_{1-3}$ haloalkylthio or $SF_3$;
X is O, and Y is $C_{1-3}$ alkyl, $C_{1-3}$ haloalkyl or $C_{3-6}$ cycloalkyl; or
X and Y are taken together with the atoms to which they are bound to form a 5-membered heteroaryl or a 5-membered heterocyclyl, wherein the heteroaryl and heterocyclyl can be further substituted by 0-2 substituents of the following groups: $C_{1-3}$ alkyl or $C_{1-3}$ haloalkyl.

2. The compound according to claim 1, or an isomer, crystal form, pharmaceutically acceptable salt, hydrate, or solvate thereof, wherein at least one of R and $R^1$ is halogen.

3. The compound according to claim 1, or an isomer, crystal form, pharmaceutically acceptable salt, hydrate, or solvate thereof, wherein each R is independently selected from the group consisting of: F, Cl, Br, Me, Et, OMe, OEt, $CF_3$, CN, $OCF_3$ or $NO_2$.

4. The compound according to claim 1, or an isomer, crystalline form, pharmaceutically acceptable salt, hydrate, or solvate thereof, wherein $R^1$ is selected from the group consisting of: H, CN, Me, Et, OMe, OEt, F, Cl, or Br.

5. The compound according to claim 1, or an isomer, crystalline form, pharmaceutically acceptable salt, hydrate, or solvate thereof, wherein A is selected from the group consisting of:

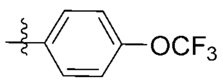, 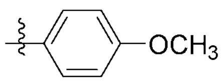, 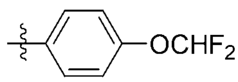,

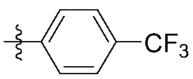, 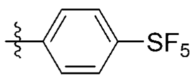, 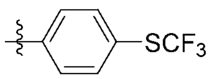, 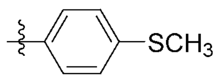, 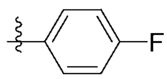,

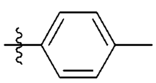, 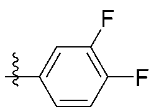, 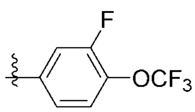, 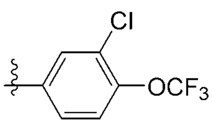, 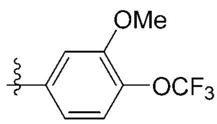,

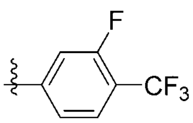, 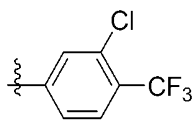, 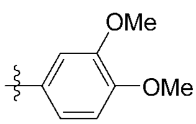, 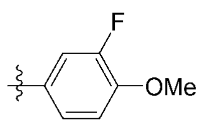, 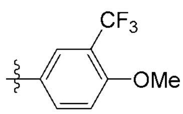,

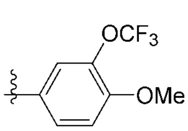, 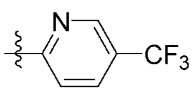, 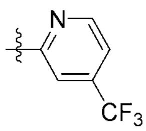, 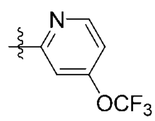, 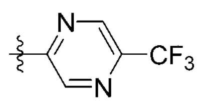,

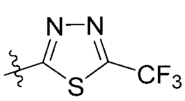, 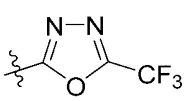, 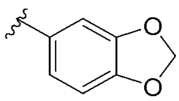, 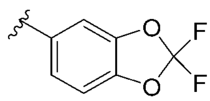, 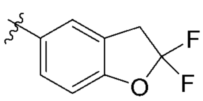,

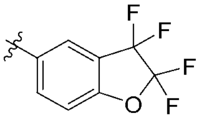,
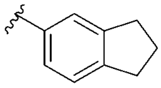,
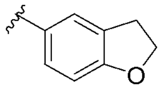,
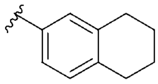,

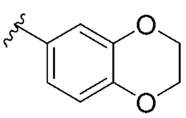 or 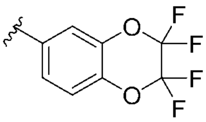.

6. The compound according to claim 1, or an isomer, crystal form, pharmaceutically acceptable salt, hydrate, or solvate thereof, **characterized in that** the compound has the structure as shown in Formula (1A):

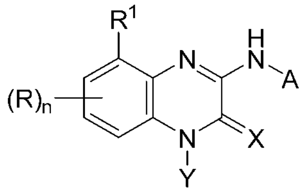

**(1A)**

wherein:

n is 0, 1 or 2;
R is selected from the group consisting of: halogen, CN, nitro, $C_{1-3}$ alkyl, $C_{1-3}$ haloalkyl or $C_{1-3}$ alkoxy;
$R^1$ is a halogen;
A is selected from the group consisting of: 6-10 membered aryl, 5-7 membered heteroaryl or benzo 5-7-membered heterocyclyl, wherein the aryl, heteroaryl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$ haloalkoxy, $C_{1-3}$ haloalkylthio or $SF_3$;
X is O, and Y is $C_{1-3}$ alkyl, $C_{1-3}$ haloalkyl or $C_{3-6}$ cycloalkyl; or
X and Y are taken together with the atoms to which they are bound to form a 5-membered heteroaryl or a 5-membered heterocyclyl, wherein the heteroaryl and heterocyclyl can be further substituted by 0-2 substituents of the following groups: $C_{1-3}$ alkyl or $C_{1-3}$ haloalkyl.

7. The compound according to claim 1, or an isomer, crystal form, pharmaceutically acceptable salt, hydrate, or solvate thereof, wherein

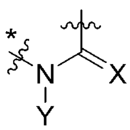

has a structure selected from the group consisting of:

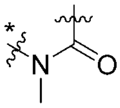, 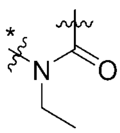, 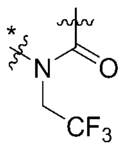,

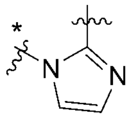, 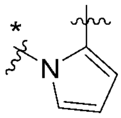, 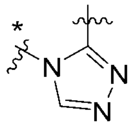, 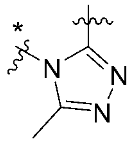, 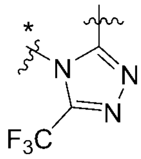, 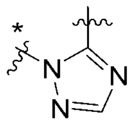, 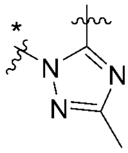

or

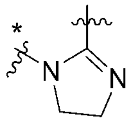;

wherein "*" represents the position connected to the benzene ring.

8. The compound according to claim 1, or an isomer, crystal form, pharmaceutically acceptable salt, hydrate, or solvate thereof, **characterized in that** the compound has a structure selected from the group consisting of:

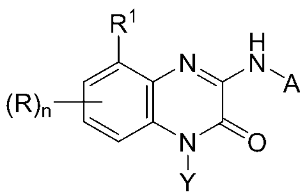

**(1A-1)**

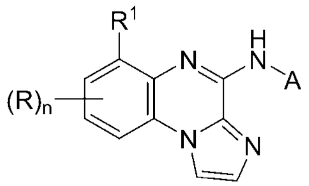

**(1A-2)**

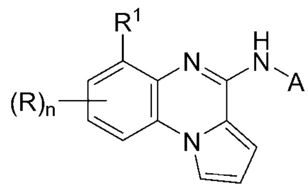

**(1A-3)**

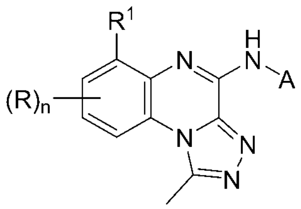

**(1A-4)**

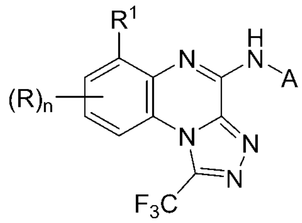

**(1A-5)**

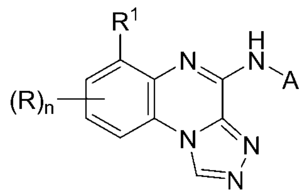

**(1A-6)**

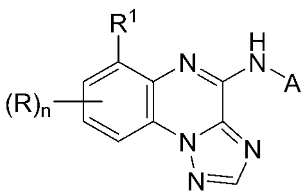

**(1A-7)**

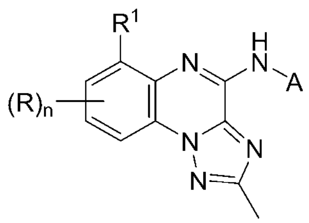

**(1A-8)**

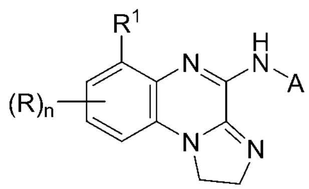

**(1A-9)**

wherein n, Y, R, $R^1$ and A are as defined in claim 1.

9. The compound according to claim 1, or an isomer, crystal form, pharmaceutically acceptable salt, hydrate, or solvate thereof, **characterized in that** the compound has the structure as shown in Formula (1B):

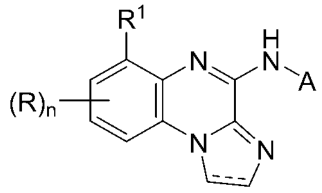

**(1B)**

wherein:

"- - -" indicates a single bond or a double bond;
n is 1, 2 or 3;
each R is independently selected from the group consisting of: halogen, CN, $C_{1-3}$ alkyl, $C_{1-3}$ haloalkyl, $C_{1-3}$ alkoxy or $C_{1-3}$ haloalkoxy;
$R^1$ is selected from the group consisting of: H, CN, $C_{1-3}$ alkyl or $C_{1-3}$ alkoxy;
A is selected from the group consisting of: 6-10 membered aryl, 5-7 membered heteroaryl, benzo 5-7-membered cycloalkyl or benzo 5-7-membered heterocyclyl, wherein the aryl, heteroaryl, benzo-cycloalkyl and benzo heterocyclyl can be further substituted by 0-4 substituents selected from the group consisting of: halogen, CN, $C_{1-3}$ alkyl, $C_{3-6}$ cycloalkyl, $C_{1-3}$ alkoxy, $C_{1-3}$ alkylthio, $C_{1-3}$ haloalkyl, $C_{1-3}$ haloalkoxy, $C_{1-3}$ haloalkylthio or $SF_5$.

**10.** The compound according to any one of claims 1-9, or an isomer, crystal form, pharmaceutically acceptable salt, hydrate, or solvate thereof, **characterized in that** the compound has the structure as shown in Formula (2):

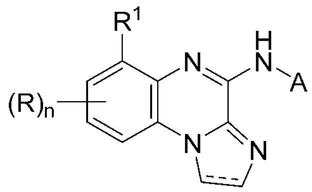

(2)

wherein:

"- - -" indicates a single bond or a double bond;
n is 0, 1 or 2;
$R^1$ is selected from the group consisting of: H, F, Cl or Br;
each R is independently selected from the group consisting of: F, Cl or Br.

**11.** The compound according to claim 10, or an isomer, crystal form, pharmaceutically acceptable salt, hydrate, or solvate thereof, wherein A is selected from the group consisting of:

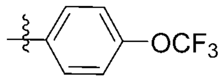, 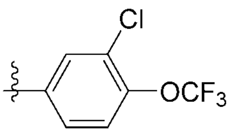 or 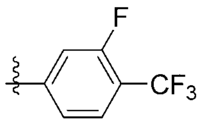.

**12.** The compound according to claim 10, or an isomer, crystal form, pharmaceutically acceptable salt, hydrate, or solvate, wherein n is 1 or 2 when $R^1$ is H.

**13.** The compound according to claim 1, an isomer thereof or an pharmaceutically acceptable salt thereof, wherein the compound has a structure selected from the group consisting of:

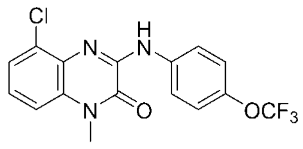

**1**

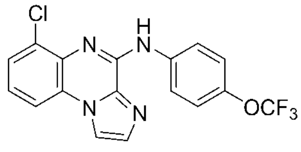

**2**

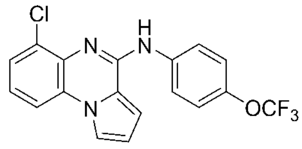

**3**

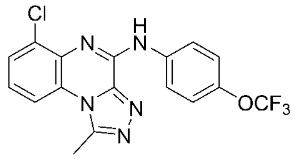

**4**

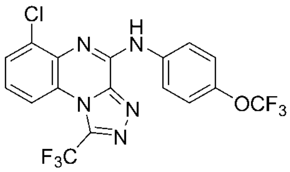

**5**

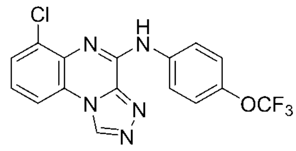

**6**

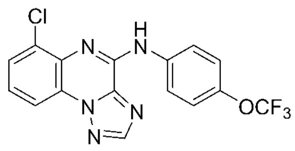

**7**

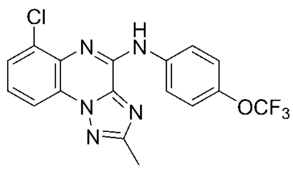

**8**

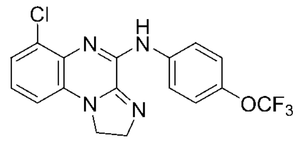

**9**

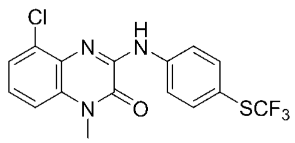

**10**

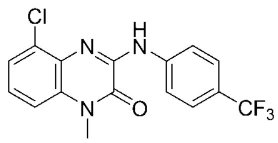

**11**

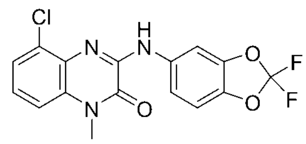

**12**

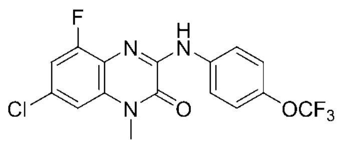

**13**

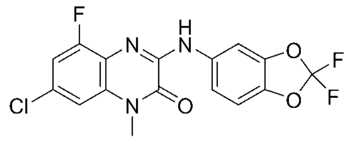

**14**

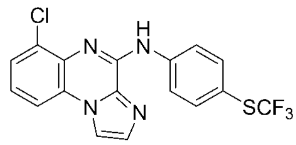

**15**

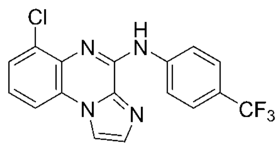

**16**

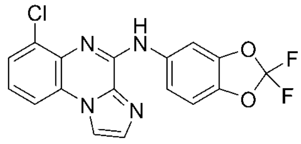

**17**

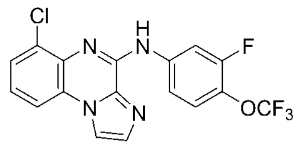

**18**

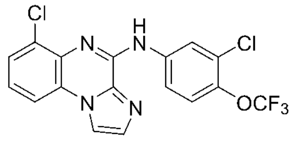

**19**

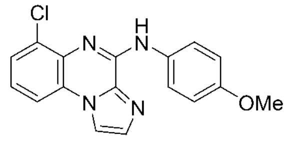

**20**

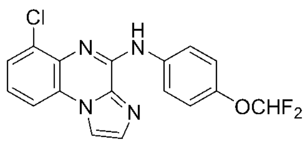

**21**

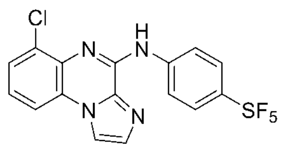

**22**

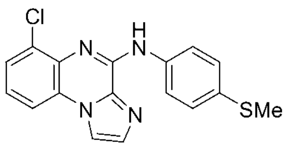

**23**

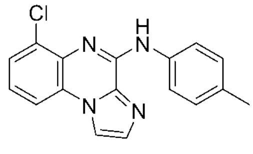

**24**

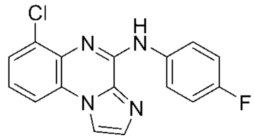

**25**

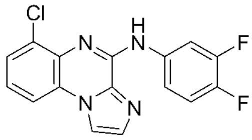

**26**

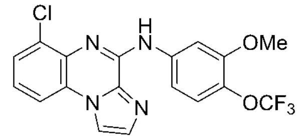

**27**

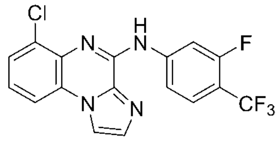

**28**

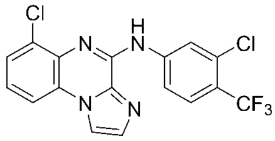

**29**

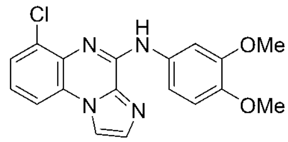

**30**

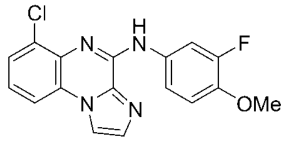

**31**

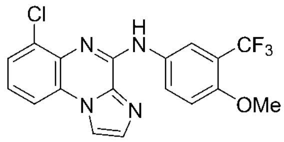

**32**

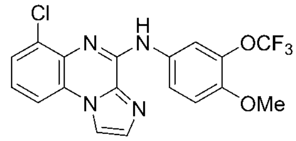

**33**

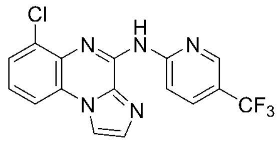

**34**

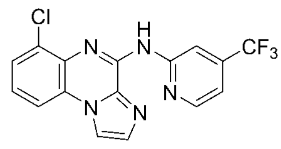

**35**

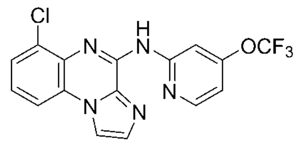

**36**

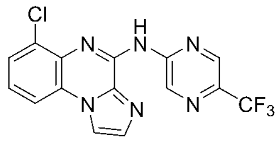

**37**

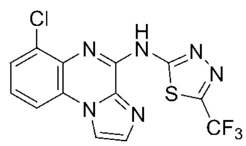

**38**

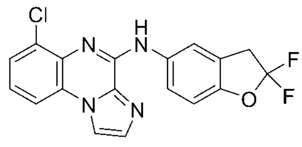

**39**

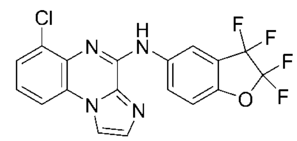

**40**

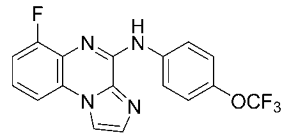

**41**

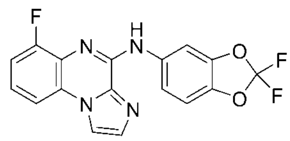

**42**

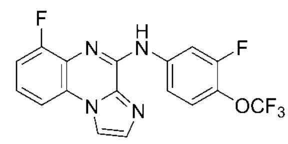

**43**

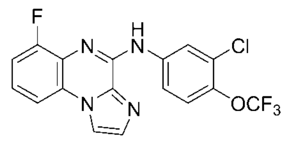

**44**

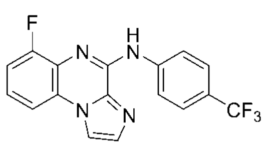

**45**

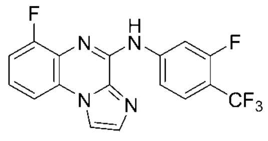

46

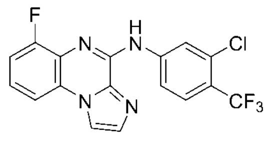

47

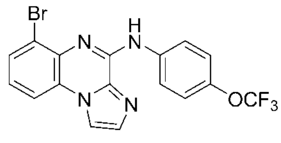

48

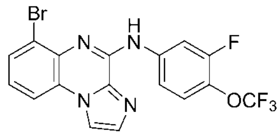

49

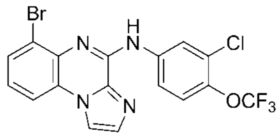

50

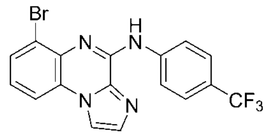

51

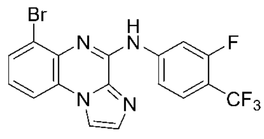

52

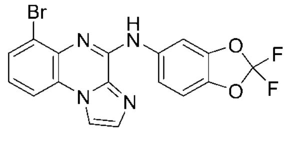

53

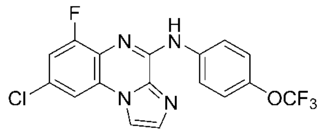

54

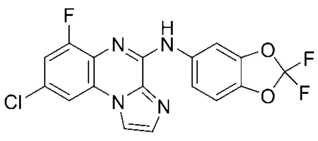

55

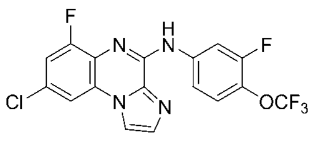

56

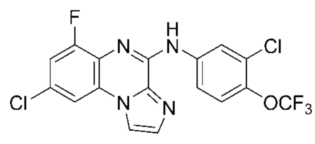

57

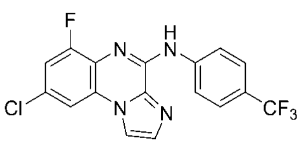

58

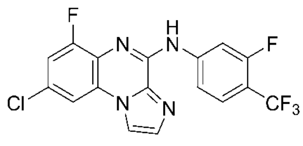

59

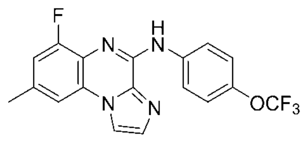

60

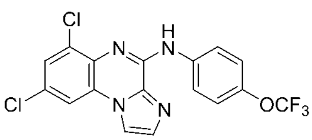

61

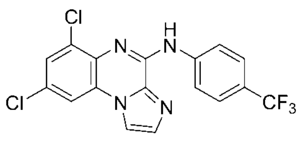

62

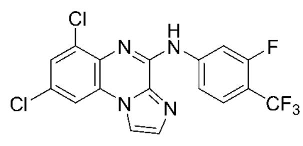

63

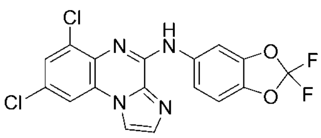

64

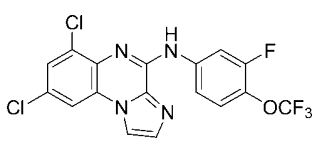

65

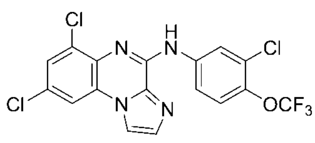

66

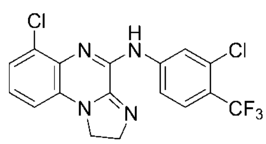

67

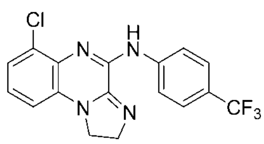

68

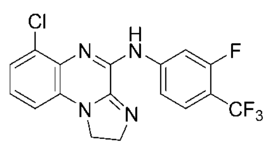

69

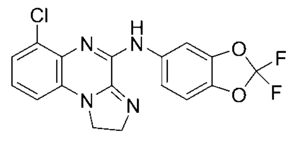

70

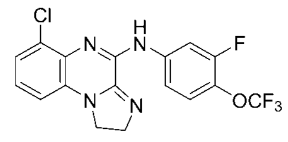

71

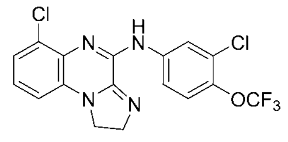

72

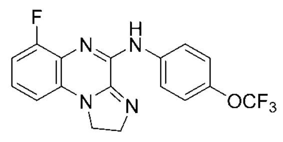

73

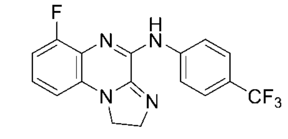

74

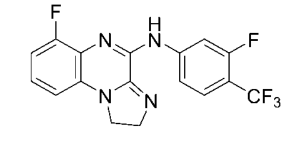

75

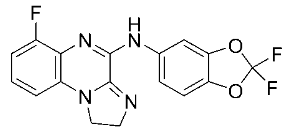

76

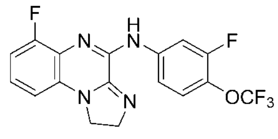

77

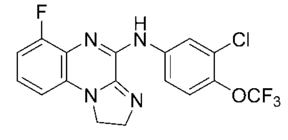

78

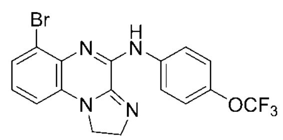

79

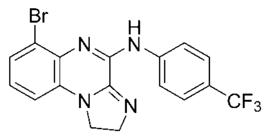

80

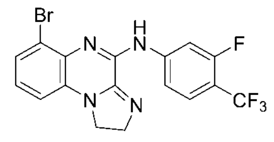

81

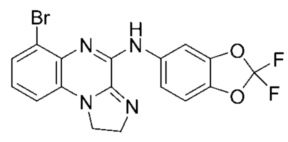

82

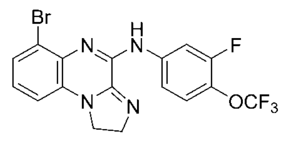

83

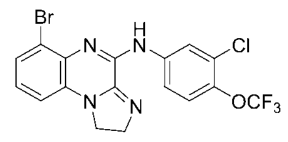

84

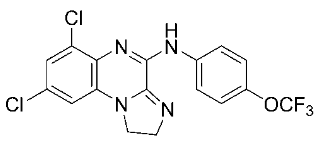

85

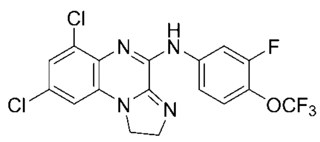

86

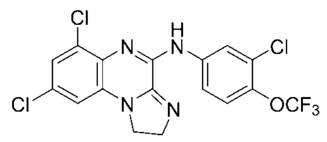

87

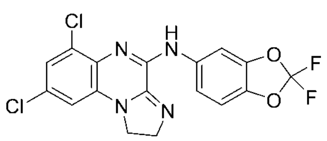

88

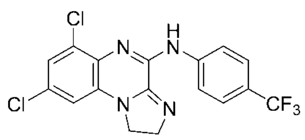

89

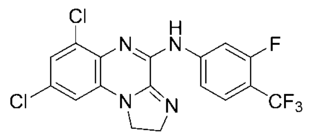

90

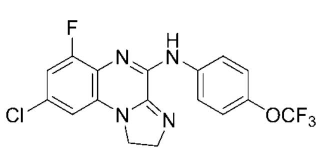

91

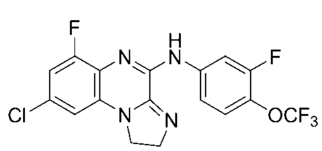

92

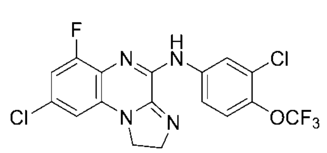

93

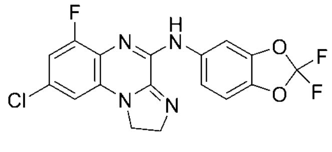

**94**

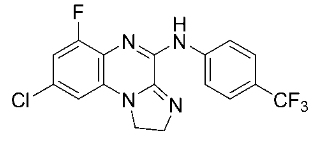

**95**

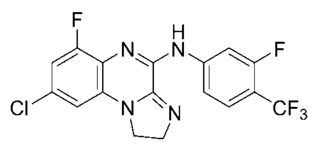

**96**

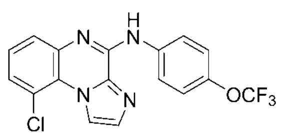

**B1**

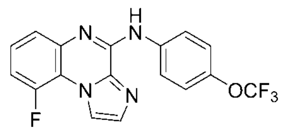

**B2**

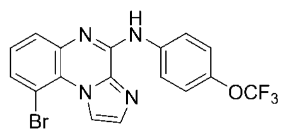

**B3**

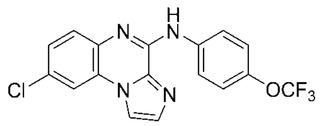

**B4**

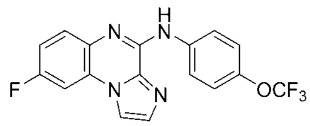

**B5**

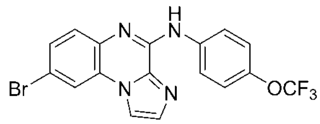

**B6**

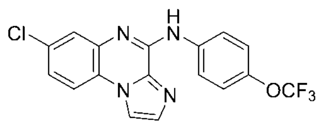

**B7**

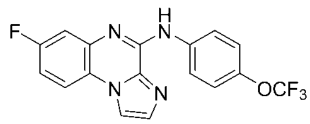

**B8**

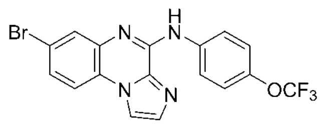

**B9**

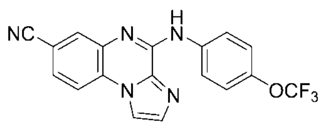

**B10**

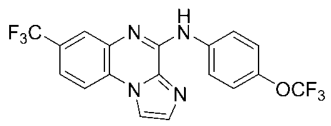

**B11**

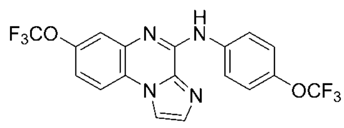

**B12**

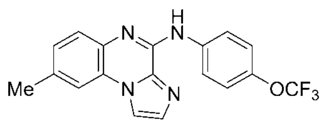

**B13**

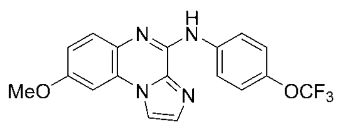

**B14**

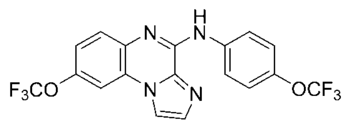

**B15**

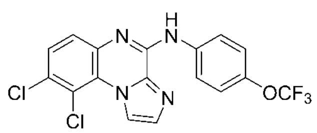

**B16**

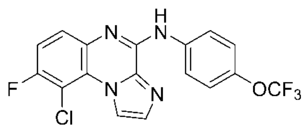

**B17**

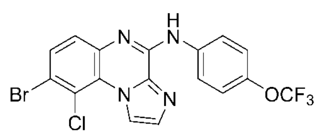

**B18**

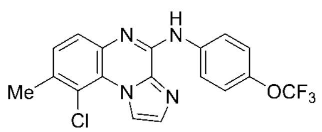

**B19**

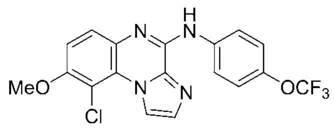

**B20**

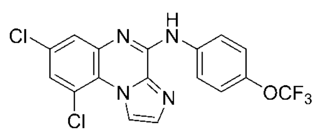

**B21**

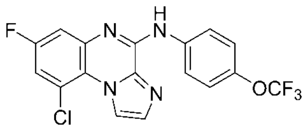

**B22**

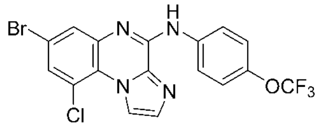

**B23**

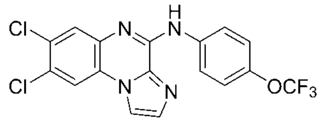

**B24**

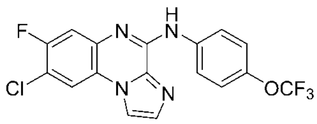

**B25**

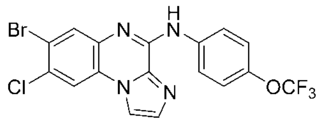

**B26**

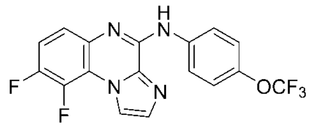

**B27**

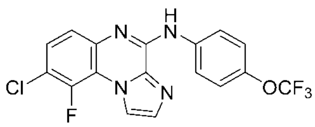

**B28**

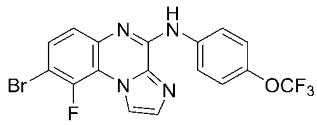

**B29**

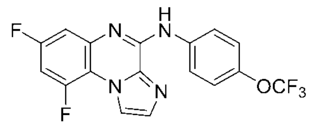

**B30**

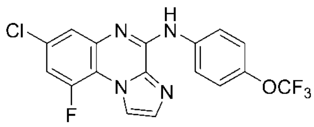

**B31**

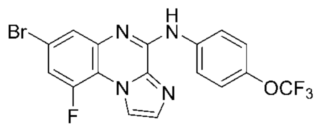

**B32**

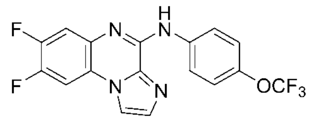

**B33**

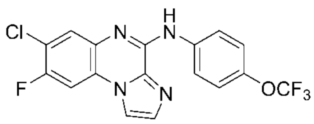

**B34**

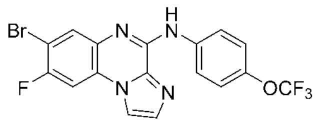

**B35**

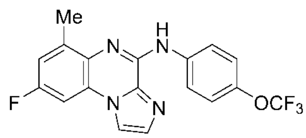

**B36**

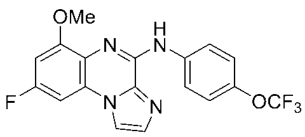

**B37**

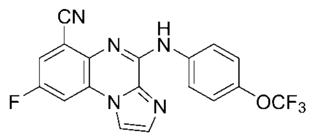

**B38**

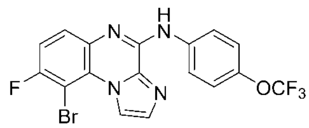

**B39**

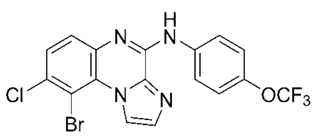

**B40**

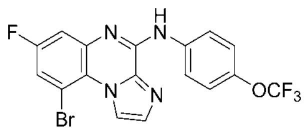

**B41**

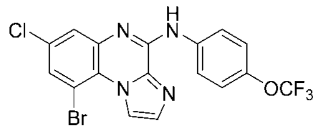

**B42**

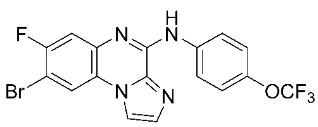

**B43**

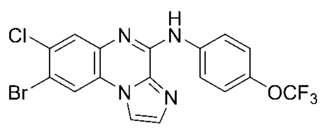

**B44**

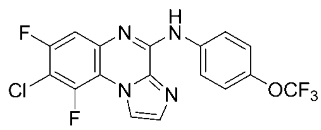

**B45**

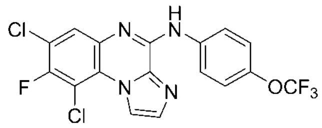

**B46**

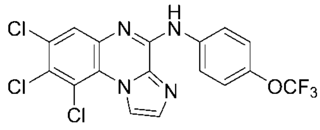

**B47**

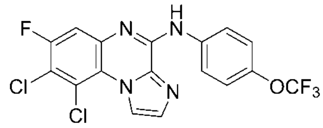

**B48**

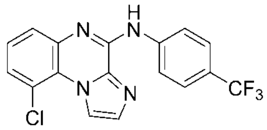

**B49**

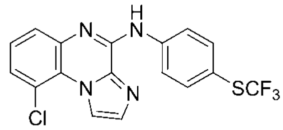

**B50**

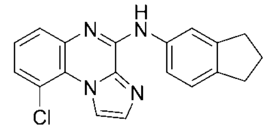

**B51**

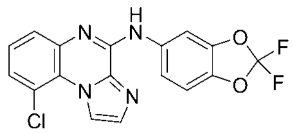

**B52**

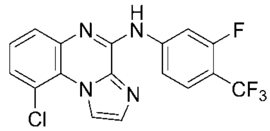

**B53**

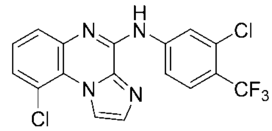

**B54**

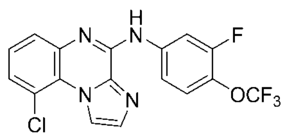

**B55**

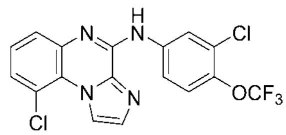

**B56**

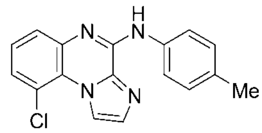

**B57**

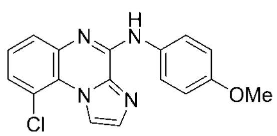

**B58**

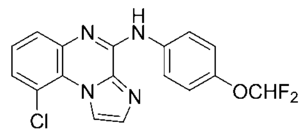

**B59**

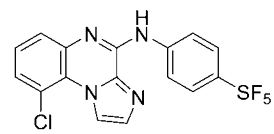

**B60**

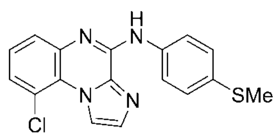

**B61**

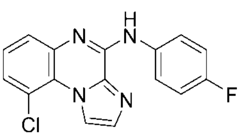

**B62**

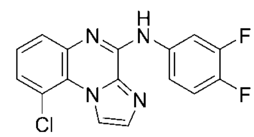

**B63**

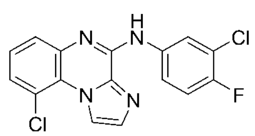

**B64**

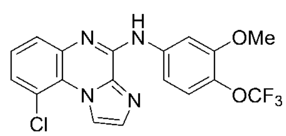

**B65**

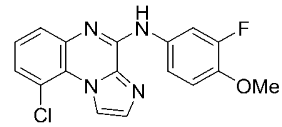

**B66**

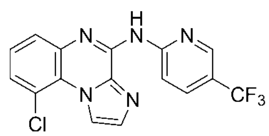

**B67**

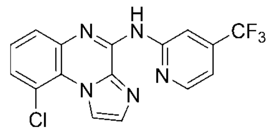

**B68**

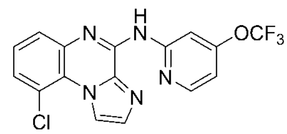

**B69**

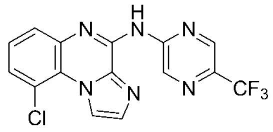

**B70**

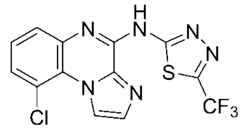

**B71**

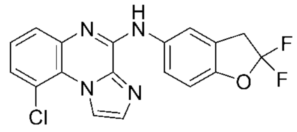

**B72**

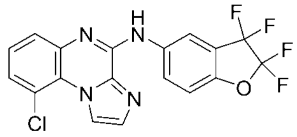

**B73**

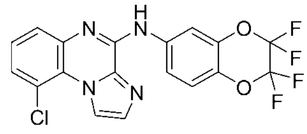

**B74**

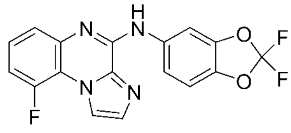

**B75**

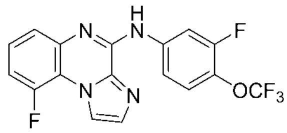

**B76**

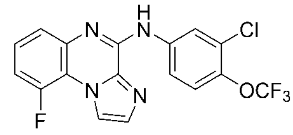

**B77**

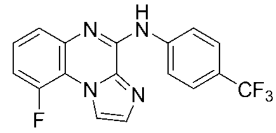

**B78**

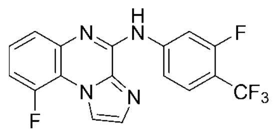

**B79**

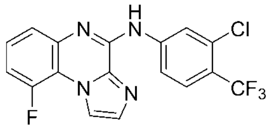

**B80**

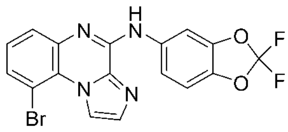

**B81**

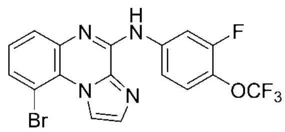

**B82**

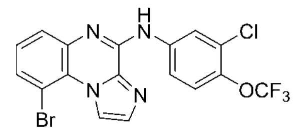

**B83**

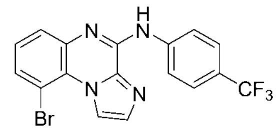

**B84**

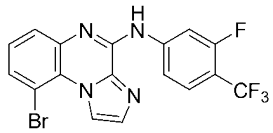

**B85**

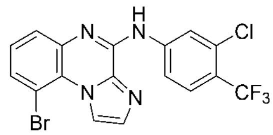

**B86**

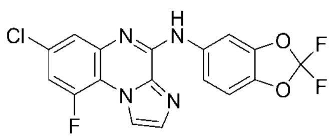

**B87**

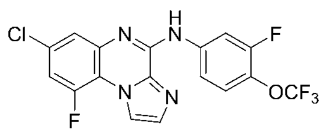

**B88**

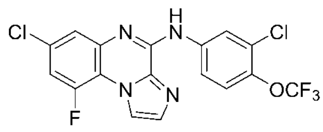

**B89**

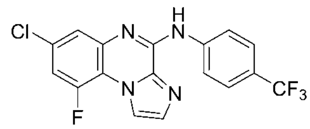

**B90**

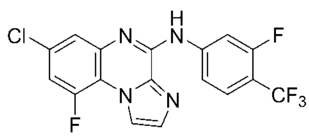

**B91**

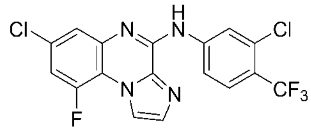

**B92**

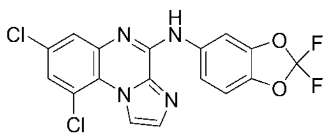

**B93**

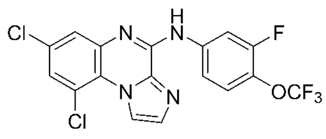

**B94**

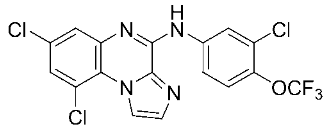

**B95**

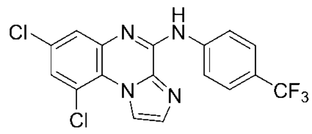

**B96**

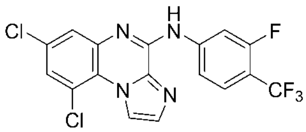

**B97**

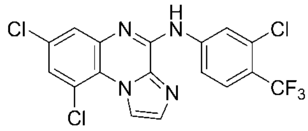

**B98**

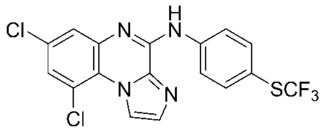

**B99**

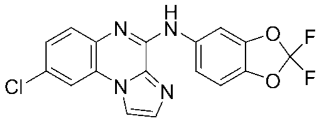

**B100**

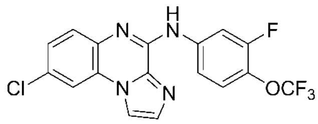

**B101**

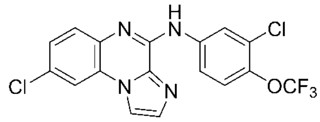

**B102**

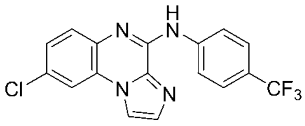

**B103**

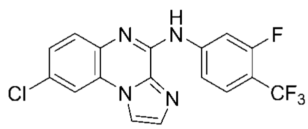

**B104**

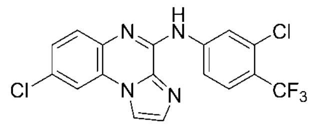

**B105**

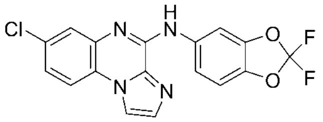

**B106**

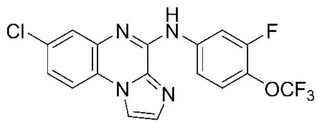

**B107**

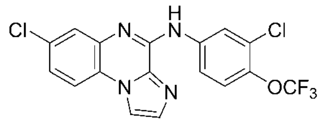

**B108**

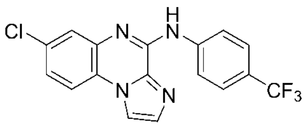

**B109**

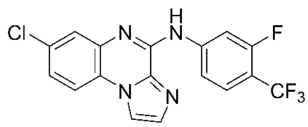

**B110**

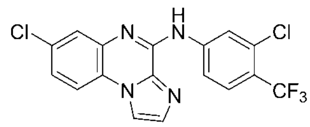

**B111**

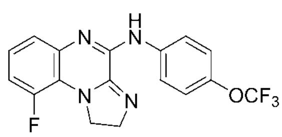

**B112**

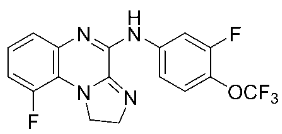

**B113**

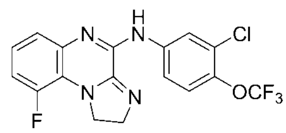

**B114**

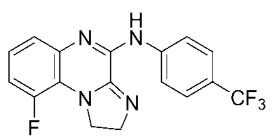

**B115**

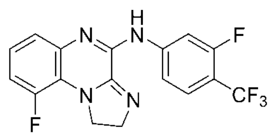

**B116**

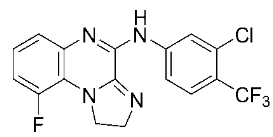

**B117**

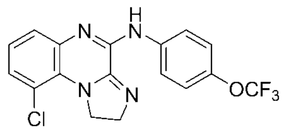
B118

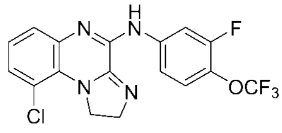
B119

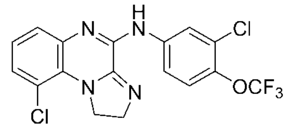
B120

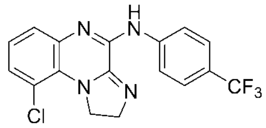
B121

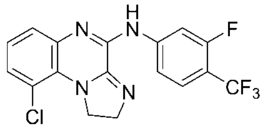
B122

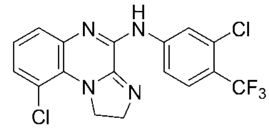
B123

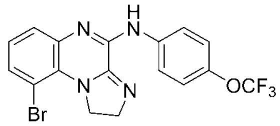
B124

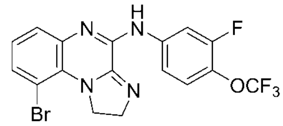
B125

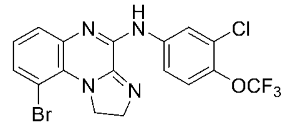
B126

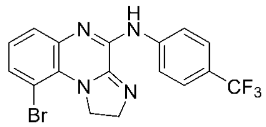
B127

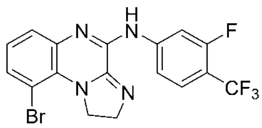
B128

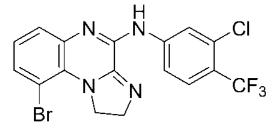
B129

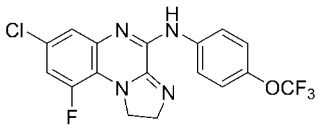
B130

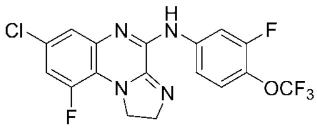
B131

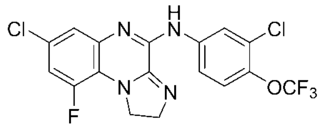
B132

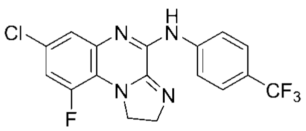
B133

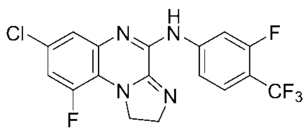
B134

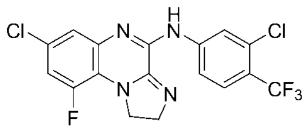
B135

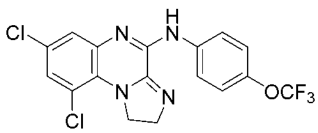
B136

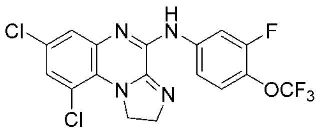
B137

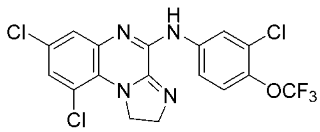
B138

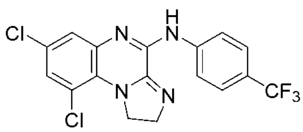
B139

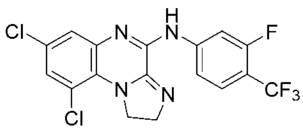
B140

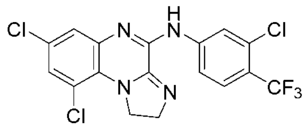
B141

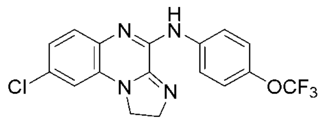

**B142**

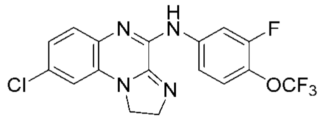

**B143**

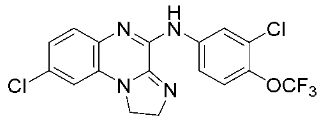

**B144**

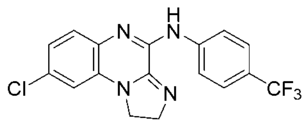

**B145**

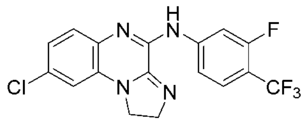

**B146**

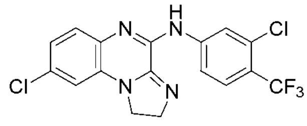

**B147**

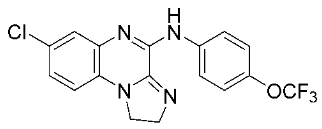

**B148**

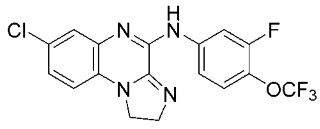

**B149**

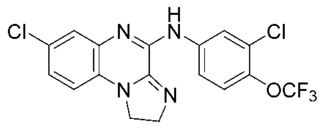

**B150**

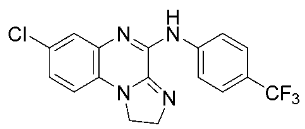

**B151**

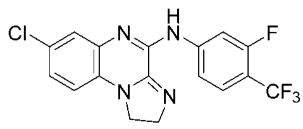

**B152**

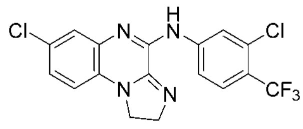

**B153**

.

**14.** A pharmaceutical composition for treating, regulating, and/or preventing a disease associated with miR-124, **characterized in that** the pharmaceutical composition comprises:

(1) the compound as an active ingredient according to any one of claims 1-13, or an isomer, crystal form, pharmaceutically acceptable salt, hydrate, or solvate thereof; and

optionally (2) a pharmaceutically acceptable excipient or carrier.

**15.** Use of the compound according to any one of claims 1-13, or an isomer, crystal form, pharmaceutically acceptable salt, hydrate or solvate thereof, or the pharmaceutical composition according to claim 14, for preparing a pharmaceutical composition for treating, regulating and/or preventing a disease mediated by miR-124.

**16.** The use according to claim 15, **characterized in that** the disease mediated by miR-124 is selected from the group consisting of: Alzheimer's disease, Parkinson's disease, brain tumor, gastric cancer, liver cancer, lung cancer, colorectal cancer, pancreatic cancer, breast cancer, cervical cancer, endometrial cancer, prostate cancer, Crohn's disease, ulcerative colitis, rheumatoid arthritis, allergic rhinitis, osteoarthritis, fibrosis disease, AIDS and/or COVID-19.

**17.** A method for preparing a compound of Formula (1), **characterized in that** the method includes the steps of:

(1) in an inert solvent, the compound of formula (A) and its tautomer react to obtain the compound of formula (B):

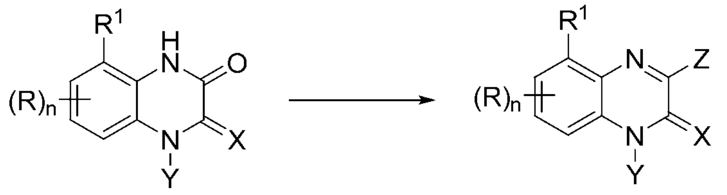

A B ;

(2) in an inert solvent, the compound of formula (B) reacts with the compound of formula (d) to obtain the compound of formula (1):

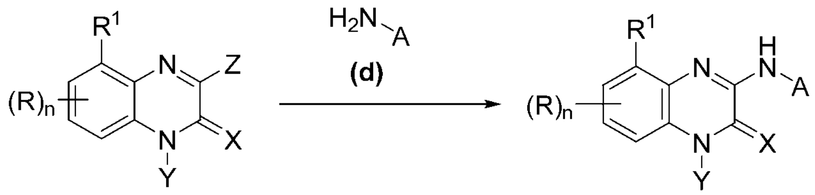


B (1) :

wherein Z is a halogen or OTf; X, Y, R, $R^1$, A and n are as defined in claim 1.

**18.** The method according to claim 17, **characterized in that** the compound of formula (1) is a compound of formula (1a), and the method includes the following steps:

(s1) in an inert solvent, the compound of formula (A1) and its tautomer react to obtain the compound of formula (B1):

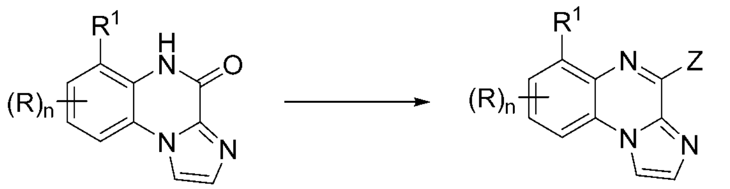

A1 B1 ;

(s2) in an inert solvent, the compound of formula (B1) reacts with the compound of formula (d) to obtain the compound of formula (1a):

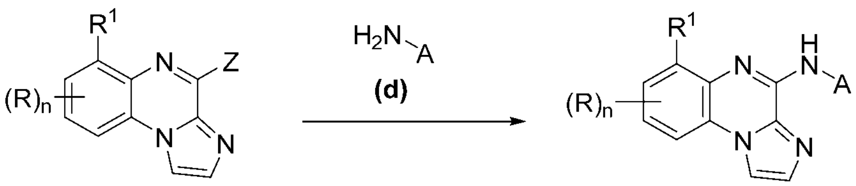


B1 (1a) ;

wherein Z is a halogen or OTf; R, $R^1$, A and n are as defined in claim 1.

**19.** The method according to claim 17, **characterized in that** the compound of formula (1) is a compound of formula (1b), and the method includes the following steps:

(t1) in an inert solvent, the compound of formula (C) undergoes a cyclization reaction to yield the compound of

formula (D):

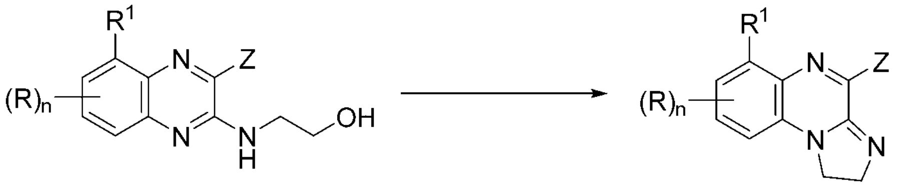

C

D

;

(t2) in an inert solvent, the compound of formula (D) reacts with the compound of formula (d) to obtain the compound of formula (1b):

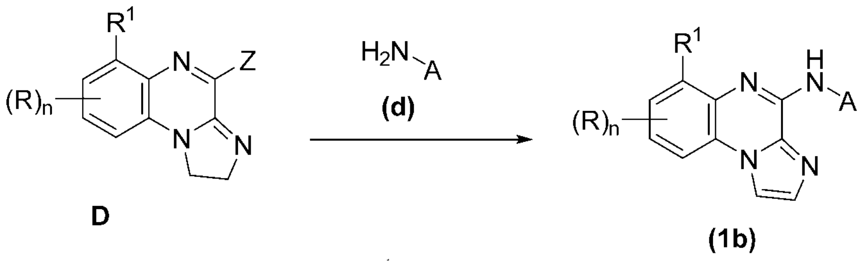


D

(1b)

wherein Z is a halogen or OTf; R, $R^1$, A and n are as defined in claim 1.

**20.** A method for treating, regulating, and/or preventing a disease mediated by miR-124, **characterized by** comprising administering to an individual in need thereof an amount of the compound according to any one of claims 1-13, or an isomer, crystalline form, pharmaceutically acceptable salt, hydrate, or solvate thereof, or a pharmaceutical composition according to claim 14.

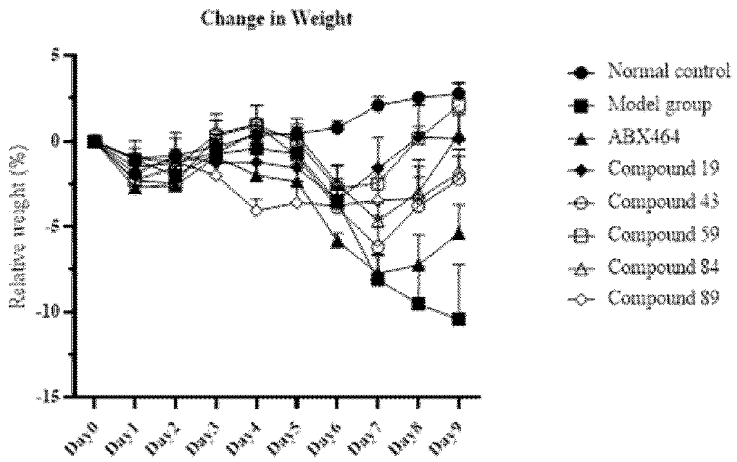
Change in Weight
Relative weight (%)
5
0
-5
-10
-15
Day0
Day1
Day2
Day3
Day4
Day5
Day6
Day7
Day8
Day9
Normal control
Model group
ABX464
Compound 19
Compound 43
Compound 59
Compound 84
Compound 89

Figure 1

## INTERNATIONAL SEARCH REPORT

International application No.
**PCT/CN2024/076609**

### A. CLASSIFICATION OF SUBJECT MATTER

C07D241/44 (2006.01)i; C07D241/42(2006.01)i; C07D403/02(2006.01)i; C07D403/04(2006.01)i; A61K31/498(2006.01)i; A61K31/496(2006.01)i; A61P35/00(2006.01)i; A61P29/00(2006.01)i; A61P25/28(2006.01)i; A61P25/16(2006.01)i

According to International Patent Classification (IPC) or to both national classification and IPC

### B. FIELDS SEARCHED

Minimum documentation searched (classification system followed by classification symbols)

IPC: C07D; A61K31; A61P

Documentation searched other than minimum documentation to the extent that such documents are included in the fields searched

Electronic data base consulted during the international search (name of data base and, where practicable, search terms used)

VCN, CNTXT, ENTXTC, ENTXT, DWPI, VEN, CNKI, 万方数据库, WANFANG DATABASE, STN-REGISTRY, STN-MARPAT, STN-CAPLUS, STN-CASREACT, WEB OF SCIENCE: 南京同诺康, 喹喔啉酮, miR-124, 癌, 肿瘤, 阿尔茨海默病, 艾滋病, 肠炎, 关节炎, 鼻炎, 纤维化, 肺炎, NANJING TONGNUOKANG, quinoxalinone, cancer, carcinoma, tumo?r, Alzheimer's disease, AIDS, enteritis, arthritis, rhinitis, fibrosis, pneumonia

### C. DOCUMENTS CONSIDERED TO BE RELEVANT

| Category* | Citation of document, with indication, where appropriate, of the relevant passages | Relevant to claim No. |
|---|---|---|
| X | WO 9909845 A1 (BRISTOL-MYERS SQUIBB COMPANY) 04 March 1999 (1999-03-04)<br>claims 1-33 and 40-42, and description, table 5, and embodiments 1, 7-8 and 11 | 1-20 |
| X | SERGIO A. et al. "Quinoxaline Chemistry. Part 16. 4-Substituted Anilino and 4-substituted Phenoxymethyl Pyrrolo[1,2-a] Quinoxalines and N-[4- (Pyrrolo[1,2-a]quinoxalin-4-yl)amino and Hydroxymethyl] Benzoyl Glutamates. Synthesis and Evaluation of In Vitro Biological Activity"<br>*Farmaco*, Vol. 58, No. (9), 31 December 2003 (2003-12-31), 639-650<br>ISSN: 0014-827X,<br>page 640, figure 1, and page 645, table 2 | 1-20 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 May 2024** | **28 May 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/076609

C. DOCUMENTS CONSIDERED TO BE RELEVANT

| Category* | Citation of document, with indication, where appropriate, of the relevant passages | Relevant to claim No. |
|---|---|---|
| X | SUN H. et al. "Discovery and Design of Tricyclic Scaffolds as Protein Kinase CK2 (CK2) InhStructure-Based Molecular Modificationibitors through a Combination of Shape-Based Virtual Screening and Structure-Based Molecular Modification" *Journal of Chemical Information and Modeling,* Vol. 53, No. (8), 16 July 2013 (2013-07-16), 2093-2102 ISSN: 1549-9596, page 2093, abstract, page 2097, table 1, and page 2098, table 2 | 1-20 |
| X | US 2010298302 A1 (PIERRE FABRICE et al.) 25 November 2010 (2010-11-25) claims 1-20; and description, table 1, and embodiments 1-2 | 1-20 |
| X | YI B. et al. "Visible-light-photocatalysis Driven Denitrogenative/Radical 1,3-shift of Benzotriazole: Access to 3-arylaminoquinoxalin-2(1H)-one Scaffolds" *Organic Chemistry Frontiers,* Vol. 10, No. (2), 07 December 2022 (2022-12-07), 531-539 ISSN: 2052-4129, page 533, table 2 | 1-4, 7, 8 |
| X | US 6200976 B1 (BOEHRINGER INGELHEIM PHARMA) 13 March 2001 (2001-03-13) embodiment 43 | 1-4, 7, 8 |
| A | WO 2021219091 A2 (BEIJING TIDE PHARMACEUTICAL CO., LTD.) 04 November 2021 (2021-11-04) entire document | 1-20 |
| A | CN 114853752 A (YAOYA TECHNOLOGY (SHANGHAI) CO., LTD.) 05 August 2022 (2022-08-05) entire document | 1-20 |

Form PCT/ISA/210 (second sheet) (July 2022)

## INTERNATIONAL SEARCH REPORT

International application No.
**PCT/CN2024/076609**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20**
because they relate to subject matter not required to be searched by this Authority, namely:

Claim 20 relates to a treatment method, which falls within subject matter for which a search is not required (PCT Rule 39(iv)). However, it is expected that the applicant may amend the subject matter of claim 20 to be a pharmaceutical use; therefore, an international search report is provided on this basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/076609**

| Patent document cited in search report | Publication date (day/month/year) | Patent family member(s) | Publication date (day/month/year) |
|---|---|---|---|
| WO 9909845 A1 | 04 March 1999 | AR 013441 A1 | 27 December 2000 |
| | | US 6235740 B1 | 22 May 2001 |
| | | AU 8681798 A | 16 March 1999 |
| | | PE 106300 A1 | 23 January 2000 |
| US 2010298302 A1 | 25 November 2010 | WO 2010135571 A1 | 25 November 2010 |
| US 6200976 B1 | 13 March 2001 | None | |
| WO 2021219091 A2 | 04 November 2021 | WO 2021219091 A3 | 23 December 2021 |
| | | WO 2021219090 A1 | 04 November 2021 |
| | | TW 202200563 A | 01 January 2022 |
| | | TW 202200562 A | 01 January 2022 |
| CN 114853752 A | 05 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Stem Cells*, 2013, vol. 31, 2205-2217
- *Trends Mol Med*, 2014, vol. 331, 1-10
- *Oncogene*, 2012, vol. 31, 1609-1622
- *Cell Mol Life Sci*, 2018, vol. 75, 4557-4581
- *Retrovirology*, 2015, vol. 12, 1-15